# EUROPÄISCHE PATENTSCHRIFT

(11) **EP 1 581 569 B1**
(45) Veröffentlichungstag und Bekanntmachung des Hinweises auf die Patenterteilung: **03.01.2007**
(21) Anmeldenummer: 03813917.6
(22) Anmeldetag: 29.12.2003
(51) Int. Cl.: C08F 246/00, C08F 220/04, C08F 220/34, C08F 226/10, C08F 220/60, A61K 8/72

(54) **AMPHOLYTISCHES COPOLYMER UND DESSEN VERWENDUNG**
AMPHOLYTIC COPOLYMER AND USE THEREOF
COPOLYMERE AMPHOLYTIQUE ET SON UTILISATION

(30) Priorität: 30.12.2002 DE 10261750
(43) Veröffentlichungstag der Anmeldung: 05.10.2005
(73) Patentinhaber: BASF Aktiengesellschaft, 67056 Ludwigshafen (DE)
(72) Erfinder: NGUYEN-KIM, Son, 69502 Hemsbach (DE); SCHUCH, Horst, 69126 Heidelberg (DE); KAISER, Thomas, 67454 Hassloch (DE); WOOD, Claudia, 69469 Weinheim (DE); HÖSSEL, Peter, 67105 Schifferstadt (DE)
(74) Vertreter: Reitstötter - Kinzebach
(86) Internationale Anmeldenummer: PCT/EP2003/014944
(87) Internationale Veröffentlichungsnummer: WO 2004/058837

(56) Entgegenhaltungen:
- WO-A-00/39176
- FR-A- 2 393 011

## Beschreibung

Die vorliegende Erfindung betrifft ein ampholytisches Copolymer, Polyelektrolytkomplexe, die ein solches ampholytisches Copolymer enthalten sowie kosmetische oder pharmazeutische Mittel, die wenigstens ein ampholytisches Copolymer oder einen Polyelektrolytkomplex enthalten.

Polymere mit einer größeren Anzahl ionisch dissoziierbarer Gruppen in der Hauptkette und/oder einer Seitenkette werden als Polyelektrolyte bezeichnet. Weisen diese Polymere sowohl anionogene/anionische als auch kationogene/kationische Gruppen auf, so handelt es sich um amphotere Polyelektrolyte bzw. ampholytische Polymere. Ein ionogenes bzw. ionisches Polymer kann mit einem gegensätzlich ladbaren bzw. geladenen Polymer unter Ausbildung eines Polyelektrolyt-Komplexes (Symplexes) reagieren. Ampholytische Polymere können dabei prinzipiell mit anionogenen/anionischen, kationogenen/kationischen und/oder wenigstens einem weiteren ampholytischen Polymer derartige Polyelektrolyt-Komplexe ausbilden. Polyelektrolyte mit ausreichender Anzahl dissoziierbarer Gruppen sind wasserlöslich oder wasserdispergierbar und haben vielfältige Anwendungen im Bereich der Anstrichmittel, Papierhilfsmittel, bei der Textilherstellung sowie speziell in der Pharmazie und Kosmetik gefunden.

Kosmetisch und pharmazeutisch akzeptable wasserlösliche Polymere dienen beispielsweise in Seifen, Cremes und Lotionen als Formulierungsmittel, z. B. als Verdicker, Schaumstabilisator oder Wasserabsorbens oder auch dazu, die reizende Wirkung anderer Inhaltsstoffe abzumildern oder die dermale Applikation von Wirkstoffen zu verbessern. Ihre Aufgabe in der Haarkosmetik besteht darin, die Eigenschaften des Haares zu beeinflussen. In der Pharmazie dienen sie beispielsweise als Beschichtungsmittel oder Bindemittel für feste Arzneiformen.

Für die Haarkosmetik werden filmbildende Polymere mit ionischen Gruppen beispielsweise als Conditioner dazu eingesetzt, um die Trocken- und Nasskämmbarkeit, das Anfassgefühl, den Glanz und die Erscheinungsform des Haars zu verbessern sowie dem Haar antistatische Eigenschaften zu verleihen. Struktur und Wirkungsweise verschiedener Haarbehandlungspolymere sind in Cosmetic & Toiletries 103 (1988) 23 beschrieben. Je nach Anwendungszweck werden beispielsweise wasserlösliche Polymere mit kationischen Funktionalitäten eingesetzt, die eine hohe Affinität zur strukturell bedingt negativ geladenen Oberfläche des Haares aufweisen. Handelsübliche kationische Conditionerpolymere sind z. B. kationische Hydroxyethylcellulose, kationische Polymere auf der Basis von N-Vinylpyrrolidon, z. B. Copolymere aus N-Vinylpyrrolidon und quarterniertem N-Vinylimidazol, Acrylamid und Diallyldimethylammoniumchlorid. Wasserlösliche Polymere mit anionischen Funktionalitäten, wie z. B. ggf. vernetzte Polyacrylsäure, dienen beispielsweise als Verdicker, weiterhin werden Carboxylatgruppenhaltige Polymere beispielsweise zur Fertigung von Haarfrisuren eingesetzt.

Schwierigkeiten bereitet oft die Bereitstellung von Produkten mit einem komplexen Eigenschaftsprofil. So besteht ein Bedarf an Polymeren für kosmetische Mittel, die zur Bildung im Wesentlichen glatter, klebfreier Filme befähigt sind, die dem Haar und der Haut einen angenehmen Griff verleihen und gleichzeitig eine gute Konditionierwirkung bzw. Festigungswirkung aufweisen. Anforderungen an Haarfestigerharze sind zum Beispiel eine starke Festigung bei hoher Luftfeuchtigkeit, Elastizität, Auswaschbarkeit vom Haar, Verträglichkeit in der Formulierung und ein angenehmer Griff des damit behandelten Haares. Zudem werden an kosmetische und pharmazeutische Produkte vom Verbraucher zunehmend ästhetische Anforderungen gestellt. So wird bei derartigen Produkten derzeit eine Bevorzugung von klaren, opaquen Formulierungen in Form von Gelen beobachtet. In vielen Fällen lässt sich das gewünschte Eigenschaftsprofil nur durch Einsatz mehrerer Polymere mit ionischen Gruppen erzielen. Dabei zeigt sich jedoch häufig eine Unverträglichkeit der verschiedenen Polymere miteinander, was beispielsweise zu einem unerwünschten Aussalzen führen kann. Es besteht daher Bedarf an kosmetisch und pharmazeutisch verträglichen Polyelektrolyten, die bei einem Einsatz als einzige Polymerkomponente geeignet sind, ein bestimmtes Eigenschaftsprofil bereitzustellen und/oder die mit einer Vielzahl verschiedener Polyelektrolyte verträglich sind.

Die EP-A-0 100 890 beschreibt Copolymerisate, erhalten durch radikalische Copolymerisation von
a) 20 bis 75 Gewichtsteilen mindestens eines C₂-C₂₀-Alkylesters der (Meth)acrylsäure,
b) 5 bis 50 Gewichtsteilen mindestens eines stickstoffhaltigen, neutral reagierenden wasserlöslichen Monomeren,
c) 1 bis 25 Gewichtsteilen mindestens eines kationische Gruppen enthaltenden Monomeren und
d) 1 bis 25 Gewichtsteilen mindestens einer mit a), b) und c) copolymerisierbaren olefinisch ungesättigten C₃-C₅-Carbonsäure.

Die WO 01/62809 beschreibt ein kosmetisches Mittel, das wenigstens ein wasserlösliches oder wasserdispergierbares Polymer enthält, das
a) 5 bis 50 Gew.-% wenigstens eines α,β-ethylenisch ungesättigten Monomers mit einer tert.-Butylgruppe,
b) 25 bis 90 Gew.-% wenigstens eines N-Vinylamids und/oder N-Vinyllactams,
c) 0,5 bis 30 Gew.-% wenigstens einer Verbindung mit einer radikalisch polymerisierbaren, α,β-ethylenisch ungesättigten Doppelbindung und mindestens einer kationogenen und/oder kationischen Gruppe pro Molekül, und
d) 0 bis 30 Gew.-% wenigstens einer weiteren α,β-ethylenisch ungesättigten Verbindung, wobei es sich um Verbindungen mit mindestens einer anionogenen und/oder anionischen Gruppe pro Molekül handeln kann,
eingebaut enthält.

Die EP-A-1038891 beschreibt wasserlösliche oder wasserdispergierbare polymere Salze aus wenigstens einem Polymer und wenigstens einem entgegengesetzt geladenen Neutralisationsmittel, wobei das Polymer und das Neutralisationsmittel jeweils nur eine Art ionischer Gruppen aufweist.

Die WO 00/39176 beschreibt ein hydrophiles kationisches ampholytisches Copolymer, das 0,05 bis 20 Mol-% eines anionischen Monomers mit wenigstens einer Carboxygruppe und 10 bis 45 Mol-% eines kationischen Monomers mit wenigstens einer Aminogruppe einpolymerisiert enthält, wobei das Molverhältnis von kationischem zu anionischem Monomer etwa 2:1 bis 16:1 beträgt. Diese ampholytischen Copolymere können unter Anderem zur Modifizierung der rheologischen Eigenschaften von Körperpflegemitteln eingesetzt werden. In den Ausführungsbeispielen werden ausschließlich Polymere auf Basis von Methacrylsäure und Dimethylaminopropylmethacrylamid sowie von Acrylsäure und Dimethylaminoethylmethacrylat eingesetzt.

Die FR 2 393 011 A beschreibt Herstellung und Verwendung amphoterer Polyelektrolyte, die durch Copolymerisation von vinylischen Monomeren erzeugt werden, als Additiv bei der Papierherstellung.

Der vorliegenden Erfindung liegt die Aufgabe zugrunde, neue kosmetische und pharmazeutische Mittel zur Verfügung zu stellen. Sie sollen geeignet sein, ein komplexes Anforderungsspektrum abzudekken und beispielsweise zur Bildung von klebfreien glatten Filmen befähigt sein, eine gute Festigungswirkung aufweisen und sich zur Herstellung von Produkten in Form von Gelen eignen oder mit möglichst vielen verschiedenen Polyelektrolyten für kosmetische und pharmazeutische Anwendungen verträglich sein.

Überraschenderweise wurde nun gefunden, dass diese Aufgabe durch ampholytische Copolymere gelöst wird, die wenigstens ein Monomer mit mindestens einer anionogenen und/oder anionischen Gruppe pro Molekül, wenigstens ein Monomer mit mindestens einer kationogenen und/oder kationischen Gruppe pro Molekül und wenigstens ein hydrophiles Monomer mit einer Amidgruppe eingebaut enthalten.

Gegenstand der Erfindung ist daher ein kosmetisches oder pharmazeutisches Mittel, enthaltend
A) wenigstens ein ampholytisches Copolymer, erhältlich durch radikalische Copolymerisation von
   a) wenigstens einer Verbindung mit einer radikalisch polymerisierbaren, α,β-ethylenisch ungesättigten Doppelbindung und mindestens einer anionogenen und/oder anionischen Gruppe pro Molekül,
   b) wenigstens einer Verbindung mit einer radikalisch polymerisierbaren, α,β-ethylenisch ungesättigten Doppelbindung und mindestens einer kationogenen und/oder kationischen Gruppe pro Molekül,
   c) wenigstens einer α,β-ethylenisch ungesättigten amidgruppenhaltigen Verbindung der allgemeinen Formel I 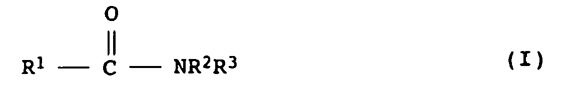
   worin
   einer der Rest R¹ bis R³ für eine Gruppe der Formel CH₂=CR⁴- mit R⁴ = H oder C₁-C₄-Alkyl steht und die übrigen Reste R¹ bis R³ unabhängig voneinander für H, Alkyl, Cycloalkyl, Heterocycloalkyl, Aryl oder Hetaryl stehen,
   wobei R¹ und R² gemeinsam mit der Amidgruppe, an die sie gebunden sind, auch für ein Lactam mit 5 bis 8 Ringatomen stehen können,
   wobei R² und R³ gemeinsam mit dem Stickstoffatom, an das sie gebunden sind, auch für einen fünf- bis siebengliedrigen Heterocyclus stehen können,
   mit der Maßgabe, dass die Summe der Kohlenstoffatome der Reste R¹, R² und R³ höchstens 8 beträgt,
   oder einen Polyelektrolytkomplex, enthaltend wenigstens ein solches ampholytisches Copolymer und wenigstens einen weiteren, davon verschiedenen Polyelektrolyten und
B) wenigstens einen kosmetisch akzeptablen Träger.

Ein weiterer Gegenstand der Erfindung ist ein ampholytisches Copolymer, erhältlich durch radikalische Copolymerisation von
a) wenigstens einer Verbindung mit einer radikalisch polymerisierbaren, α,β-ethylenisch ungesättigten Doppelbindung und mindestens einer anionogenen und/oder anionischen Gruppe pro Molekül,
b) wenigstens einer Verbindung mit einer radikalisch polymerisierbaren, α,β-ethylenisch ungesättigten Doppelbindung und mindestens einer kationogenen und/oder kationischen Gruppe pro Molekül,
c) wenigstens einer α,β-ethylenisch ungesättigten amidgruppenhaltigen Verbindung der allgemeinen Formel I 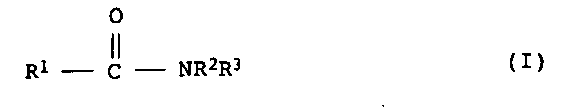 worin
   einer der Rest R¹ bis R³ für eine Gruppe der Formel CH₂=CR⁴- mit R⁴ = H oder C₁-C₄-Alkyl steht und die übrigen Reste R¹ bis R³ unabhängig voneinander für H, Alkyl, Cycloalkyl, Heterocycloalkyl, Aryl oder Hetaryl stehen,
   wobei R¹ und R² gemeinsam mit der Amidgruppe, an die sie gebunden sind, auch für ein Lactam mit 5 bis 8 Ringatomen stehen können,
   wobei R² und R³ gemeinsam mit dem Stickstoffatom, an das sie gebunden sind, auch für einen fünf- bis siebengliedrigen Heterocyclus stehen können,
mit der Maßgabe, dass die Summe der Kohlenstoffatome der Reste R¹, R² und R³ höchstens 8 beträgt, wobei die Komponente c) ausgewählt ist unter N-Vinylamiden gesättigter Monocarbonsäuren, N-Vinyllactamen, N-Alkyl- und N,N-Dialkylamiden α,β-ethylenisch ungesättigter Monocarbonsäuren und Mischungen davon.

Im Rahmen der vorliegenden Erfindung umfasst der Ausdruck Alkyl geradkettige und verzweigte Alkylgruppen. Geeignete kurzkettige Alkylgruppen sind z.B. geradkettige oder verzweigte C₁-C₇-Alkyl-, bevorzugt C₁-C₆-Alkyl- und besonders bevorzugt C₁-C₄-Alkylgruppen. Dazu zählen insbesondere Methyl, Ethyl, Propyl, Isopropyl, n-Butyl, 2-Butyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl- 2-methylpropyl, n-Heptyl, 2-Heptyl, 3-Heptyl, 2-Ethylpentyl, 1-Propylbutyl, Octyl etc.

Geeignete längerkettige C₈-C₃₀-Alkyl- bzw. C₈-C₃₀-Alkenylgruppen sind geradkettige und verzweigte Alkyl- bzw. Alkenylgruppen. Bevorzugt handelt es sich dabei um überwiegend lineare Alkylreste, wie sie auch in natürlichen oder synthetischen Fettsäuren und Fettalkoholen sowie Oxoalkoholen vorkommen, die gegebenenfalls zusätzlich einfach, zweifach oder mehrfach ungesättigt sein können. Dazu zählen z.B. n-Hexyl(en), n-Heptyl(en), n-Octyl(en), n-Nonyl(en), n-Decyl(en), n-Undecyl(en), n-Dodecyl(en), n-Tridecyl(en), n-Tetradecyl(en), n-Pentadecyl(en), n-Hexadecyl(en), n-Heptadecyl(en), n-Octadecyl(en), n-Nonadecyl(en) etc.

Cycloalkyl steht vorzugsweise für C₅-C₈-Cycloalkyl, wie Cyclopentyl, Cyclohexyl, Cycloheptyl oder Cyclooctyl.

Aryl umfasst unsubstituierte und substituierte Arylgruppen und steht vorzugsweise für Phenyl, Tolyl, Xylyl, Mesityl, Naphthyl, Fluorenyl, Anthracenyl, Phenanthrenyl, Naphthacenyl und insbesondere für Phenyl, Tolyl, Xylyl oder Mesityl.

Im Folgenden werden Verbindungen, die sich von Acrylsäure und Methacrylsäure ableiten können teilweise verkürzt durch Einfügen der Silbe "(meth)" in die von der Acrylsäure abgeleitete Verbindung bezeichnet.

Die erfindungsgemäß verwendeten oder erfindungsgemäßen ampholytischen Copolymere und Polyelektrolyt-Komplexe lassen sich unter Normalbedingungen (20 °C) vorteilhaft als Gele formulieren. "Gelförmige Konsistenz" zeigen Formulierungen, die eine höhere Viskosität als eine Flüssigkeit aufweisen und die selbsttragend sind, d. h. die eine ihnen verliehene Form ohne formstabilisierende Umhüllung behalten. Im Gegensatz zu festen Formulierungen lassen sich gelförmige Formulierungen jedoch leicht unter Anwendung von Scherkräften deformieren. Die Viskosität der gelförmigen Mittel liegen vorzugsweise in einem Bereich von größer als 600 bis etwa 60000 mPas. Vorzugsweise handelt es sich bei den Gelen um Haargele, wobei dies eine Viskosität von vorzugsweise 6000 bis 30000 mPas aufweisen.

Unter wasserlöslichen Monomeren und Polymeren werden im Rahmen der vorliegenden Erfindung Monomere und Polymere verstanden, die sich zu mindestens 1 g/l bei 20°C in Wasser lösen. Unter wasserdispergierbaren Monomeren und Polymeren werden Monomere und Polymere verstanden, die unter Anwendung von Scherkräften beispielsweise durch Rühren in dispergierbare Partikel zerfallen. Hydrophile Monomere sind vorzugsweise wasserlöslich oder zumindest wasserdispergierbar. Die erfindungsgemäß verwendeten oder erfindungsgemäßen Copolymere und Polyelektrolyt-Komplexe sind im Allgemeinen wasserlöslich oder zumindest wasserdispergierbar.

Die erfindungsgemäß verwendeten oder erfindungsgemäßen ampholytischen Copolymere weisen sowohl anionogene und/oder anionische Gruppen, als auch kationogene und/oder kationische Gruppen auf. Zu ihrer Herstellung werden vorzugsweise Monomere mit zunächst nichtgeladenen, das heißt mit anionogenen und kationogenen Gruppen eingesetzt. In einer geeigneten Ausführungsform erfolgt der Einsatz dieser Monomere gemeinsam, d. h. in Form so genannter "Salzpaare". Vorzugsweise wird dabei wenigstens ein Teil der Verbindungen a) und b) in Form einer Monomerzusammensetzung eingesetzt, wobei für diese Monomerzusammensetzung das molare Verhältnis von anionogenen und anionischen Gruppen der Komponente a) zu kationogenen und kationischen Gruppen der Komponente b) etwa 1:1 beträgt.

Gewünschtenfalls können zur Herstellung der erfindungsgemäß verwendeten oder erfindungsgemäßen ampholytischen Copolymere anstelle von ungeladenen Monomeren oder zusätzlich zu ungeladenen Monomeren oder zusätzlich zu Salzpaaren auch bereits geladene Monomere, das heißt Monomere mit anionischen und kationischen Gruppen eingesetzt werden. Die Gegenionen, die diese Monomere tragen, leiten sich dann vorzugsweise von Säuren oder Basen ab, wie sie im Folgenden zur Einstellung des pH-Werts bei der Polymerisation oder der erhaltenen Polymerisate beschrieben werden. Kationische Monomere können zudem in teilweise oder vollständig quaternisierter Form eingesetzt werden.

Bevorzugt liegt das Molmengenverhältnis von Verbindungen a) zu Verbindungen b) (d. h. von anionogenen/anionischen Verbindungen zu kationogenen/kationischen Verbindungen) in einem Bereich von 0,5:1 bis weniger als 2:1 und insbesondere in einem Bereich von 0,7:1 bis 1,8:1.

In einer geeigneten Ausführungsform sind die erfindungsgemäß verwendeten oder erfindungsgemäßen ampholytischen Copolymere nach außen hin im Wesentlichen elektroneutral. Derartige Copolymere weisen an das Polymergerüst gebundene anionische und kationische Gruppen in solchen Mengenverhältnissen auf, dass sich positive und negative Ladungen im Wesentlichen kompensieren. Vorzugsweise liegt das Verhältnis von positiven zu negativen Ladungsäquivalenten in einem Bereich von 0,8:1 bis 1:0,8, besonders bevorzugt 0,9:1 bis 1:0,9 und speziell 0,95:1 bis 1:0,95.

Der pH-Wert einer 0,1 molaren wässrigen Lösung der erfindungsgemäß verwendeten oder erfindungsgemäßen wasserlöslichen ampholytischen Copolymere liegt bei einer Temperatur von 20°C vorzugsweise in einem Bereich von 5,5 bis 8,0, besonders bevorzugt von 5,6 bis 7,5 und insbesondere von 5,8 bis 7,3. Da die erfindungsgemäßen ampholytischen Copolymere im Allgemeinen als Puffer wirken sind die pH-Werte ihrer wässrigen Lösungen in der Regel in einem weitem Bereich relativ stabil gegen Verdünnung und Säure- oder Basenzusatz.

Bei Herstellung der erfindungsgemäß verwendeten oder erfindungsgemäßen ampholytischen Copolymere durch radikalische Copolymerisation in einem wässrigen Medium liegt der pH-Wert vorzugsweise in einem Bereich von 5,5 bis 9,0, besonders bevorzugt von 5,6 bis 8,5 und insbesondere von 5,8 bis 7,5. Die Einstellung des pH-Werts kann zum einen durch geeignete Auswahl der zur Copolymerisation eingesetzten Monomere mit anionogenen und kationogenen Gruppen erfolgen. Des Weiteren kann die Einstellung des pH auf einen gewünschten Wert auch durch Zugabe wenigstens einer Säure oder wenigstens einer Base erfolgen.

Bei Herstellung der erfindungsgemäß verwendeten oder erfindungsgemäßen ampholytischen Copolymere durch radikalische Copolymerisation in nichtwässrigen Medien, beispielsweise durch Fällungspolymerisation, erfolgt die Auswahl der Monomere ebenfalls vorzugsweise so, dass der pH-Wert einer entsprechenden wässrigen Lösung in einem Bereich von 5,5 bis 8,0, besonders bevorzugt von 5,6 bis 7,5 und insbesondere von 5,8 bis 7,3 liegt oder wird eine Säure oder Base zugesetzt, um den pH-Wert einer entsprechenden wässrigen Lösung auf einen Wert in diesem Bereich einzustellen.

Die Einstellung des pH-Wertes erfolgt durch Zugabe wenigstens einer geeigneten Säure, z.B. einer Carbonsäure, wie Milchsäure oder Weinsäure, oder einer Mineralsäure, wie Phosphorsäure, Schwefelsäure oder Salzsäure oder durch Zugabe wenigstens einer geeigneten Base, vorzugsweise eines Alkalihydroxids, wie NaOH oder KOH, Ammoniak oder eines Amins, wie Triethylamin und insbesondere eines Aminoalkohols, wie Triethanolamin, Methyldiethanolamin, Dimethylethanolamin oder 2-Amino-2-methylpropanol.

Das erfindungsgemäß verwendete oder erfindungsgemäße ampholytische Copolymer enthält vorzugsweise 0,1 bis 25 Gew.-%, besonders bevorzugt 0,5 bis 20 Gew.-%, bezogen auf das Gesamtgewicht der zur Polymerisation eingesetzten Komponenten, wenigstens einer Verbindung a) einpolymerisiert.

Vorzugsweise sind die Verbindungen a) ausgewählt unter monoethylenisch ungesättigten Carbonsäuren, Sulfonsäuren, Phosphonsäuren und Mischungen davon.

Zu den Monomeren a) zählen monoethylenisch ungesättigte Mono- und Dicarbonsäuren mit 3 bis 25 vorzugsweise 3 bis 6 C-Atomen, die auch in Form ihrer Salze oder Anhydride eingesetzt werden können. Beispiele hierfür sind Acrylsäure, Methacrylsäure, Ethacrylsäure, α-Chloracrylsäure, Crotonsäure, Maleinsäure, Maleinsäureanhydrid, Itaconsäure, Citraconsäure, Mesaconsäure, Glutaconsäure, Aconitsäure und Fumarsäure. Zu den Monomeren a) zählen weiterhin die Halbester von monoethylenisch ungesättigten Dicarbonsäuren mit 4 bis 10 vorzugsweise 4 bis 6 C-Atomen, z. B. von Maleinsäure wie Maleinsäuremonomethylester. Zu den Monomeren a) zählen auch monoethylenisch ungesättigte Sulfonsäuren und Phosphonsäuren, beispielsweise Vinylsulfonsäure, Allylsulfonsäure, Sulfoethylacrylat, Sulfoethylmethacrylat, Sulfopropylacrylat, Sulfopropylmethacrylat, 2-Hydroxy-3-acryloxypropylsulfonsäure, 2-Hydroxy-3-methacryloxypropylsulfonsäure, Styrolsulfonsäure, 2-Acrylamido-2-methylpropansulfonsäure, Vinylphosphonsäure und Allylphosphonsäure. Zu den Monomeren a) zählen auch die Salze der zuvor genannten Säuren, insbesondere die Natrium-, Kalium- und Ammoniumsalze sowie die Salze mit den zuvor genannten Aminen. Die Monomere a) können als solche oder als Mischungen untereinander eingesetzt werden. Die angegebenen Gewichtsanteile beziehen sich sämtlich auf die Säureform.

Vorzugsweise ist die Komponente a) ausgewählt unter Acrylsäure, Methacrylsäure, Ethacrylsäure, α-Chloracrylsäure, Crotonsäure, Maleinsäure, Maleinsäureanhydrid, Fumarsäure, Itaconsäure, Citraconsäure, Mesaconsäure, Glutaconsäure, Aconitsäure und Mischungen davon.

Besonders bevorzugt ist die Komponente a) ausgewählt unter Acrylsäure, Methacrylsäure, Itaconsäure und Mischungen davon.

Des Weiteren besonders bevorzugt ist die Komponente a) ausgewählt unter 2-Acrylamido-2-methyl-1-propansulfonsäure, Styrolsulfonsäure, Vinylsulfonsäure, Vinylphosphonsäure, Mischungen davon und Mischungen mit den zuvor genannten Monomeren a). Insbesondere wird 2-Acrylamido-2-methylpropansulfonsäure als alleinige Verbindung der Komponente a) oder werden Mischungen, die 2-Acrylamido-2-methylpropansulfonsäure enthalten, eingesetzt.

Das ampholytische Copolymer enthält vorzugsweise 0,1 bis 40 Gew.-%, besonders bevorzugt 0,5 bis 35 Gew.-%, insbesondere 1 bis 30 Gew.-%, bezogen auf das Gesamtgewicht der zur Polymerisation eingesetzten Komponenten, wenigstens eine Verbindung der Komponente b) einpolymerisiert.

Bevorzugt handelt es sich bei den kationogenen bzw. kationischen Gruppen der Komponente b) um stickstoffhaltige Gruppen, wie primäre, sekundäre und tertiäre Aminogruppen sowie quaternäre Ammoniumgruppen. Vorzugsweise handelt es sich bei den stickstoffhaltigen Gruppen um tertiäre Aminogruppen oder quaternäre Ammoniumgruppen. Geladene kationische Gruppen lassen sich aus den Aminstickstoffen entweder durch Protonierung, z. B. mit einwertigen oder mehrwertigen Carbonsäuren, wie Milchsäure oder Weinsäure, oder Mineralsäuren, wie Phosphorsäure, Schwefelsäure und Salzsäure, oder durch Quaternisierung, z. B. mit Alkylierungsmitteln, wie C₁- bis C₄-Alkylhalogeniden oder -sulfaten, erzeugen. Beispiele solcher Alkylierungsmittel sind Ethylchlorid, Ethylbromid, Methylchlorid, Methylbromid, Dimethylsulfat und Diethylsulfat.

Geeignete Verbindungen b) sind z. B. die Ester von α,β-ethylenisch ungesättigten Mono- und Dicarbonsäuren mit Aminoalkoholen. Bevorzugte Aminoalkohole sind C₂- bis C₁₂-Aminoalkohole welche am Aminstickstoff C₁- bis C₈-dialkyliert sind. Als Säurekomponente dieser Ester eignen sich z. B. Acrylsäure, Methacrylsäure, Fumarsäure, Maleinsäure, Itaconsäure, Crotonsäure, Maleinsäureanhydrid, Monobutylmaleat und Gemische davon. Bevorzugt werden Acrylsäure, Methacrylsäure und deren Gemische eingesetzt. Bevorzugt sind N,N-Dimethylaminomethyl(meth)acrylat, N,N-Dimethylaminoethyl(meth)acrylat, N,N-Diethylaminoethyl(meth)acrylat, N,N-Dimethylaminopropyl(meth)acrylat, N,N-Diethylaminopropyl(meth)acrylat und N,N-Dimethylaminocyclohexyl(meth)acrylat.

Geeignete Monomere b) sind weiterhin die Amide der zuvor genannten α,β-ethylenisch ungesättigten Mono- und Dicarbonsäuren mit Diaminen, welche mindestens eine primäre oder sekundäre Aminogruppe aufweisen. Bevorzugt sind Diamine, die eine tertiäre und eine primäre oder sekundäre Aminogruppe aufweisen. Bevorzugt werden als Monomere b) N-[2-(dimethylamino)ethyl]acrylamid,
N-[2-(dimethylamino)ethyl]methacrylamid,
N-[3-(dimethylamino)propyl]acrylamid,
N-[3-(dimethylamino)propyl]methacrylamid,
N-[4-(dimethylamino)butyl]acrylamid,
N-[4-(dimethylamino)-butyl]methacrylamid,
N-[2-(diethylamino)ethyl]acrylamid,
N-[4-(dimethylamino)cyclohexyl]acrylamid,
N-[4-(dimethylamino)cyclohexyl]methacrylamid etc eingesetzt. Besonders bevorzugt werden N-[3-(dimethyl-amino)propyl]acrylamid und/oder N-[3-(dimethylamino)propyl]methacrylamid eingesetzt.

Geeignete Monomere b) sind weiterhin N,N-Diallylamine und N,N-Diallyl-N-alkylamine und deren Säureadditionssalze und Quaternisierungsprodukte. Alkyl steht dabei vorzugsweise für C₁- bis C₂₄-Alkyl. Bevorzugt sind N,N-Diallyl-N-methylamin und N,N-Diallyl-N,N-dimethylammonium-Verbindungen, wie z. B. die Chloride und Bromide.

Geeignete Monomere b) sind weiterhin vinyl- und allylsubstituierte Stickstoffheterocyclen, wie N-Vinylimidazol, N-Vinyl-2-methylimidazol, vinyl- und allylsubstituierte heteroaromatische Verbindungen, wie 2- und 4-Vinylpyridin, 2- und 4-Allylpyridin, und die Salze davon.

Bevorzugt ist die Komponente b) ausgewählt unter N,N-Dimethylaminoethyl(meth)acrylat, N,N-Dimethylaminopropyl(meth)acrylat, Vinylimidazol und Mischungen davon.

Bevorzugte Kombinationen der Komponenten a) und b), die z. B. als Salzpaar zur radikalischen Copolymerisation eingesetzt werden können, sind (Meth)acrylsäure/N,N-Dimethylaminopropyl(meth)acrylat und (Meth)acrylsäure/Vinylimidazol.

Weitere bevorzugte Kombinationen der Komponenten a) und b) sind:
- 2-Acrylamido-2-methylpropansulfonsäure und
- wenigstens ein Monomer, ausgewählt unter
   N-[3-(dimethylamino)propyl]methacrylamid, Vinylimidazol, N-(tert.-Butyl)aminoethyl(meth)acrylat, N,N-Diallylamin, N,N-Diallyl-N-methylamin und Mischungen davon.

Weitere bevorzugte Kombinationen der Komponenten a) und b) sind:
- Styrolsulfonsäure und
- wenigstens ein Monomer, ausgewählt unter
   N-[3-(dimethylamino)propyl]methacrylamid, Vinylimidazol, N-(tert.-Butyl)aminoethyl(meth)acrylat, N,N-Diallylamin, N,N-Diallyl-N-methylamin und Mischungen davon.

Weitere bevorzugte Kombinationen der Komponenten a) und b) sind:
- Vinylsulfonsäure und
- wenigstens ein Monomer, ausgewählt unter
   N-[3-(dimethylamino)propyl]methacrylamid, Vinylimidazol, N-(tert.-Butyl)aminoethyl(meth)acrylat, N,N-Diallylamin, N,N-Diallyl-N-methylamin und Mischungen davon.

Weitere bevorzugte Kombinationen der Komponenten a) und b) sind:
- Vinylphosphonsäure und
- wenigstens ein Monomer, ausgewählt unter
   N-[3-(dimethylamino)propyl]methacrylamid, Vinylimidazol, N-(tert.-Butyl)aminoethyl(meth)acrylat, N,N-Diallylamin, N,N-Diallyl-N-methylamin und Mischungen davon.

Das erfindungsgemäß verwendete oder erfindungsgemäße ampholytische Copolymer enthält vorzugsweise 40 bis 99,8 Gew.-%, besonders bevorzugt 45 bis 99 Gew.-%, insbesondere 50 bis 98 Gew.-%, bezogen auf das Gesamtgewicht der zur Polymerisation eingesetzten Komponenten, wenigstens einer Verbindung c) einpolymerisiert.

Vorzugsweise weisen die Verbindungen der Komponente c) zusätzlich zu dem Carbonyl-Kohlenstoffatom der Amidgruppe höchstens 7 weitere Kohlenstoffatome auf.

Bevorzugt sind die Verbindungen der Komponente c) ausgewählt unter primären Amiden α,β-ethylenisch ungesättigter Monocarbonsäuren, N-Vinylamiden gesättigter Monocarbonsäuren, N-Vinyllactamen, N-Alkyl- und N,N-Dialkylamiden α,β-ethylenisch ungesättigter Monocarbonsäuren und Mischungen davon.

Geeignete N-Alkyl- und N,N-Dialkylamide α,β-ethylenisch ungesättigter Monocarbonsäuren, die zusätzlich zu dem Carbonyl-Kohlenstoffatom der Amidgruppe höchstens 8 weitere Kohlenstoffatome aufweisen, sind beispielsweise N-Methyl(meth)acrylamid, N-Ethyl(meth)acrylamid, N-Propyl(meth)acrylamid, N-(n-Butyl)(meth)acrylamid, N-tert.-Butyl(meth)acrylamid, N,N-Dimethyl(meth)acrylamid, N,N-Diethyl(meth)acrylamid, Piperidinyl(meth)acrylamid, Morpholinyl(meth)acrylamid und Mischungen davon.

Besonders bevorzugt sind die Verbindungen der Komponente c) ausgewählt unter Acrylsäureamid, Methacrylsäureamid, N-Vinylpyrrolidon, N-Vinylcaprolactam, N-Vinylformamid, N-Vinylacetamid und Mischungen davon.

Die erfindungsgemäße verwendeten oder erfindungsgemäßen wasserlöslichen oder wasserdispergierbaren Copolymere können gewünschtenfalls bis zu 20 Gew.-%, bevorzugt bis zu 15 Gew.-%, besonders bevorzugt 0,1 bis 10 Gew.-%, wenigstens eines weiteren Monomers d) einpolymerisiert enthalten. Vorzugsweise sind diese zusätzlichen Monomere ausgewählt unter Estern α,β-ethylenisch ungesättigter Mono- und Dicarbonsäuren mit C₁-C₃₀-Alkanolen und C₁-C₃₀-Alkandiolen, Amiden α,β-ethylenisch ungesättigter Mono- und Dicarbonsäuren mit C₂-C₃₀-Aminoalkoholen, die eine primäre oder sekundäre Aminogruppe aufweisen, N-Alkyl- und N,N-Dialkylamiden α,β-ethylenisch ungesättigter Monocarbonsäuren, die zusätzlich zu dem Carbonyl-Kohlenstoffatom der Amidgruppe mehr als 8 weitere Kohlenstoffatome aufweisen, Estern von Vinylalkohol und Allylalkohol mit C₁-C₃₀-Monocarbonsäuren, Vinylethern, Vinylaromaten, Vinylhalogeniden, Vinylidenhalogeniden, C₁-C₈-Monoolefinen, nicht aromatischen Kohlenwasserstoffen mit mindestens zwei konjugierten Doppelbindungen, Siloxanmacromeren und Mischungen davon.

Bevorzugt als Monomere d) sind Ester α,β-ethylenisch ungesättigter Mono- und Dicarbonsäuren mit C₈-C₃₀-Alkanolen und C₈-C₃₀-Alkandiolen, besonders bevorzugt mit C₁₂-C₂₂-Alkanolen und C₁₂-C₂₂-Alkandiolen.

Bevorzugt ist das Monomer d) ausgewählt unter Methyl(meth)acrylat, Methylethacrylat, Ethyl(meth)acrylat, Ethylethacrylat, tert.-Butylethacrylat, n-Octyl(meth)acrylat, 1,1,3,3-Tetramethylbutyl(meth)acrylat, Ethylhexyl(meth)acrylat, n-Nonyl(meth)acrylat, n-Decyl(meth)acrylat, n-Undecyl(meth)acrylat, Tridecyl(meth)acrylat, Myristyl(meth)acrylat, Pentadecyl(meth)acrylat, Palmityl(meth)acrylat, Heptadecyl(meth)acrylat, Nonadecyl(meth)acrylat, Arrachinyl(meth)acrylat, Behenyl(meth)acrylat, Lignocerenyl(meth)acrylat, Cerotinyl(meth)acrylat, Melissinyl(meth)acrylat, Palmitoleinyl(meth)acrylat, Oleyl(meth)acrylat, Linolyl(meth)acrylat, Linolenyl(meth)acrylat, Stearyl(meth)acrylat, Lauryl(meth)acrylat, tert.-Butyl(meth)acrylamid, n-Octyl(meth)acrylamid, 1,1,3,3-Tetramethylbutyl(meth)acrylamid, Ethylhexyl(meth)acrylamid, n-Nonyl(meth)acrylamid, n-Decyl(meth)acrylamid, n-Undecyl(meth)acrylamid, Tridecyl(meth)acrylamid, Myristyl(meth)acrylamid, Pentadecyl(meth)acrylamid, Palmityl(meth)acrylamid, Heptadecyl(meth)acrylamid, Nonadecyl(meth)acrylamid, Arrachinyl(meth)acrylamid, Behenyl(meth)acrylamid, Lignocerenyl(meth)acrylamid, Cerotinyl(meth)acrylamid, Melissinyl(meth)acrylamid, Palmitoleinyl(meth)acrylamid, Oleyl(meth)acrylamid, Linolyl(meth)acrylamid, Linolenyl(meth)acrylamid, Stearyl(meth)acrylamid, Lauryl(meth)acrylamid und Mischungen davon. Besonders bevorzugt sind die Ester α,β-ethylenisch ungesättigter Mono- und Dicarbonsäuren mit linearen C₁-C₃₀-Alkanolen.

Bevorzugte Monomere d) sind weiterhin 2-Hydroxyethylacrylat, 2-Hydroxyethylmethacrylat, 2-Hydroxyethylethacrylat, 2-Hydroxypropylacrylat, 2-Hydroxypropylmethacrylat, 3-Hydroxypropylacrylat, 3-Hydroxypropylmethacrylat, 3-Hydroxybutylacrylat, 3-Hydroxybutylmethacrylat, 4-Hydroxybutylacrylat, 4-Hydroxybutylmethacrylat, 6-Hydroxyhexylacrylat, 6-Hydroxyhexylmethacrylat, 3-Hydroxy-2-ethylhexylacrylat, 3-Hydroxy-2-ethylhexylmethacrylat etc.

Die erfindungsgemäß verwendeten oder erfindungsgemäßen ampholytischen Copolymere können zusätzlich ein von den Komponeneten a) bis d) verschiedenes, damit copolymerisierbares oberflächenaktives Monomer e) einpolymerisiert enthalten.

Geeignete Monomere e) sind Polyetheracrylate, worunter im Rahmen dieser Erfindung allgemein Ester α,β-ethylenisch ungesättigter Mono- und Dicarbonsäuren mit Polyetherolen verstanden werden. Geeignete Polyetherole sind lineare oder verzweigte, endständige Hydroxylgruppen aufweisende Substanzen, die Etherbindungen enthalten. Im Allgemeinen weisen sie ein Molekulargewicht im Bereich von etwa 150 bis 20000 auf. Geeignete Polyetherole sind Polyalkylenglykole, wie Polyethylenglykole, Polypropylenglykole, Polytetrahydrofurane und Alkylenoxidcopolymere. Geeignete Alkylenoxide zur Herstellung von Alkylenoxidcopolymeren sind z. B. Ethylenoxid, Propylenoxid, Epichlorhydrin, 1,2- und 2,3-Butylenoxid. Die Alkylenoxidcopolymere können die Alkylenoxideinheiten statistisch verteilt oder in Form von Blöcken einpolymerisiert enthalten. Bevorzugt sind Ethylenoxid/Propylenoxid-Copolymere.

Bevorzugt als Komponente e) sind Polyetheracrylate der allgemeinen Formel II 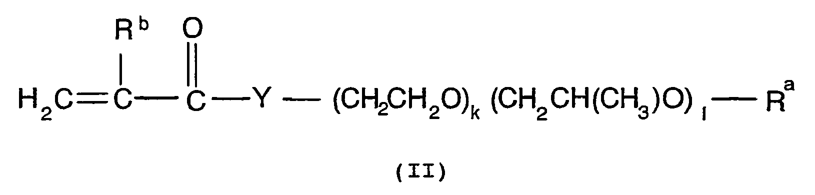 worin
die Reihenfolge der Alkylenoxideinheiten beliebig ist,
- k und l: unabhängig voneinander für eine ganze Zahl von 0 bis 1000 stehen, wobei die Summe aus k und 1 mindestens 5 beträgt,
- R^{a}: für Wasserstoff, C₁-C₃₀-Alkyl oder C₅-C₈-Cycloalkyl steht,
- R^{b}: für Wasserstoff oder C₁-C₈-Alkyl steht,
- Y: für O oder NR^{b} steht, wobei R^{b} für Wasserstoff, C₁-C₃₀-Alkyl oder C₅-C₈-Cycloalkyl steht,

Bevorzugt steht k für eine ganze Zahl von 1 bis 500, insbesondere 3 bis 250. Bevorzugt steht 1 für eine ganze Zahl von 0 bis 100.

Bevorzugt steht R^{b} für Wasserstoff, Methyl, Ethyl, n-Propyl, Isopropyl, n-Butyl, sec.-Butyl, tert.-Butyl, n-Pentyl oder n-Hexyl, insbesondere für Wasserstoff, Methyl oder Ethyl.

Vorzugsweise steht R^{a} in der Formel II für C₈-C₃₀-Alkyl, insbesondere für C₁₂-C₃₀-Alkyl, wie Decyl, Undecyl, Tridecyl, Myristyl, Pentadecyl, Palmityl, Lauryl, Stearyl; etc.

Vorzugsweise steht Y in der Formel II für O oder NH.

Geeignete Polyetheracrylate e) sind z. B. die Polykondensationsprodukte der zuvor genannten α,β-ethylenisch ungesättigten Mono- und/oder Dicarbonsäuren und deren Säurechloriden, -amiden und Anhydriden mit Polyetherolen. Geeignete Polyetherole können leicht durch Umsetzung von Ethylenoxid, 1,2-Propylenoxid und/oder Epichlorhydrin mit einem Startermolekül, wie Wasser oder einem kurzkettigen Alkohol R^{a}-OH hergestellt werden. Die Alkylenoxide können einzeln, alternierend nacheinander oder als Mischung eingesetzt werden. Geeignete Polyetheracrylate e) lassen sich auch durch Umesterung der zuvor als Komponente d) beschriebenen Ester α,β-ethylenisch ungesättigter Mono- und Dicarbonsäuren mit Polyetherolen herstellen. Dabei resultieren in der Regel Produktgemische, die sowohl die als Edukte eingesetzten Ester als auch die durch Umesterung gebildeten Polyetheracrylate enthalten. Diese Gemische können im Allgemeinen ohne vorherige Auftrennung zur Herstellung der erfindungsgemäß verwendeten oder erfindungsgemäßen ampholytischen Copolymere eingesetzt werden. Die Polyetheracrylate e) können allein oder in Mischungen zur Herstellung der erfindungsgemäß eingesetzten Polymere verwendet werden.

Die erfindungsgemäß verwendeten oder erfindungsgemäßen ampholytischen Copolymere enthalten vorzugsweise bis zu 25 Gew.-%, besonders bevorzugt bis zu 20 Gew.-%, insbesondere bis zu 15 Gew.-%, bezogen auf das Gesamtgewicht der zur Polymerisation eingesetzten Monomere, wenigstens eines Monomers e) einpolymerisiert. Wenn ein Monomer e) eingesetzt wird, dann vorzugsweise in einer Menge von mindestens 0,1 Gew.-%, besonders bevorzugt mindestens 1 Gew.-% und insbesondere wenigstens 5 Gew.-%.

Die erfindungsgemäß verwendeten oder erfindungsgemäßen ampholytischen Copolymere können gewünschtenfalls wenigstens einen Vernetzer f), d. h. eine Verbindung mit zwei oder mehr als zwei ethylenisch ungesättigten Doppelbindungen einpolymerisiert enthalten. Vorzugsweise werden Vernetzer f) in einer Menge von 0,01 bis 10 Gew.-%, besonders bevorzugt 0,03 bis 3 Gew.-%, insbesondere 0,1 bis 1 Gew.-%, bezogen auf das Gesamtgewicht der zur Polymerisation verwendeten Komponenten eingesetzt.

Als vernetzende Monomere f) können Verbindungen mit mindestens zwei ethylenisch ungesättigten Doppelbindungen eingesetzt werden, wie zum Beispiel Ester von ethylenisch ungesättigten Carbonsäuren, wie Acrylsäure oder Methacrylsäure und mehrwertigen Alkoholen, Ether von mindestens zweiwertigen Alkoholen, wie zum Beispiel Vinylether oder Allylether.

Beispiele für die zu Grunde liegenden Alkohole sind zweiwertige Alkohole wie 1,2-Ethandiol, 1,2-Propandiol, 1,3-Propandiol, 1,2-Butandiol, 1,3-Butandiol, 2,3-Butandiol, 1,4-Butandiol, But-2-en-1,4-diol, 1,2-Pentandiol, 1,5-Pentandiol, 1,2-Hexandiol, 1,6-Hexandiol, 1,10-Decandiol, 1,2-Dodecandiol, 1,12-Dodecandiol, Neopentylglykol, 3-Methylpentan-1,5-diol, 2,5-Dimethyl-1,3-hexandiol, 2,2,4-Trimethyl-1,3-pentandiol, 1,2-Cyclohexandiol, 1,4-Cyclohexandiol, 1,4-Bis(hydroxymethyl)cyclohexan, Hydroxypivalinsäure-neopentylglycolmonoester, 2,2-Bis(4-hydroxyphenyl)-propan, 2,2-Bis[4-(2-hydroxypropyl)phenyl]propan, Diethylenglykol, Triethylenglykol, Tetraethylenglykol, Dipropylenglykol, Tripropylenglykol, Tetrapropylenglykol, 3-Thio-pentan-1,5-diol, sowie Polyethylenglykole, Polypropylenglykole und Polytetrahydrofurane mit Molekulargewichten von jeweils 200 bis 10000. Außer den Homopolymerisaten des Ethylenoxids bzw. Propylenoxids können auch Blockcopolymerisate aus Ethylenoxid oder Propylenoxid oder Copolymerisate, die Ethylenoxid- und Propylenoxid-Gruppen eingebaut enthalten, eingesetzt werden. Beispiele für zu Grunde liegende Alkohole mit mehr als zwei OH-Gruppen sind Trimethylolpropan, Glycerin, Pentaerythrit, 1,2,5-Pentantriol, 1,2,6-Hexantriol, Triethoxycyanursäure, Sorbitan, Zucker wie Saccharose, Glucose, Mannose. Selbstverständlich können die mehrwertigen Alkohole auch nach Umsetzung mit Ethylenoxid oder Propylenoxid als die entsprechenden Ethoxylate bzw. Propoxylate eingesetzt werden. Die mehrwertigen Alkohole können auch zunächst durch Umsetzung mit Epichlorhydrin in die entsprechenden Glycidylether überführt werden.

Weitere geeignete Vernetzer f) sind die Vinylester oder die Ester einwertiger, ungesättigter Alkohole mit ethylenisch ungesättigten C₃-C₆-Carbonsäuren, beispielsweise Acrylsäure, Methacrylsäure, Itaconsäure, Maleinsäure oder Fumarsäure. Beispiele für solche Alkohole sind Allylalkohol, 1-Buten-3-ol, 5-Hexen-1-ol, 1-0c-ten-3-ol, 9-Decen-1-ol, Dicyclopentenylalkohol, 10-Undecen-1-ol, Zimtalkohol, Citronellol, Crotylalkohol oder cis-9-Octa-decen-1-ol. Man kann aber auch die einwertigen, ungesättigten Alkohole mit mehrwertigen Carbonsäuren verestern, beispielsweise Malonsäure, Weinsäure, Trimellitsäure, Phthalsäure, Terephthalsäure, Citronensäure oder Bernsteinsäure.

Weitere geeignete Vernetzer f) sind Ester ungesättigter Carbonsäuren mit den oben beschriebenen mehrwertigen Alkoholen, beispielsweise der Ölsäure, Crotonsäure, Zimtsäure oder 10-Undecensäure.

Außerdem geeignet sind geradkettige oder verzweigte, lineare oder cyclische aliphatische oder aromatische Kohlenwasserstoffe, die über mindestens zwei Doppelbindungen verfügen, welche bei den aliphatischen Kohlenwasserstoffen nicht konjugiert sein dürfen, z. B. Divinylbenzol, Divinyltoluol, 1,7-Octadien, 1,9-Decadien, 4-Vinyl-1-cyclohexen, Trivinylcyclohexan oder Polybutadiene mit Molekulargewichten von 200 bis 20000.

Ferner geeignet sind Amide von ungesättigten Carbonsäuren, wie z. B., Acryl- und Methacrylsäure, Itaconsäure, Maleinsäure, und N-Allylaminen von mindestens zweiwertigen Aminen, wie zum Beispiel 1,2-Diaminomethan, 1,2-Diaminoethan, 1,3-Diaminopropan, 1,4-Diaminobutan, 1,6-Diaminohexan, 1,12-Dodecandiamin, Piperazin, Diethylentriamin oder Isophorondiamin. Ebenfalls geeignet sind die Amide aus Allylamin und ungesättigten Carbonsäuren wie Acrylsäure, Methacrylsäure, Itaconsäure, Maleinsäure, oder mindestens zweiwertigen Carbonsäuren, wie sie oben beschrieben wurden.

Ferner sind Triallylamin oder entsprechende Ammoniumsalze, z. B. Triallylmethylammoniumchlorid oder -methylsulfat, als Vernetzer geeignet.

Weiterhin können N-Vinylverbindungen von Harnstoffderivaten, mindestens zweiwertigen Amiden, Cyanuraten oder Urethanen, beispielsweise von Harnstoff, Ethylenharnstoff, Propylenharnstoff oder Weinsäurediamid, z. B. N,N'-Divinylethylenharnstoff oder N,N'-Divinylpropylenharnstoff eingesetzt werden.

Weitere geeignete Vernetzer f) sind Divinyldioxan, Tetraallylsilan oder Tetravinylsilan.

Besonders bevorzugte Vernetzer sind beispielsweise Methylenbisacrylamid, Divinylbenzol, Triallylamin und Triallylammoniumsalze, Divinylimidazol, N,N'-Divinylethylenharnstoff, Umsetzungsprodukte mehrwertiger Alkohole mit Acrylsäure oder Methacrylsäure, Methacrylsäureester und Acrylsäureester von Polyalkylenoxiden oder mehrwertigen Alkoholen, die mit Ethylenoxid und/oder Propylenoxid und/oder Epichlorhydrin umgesetzt worden sind, sowie Allyl- oder Vinylether von mehrwertigen Alkoholen, beispielsweise 1,2-Ethandiol, 1,4-Butandiol, Diethylenglykol, Trimethylolpropan, Glycerin, Pentaerythrit, Sorbitan und Zucker wie Saccharose, Glucose, Mannose.

Besonders bevorzugt als Vernetzer f) sind Pentaerythrittriallylether, Allylether von Zuckern wie Saccharose, Glucose, Mannose, Divinylbenzol, N,N'-Methylenbisacrylamid, N,N'-Divinylethylenharnstoff, und (Meth-)Acrylsäureester von Glykol, Butandiol, Trimethylolpropan oder Glycerin oder (Meth)Acrylsäureester von mit Ethylenoxid und/oder Epichlorhydrin umgesetzten Glykol, Butandiol, Trimethylolpropan oder Glycerin. Ganz besonders bevorzugt sind N,N'-Methylenbisacrylamid, Diallylweinsäurediamid, Diallylphthalat, Diallylharnstoff, Glycoldi(meth)acrylat, Allyl(meth)acrylat sowie Polyallylether.

Nach einer geeigneten Ausführungsform erfolgt die Copolymerisation zur Herstellung der erfindungsgemäß verwendeten oder erfindungsgemäßen ampholytischen Copolymere in Gegenwart wenigstens einer Verbindung der Komponente g), die ausgewählt ist unter
g1) polyetherhaltigen Verbindungen,
g2) Polymeren, die mindestens 50 Gew.-% Wiederholungseinheiten aufweisen, die sich von Vinylalkohol ableiten,
g3) Cellulose, Stärke und Derivaten davon,
und Mischungen davon.

Erfolgt die radikalische Copolymerisation der Komponenten in Gegenwart wenigstens einer Verbindung der Komponente g) werden ampholytische Copolymere mit vorteilhaften Eigenschaften erhalten. Dies kann beispielsweise auf eine Wirkung der Komponente g) als Schutzkolloid oder Emulgator zurückzuführen sein. Dies kann beispielsweise auch aus einer zumindest teilweisen Pfropfung auf die Komponente g) als Pfropfgrundlage resultieren. Es sind jedoch auch andere Mechanismen als eine Pfropfung vorstellbar. Die erfindungsgemäß verwendeten oder erfindungsgemäßen ampholytischen Copolymere umfassen ganz allgemein die Verfahrensprodukte der radikalischen Copolymerisation worunter z. B. reine Pfropfpolymerisate, Mischungen von Pfropfpolymerisaten mit ungepfropften Verbindungen der Komponente g), Copolymerisate der zuvor genannten Monomeren sowie beliebige Mischungen verstanden werden. Anteile von ungepfropften Verbindungen der Komponente g) können je nach Verwendungszweck der ampholytischen Copolymere von Vorteil sein. Speziell Verbindungen g1) kann beispielsweise eine Wirkung als Emulgator oder Schutzkolloid zukommen.

Vorzugsweise beträgt die Einsatzmenge der Komponente g) 1 bis 25 Gew.-%, besonders bevorzugt 3 bis 20 Gew.-%, bezogen auf das Gesamtgewicht der zur Polymerisation eingesetzten Komponenten.

Geeignete Polyether-haltige Verbindungen g1) sind beispielsweise wasserlösliche oder wasserdispergierbare nichtionische Polymere, die Alkylenoxid-Wiederholungseinheiten aufweisen. Vorzugsweise beträgt der Anteil an Alkylenoxid-Wiederholungseinheiten mindestens 30 Gew.-%, bezogen auf das Gesamtgewicht der Verbindung g1). Geeignete polyetherhaltige Verbindungen g1) sind beispielsweise Polyalkylenglykole, Polyester auf Basis von Polyalkylenglykolen, Polyetherurethane sowie Polyalkylenoxidgruppen-haltige Silikonderivate.

Als Komponente g1) geeignete Polyalkylenglykole weisen im allgemeinen ein zahlenmittleres Molekulargewicht im Bereich von etwa 150 bis 100000, bevorzugt 300 bis 50000, besonders bevorzugt 500 bis 40000 auf. Geeignete Polyalkylenglykole sind beispielsweise Polyethylenglykole, Polypropylenglykole, Polytetrahydrofurane und Alkylenoxidcopolymere. Geeignete Alkylenoxide zur Herstellung von Alkylenoxidcopolymeren sind z. B. Ethylenoxid, Propylenoxid, Epichlorhydrin, 1,2- und 2,3-Butylenoxid. Die Alkylenoxidcopolmere können die Alkylenoxideinheiten statistisch verteilt oder in Form Blöcken einpolymerisiert enthalten. Vorteilhafterweise verwendet man Homopolymerisate des Ethylenoxids oder Copolymerisate, die Ethylenoxid enthalten. Vorzugsweise beträgt der Anteil an von Ethylenoxid abgeleiteten Wiederholungseinheiten 40 bis 99 Gew.-%. Geeignet sind beispielsweise Copolymerisate aus Ethylenoxid und Propylenoxid, Copolymerisate aus Ethylenoxid und Butylenoxid sowie Copolymerisate aus Ethylenoxid, Propylenoxid und mindestens einem Butylenoxid. Als Komponente g1) eignen sich auch die Allylether der zuvor genannten Polyalkylenglykole.

Verzweigte polyetherhaltige Polymerisate g1) können hergestellt werden, indem man beispielsweise an Polyalkoholreste, z. B. an Pentaerythrit, Glycerin oder an Zuckeralkohole wie D-Sorbit und D-Mannit oder an Polysaccharide wie Cellulose und Stärke, wenigstens eines der zuvor genannten Alkylenoxide anlagert. Die Alkylenoxid-Einheiten können im Anlagerungsprodukt statistisch verteilt sein oder in Form von Blöcken vorliegen.

Es ist auch möglich, Polyester von Polyalkylenoxiden und aliphatischen oder aromatischen Dicarbonsäuren, z. B. Oxalsäure, Bernsteinsäure, Adipinsäure und Terephthalsäure als polyetherhaltige Verbindung g1) zu verwenden. Geeignete Polyester von Polyalkylenoxiden mit Molmassen von 1500 bis 25000 sind z. B. beschrieben in EP-A-0 743 962. Des weiteren können auch Polycarbonate aus der Umsetzung von Polyalkylenoxiden mit Phosgen oder mit Carbonaten, wie z.B. Diphenylcarbonat, sowie Polyurethane aus der Umsetzung von Polyalkylenoxiden mit aliphatischen und aromatischen Diisocyanaten als Verbindung g1) verwendet werden.

Nach einer bevorzugten Ausführungsform wird zur Herstellung der ampholytischen Copolymere eine Komponente g1) eingesetzt, die wenigstens ein Polyetherurethan umfasst.

Geeignete Polyetherurethane sind die Kondensationsprodukte von Polyetherpolyolen, wie Polyetherdiolen, mit Polyisocyanaten, wie Diisocyanaten. Geeignete Polyetherpolyole sind die zuvor genannten Polyalkylenglycole, die beispielsweise aus der Polymerisation von cyclischen Ethern, wie Tetrahydrofuran, oder aus der Umsetzung von einem oder mehreren Alkylenoxiden mit einem Startermolekül, das zwei oder mehr aktive Wasserstoffatome aufweist, erhältlich sind.

Geeignete Polyisocyanate sind ausgewählt unter Verbindungen mit 2 bis 5 Isocyanatgruppen, Isocyanatpräpolymeren mit einer mittleren Anzahl von 2 bis 5 Isocyanatgruppen, und Mischungen davon. Dazu zählen z. B. aliphatische, cycloaliphatische und aromatische Di-, Tri- und Polyisocyanate. Geeignete Diisocyanate sind z. B. Tetramethylendiisocyanat, Hexamethylendiisocyanat, 2,3,3-Trimethylhexamethylendiisocyanat, 1,4-Cyclohexylendiisocyanat, Isophorondiisocyanat, 1,4-Phenylendiisocyanat, 2,4- und 2,6-Toluylendiisocyanat und deren Isomerengemische (z. B. 80 % 2,4- und 20 % 2,6-Isomer), 1,5-Naphthylendiisocyanat, 2,4- und 4,4'-Diphenylmethandiisocyanat. Ein geeignetes Triisocyanat ist z. B. Triphenylmethan-4,4',4"-triisocyanat. Weiterhin geeignet sind Isocyanatpräpolymere und Polyisocyanate, die durch Addition der zuvor genannten Isocyanate an polyfunktionelle hydroxyl- oder amingruppenhaltige Verbindungen erhältlich sind. Weiterhin geeignet sind Polyisocyanate, die durch Biuret- oder Isocyanuratbildung entstehen. Bevorzugt werden Hexamethylendiisocyanat, trimerisiertes Hexamethylendiisocyanat, Isophorondiisocyanat, 2,4-Toluylendiisocyanat, 2,6-Toluylendiisocyanat, und Mischungen davon, eingesetzt.

Nach einer weiteren bevorzugten Ausführungsform wird zur Herstellung der ampholytischen Copolymere eine Komponente g1) eingesetzt, die wenigstens ein Polyalkylenoxid-haltiges Silikonderivat umfasst.

Geeignete Silikonderivate g1) sind die unter den INCI-Namen Dimethicone-Copolyole oder Silikontenside bekannten Verbindungen, wie zum Beispiel die unter den Markennamen Abil^{®} (der Fa. T. Goldschmidt), Alkasil^{®} (der Fa. Rhöne-Poulenc), Silicone Polyol Copolymer^{®} (der Fa Genesee), Belsil^{®} (der Fa. Wacker), Silwet^{®} (der Fa. OSI) oder Dow Corning (der Fa. Dow Corning) erhältlich Verbindungen. Diese beinhalten Verbindungen mit den CAS-Nummern 64365-23-7; 68937-54-2; 68938-54-5; 68937-55-3.

Besonders geeignete Verbindungen g1) sind solche, die die folgenden Strukturelemente enthalten: 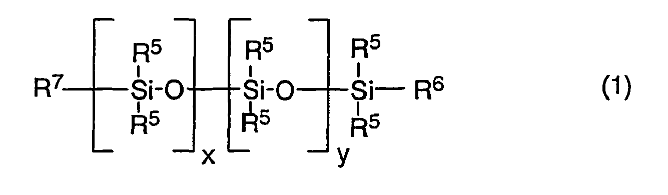 wobei:
R⁶ = CH₃ oder 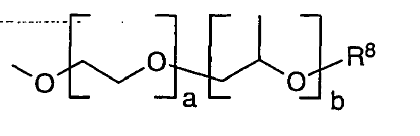 R⁷ = CH₃ oder R⁶
R⁸ = H, CH₃, 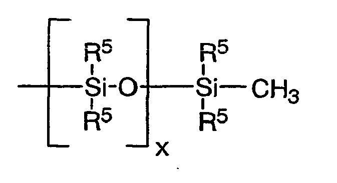 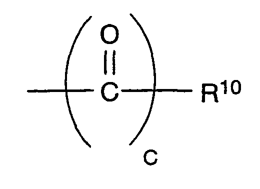
- R¹⁰: ein organischer Rest aus 1 bis 40 Kohlenstoffatomen, der Amino-, Carbonsäure- oder Sulfonatgruppen enthalten kann oder für den Fall c = O, auch das Anion einer anorganischen Säure bedeutet,
und wobei die Reste R⁵ identisch oder unterschiedlich sein können, und entweder aus der Gruppe der aliphatischen Kohlenwasserstoffe mit 1 bis 20 Kohlenstoffatomen stammen, cyclische aliphatische Kohlenwasserstoffe mit 3 bis 20 C-Atomen sind, aromatischer Natur oder gleich R⁹ sind, wobei: 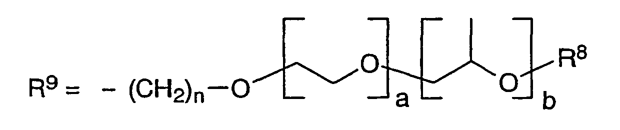 und n eine ganze Zahl von 1 bis 6 ist,
x und y ganze Zahlen derart sind, dass das Molekulargewicht des Polysiloxan-Blocks zwischen 300 und 30000 liegt,
a,b ganze Zahlen zwischen 0 und 50 sein können mit der Maßgabe, dass die Summe aus a und b größer als 0 ist, und c 0 oder 1 ist.

Bevorzugte Reste R⁶ und R⁹ sind solche, bei denen die Summe aus a+b zwischen 5 und 30 beträgt.

Bevorzugt werden die Gruppen R⁵ aus der folgenden Gruppe ausgewählt: Methyl, Ethyl,Propyl, Butyl, Isobutyl, Pentyl, Isopentyl, Hexyl, Octyl, Decyl, Dodecyl und Octadecyl, cycloaliphatische Reste, speziell Cyclohexyl, aromatische Gruppen, speziell Phenyl oder Naphthyl, gemischt aromatisch-aliphatische Reste wie Benzyl oder Phenylethyl sowie Tolyl und Xylyl und R⁹.

Besonders geeignete Reste R⁸ sind solche, bei denen im Falle von R⁸ = -(CO)_{c}-R¹⁰ R¹⁰ ein beliebiger Alkyl-, Cycloalkyl oder Arylrest bedeutet, der zwischen 1 und 40 C-Atomen besitzt und der weitere ionogene Gruppen wie NH₂, COOH, SO₃H tragen kann.

Bevorzugte anorganische Reste R¹⁰ sind, für den Fall c = O, Phosphat und Sulfat.

Besonders bevorzugte Silikonderivate e) sind solche, der allgemeinen Struktur:

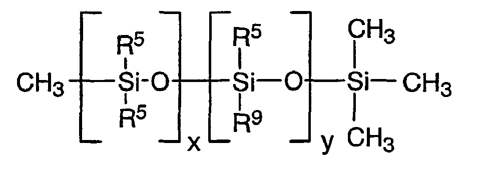

Als Pfropfgrundlage eignen sich vorzugsweise weiterhin Polymerisate g2), die mindestens 50 Gew.-% an Vinylalkoholeinheiten besitzen. Bevorzugt enthalten diese Polymerisate mindestens 70 Gew.-%, ganz besonders bevorzugt 80 Gew.-% Polyvinylalkoholeinheiten. Solche Polymerisate werden überlicherweise durch Polymerisation eines Vinylesters und anschließender zumindest teilweiser Alkoholyse, Aminolyse oder Hydrolyse hergestellt. Bevorzugt sind Vinylester linearer und verzweigter C₁-C₁₂-Carbonsäuren, ganz besonders bevorzugt ist Vinylacetat. Die Vinylester können selbstverständlich auch im Gemisch eingesetzt werden.

Als Comonomere des Vinylesters zur Synthese der Pfropfgrundlage g2) kommen beispielsweise N-Vinylcaprolactam, N-Vinylpyrrolidon, N-Vinylimidazol, N-Vinyl-2-methylimidazol, N-Vinyl-4-methylimidazol, 3-Methyl-1-vinylimidazoliumchlorid, 3-Methyl-1-vinylimidazoliummethylsulfat, Diallylammoniumchlorid, Styrol, Alkylstyrole in Frage.

Weitere geeignete Comonomere zur Herstellung der Pfropfgrundlage g2) sind beispielsweise monoethylenisch ungesättigten C₃-C₆-Carbonsäuren wie z. B. Acrylsäure, Methacrylsäure, Crotonsäure, Fumarsäure, sowie deren Ester, Amide und Nitrile wie z. B. Acrylsäuremethylester, Acrylsäureethylester, Methacrylsäuremethylester, Methacrylsäureethylester, Methacrylsäurestearylester, Hydroxyethylacrylat, Hydroxypropylacrylat, Hydroxybutylacrylat, Hydroxyethylmethacrylat, Hydroxypropylmethacrylat, Hydroxyisobutylacrylat, Hydroxyisobutylmethacrylat, Maleinsäuremonomethylester, Maleinsäuredimethylester, Maleinsäuremonoethylester, Maleinsäurediethylester, 2-Ethylhexylacrylat, 2-Ethylhexylmethacrylat, Maleinsäureanhydrid sowie dessen Halbester, Alkylenglykol(meth)acrylate, Acrylamid, Methacrylamid, N-Dimethylacrylamid, N-tert.-butylacrylamid, Acrylnitril, Methacrylnitril, Vinylether wie z. B. Methyl-, Ethyl-, Butyl oder Dodecylvinylether, kationische Monomere wie Dialkylaminoalkyl(meth)acrylate und Dialkylaminoalkyl(meth)acrylamide wie Dimethylaminothylacrylat, Diethylaminoethylacrylat, Diethylaminoethylmethacrylat, sowie die Salze der zuletzt genannten Monomeren mit Carbonsäuren oder Mineralsäuren sowie die quarternierten Produkte.

Bevorzugte Pfropfgrundlagen g2) sind Polymerisate, die durch Homopolymerisation von Vinylacetat und anschließender zumindest teilweiser Hydrolyse, Alkoholyse oder Aminolyse hergestellt werden.

Besonders bevorzugte Propfgrundlagen g2) sind Polymerisate, die durch Homopolymerisation von Vinylacetat und anschließender zumindest teilweiser Verseifung hergestellt werden. Solche Polyvinylalkoholeinheiten enthaltenden Polymere sind unter dem Namen Mowiol® erhältlich.

Bevorzugt werden als Komponente g) Cellulose, Cellulosederivate, Stärke und/oder Stärkederivate g3) eingesetzt. Dazu zählen Substanzen, die Saccharid-Strukturen enthalten. Solche natürlichen Substanzen sind beispielsweise Saccharide pflanzlicher oder tierischer Herkunft oder Produkte, die durch Metabolisierung durch Mikroorganismen entstanden sind, sowie deren Abbauprodukte. Geeignete Pfropfgrundlagen g3) sind beispielsweise Oligosaccharide, Polysaccharide, oxidativ, enzymatisch oder hydrolytisch abgebaute Polysaccharide, oxidativ hydrolytisch abgebaute oder oxidativ enzymatisch abgebaute Polysaccharide, chemisch modifizierte Oligo- oder Polysaccharide und Mischungen davon. Bevorzugte Produkte sind die in US 5,334,287 in Spalte 4, Zeile 20 bis Spalte 5, Zeile 45 genannten Verbindungen.

Geeignete kommerziell erhältliche Produkte sind die C-Pur® und C-Dry^{®}-Marken der Fa. Cerestar.

Gewünschtenfalls können Gemische von Verbindungen der Komponente g) eingesetzt werden.

Eine bevorzugte Ausführungsform sind Mittel, die ampholytische Copolymere, die durch Copolymerisation in Gegenwart wenigstens einer Verbindung g1) erhältlich sind, die ausgewählt ist unter Polyalkylenoxiden, Polyalkylenoxid-haltigen Silikonderivaten und Mischungen davon enthalten.

Vorzugsweise weisen die erfindungsgemäß verwendeten oder erfindungsgemäßen Copolymere einen K-Wert (gemessen nach E. Fikentscher, Cellulose-Chemie 13 (1932), S. 58-64) an einer 1 gew.-%igen Lösung in Wasser im Bereich von etwa 30 bis 300, besonders bevorzugt 40 bis 150 auf.

Je nach K-Wert eignen sich die erfindungsgemäß verwendeten oder erfindungsgemäßen Polymere für eine Vielzahl kosmetischer und pharmazeutischer Anwendungen. So lassen sich Polymere mit einem K-Wert bis etwa 50 vorteilhaft als Sprays (Aerosol- und Pumpsprays) formulieren. Polymere mit einem K-Wert in einem Bereich von etwa 50 bis 90 eignen sich vorteilhaft für Gele und Schäume. Für Shampoos und hautkosmetische Anwendungen eignen sich bevorzugt Polymere mit einem K-Wert von mindestens 80.

Die erfindungsgemäß verwendeten oder erfindungsgemäßen ampholytischen Copolymere sind vorzugsweise erhältlich durch radikalische Copolymerisation von
- 0,1 bis 30 Gew.-%, bevorzugt 0,3 bis 25 Gew.-%, bezogen auf das Gesamtgewicht der zur Polymerisation eingesetzten Komponenten, wenigstens einer Verbindung a),
- 0,1 bis 40 Gew.-%, bezogen auf das Gesamtgewicht der zur Polymerisation eingesetzten Komponenten, wenigstens einer Verbindung b),
- 40 bis 99,8 Gew.-%, bezogen auf das Gesamtgewicht der zur Polymerisation eingesetzten Komponenten, wenigstens einer Verbindung c),
- 0 bis 20 Gew.-% wenigstens eines weiteren Monomers d), 0 bis 10 Gew.-%, bevorzugt 0,1 bis 7 Gew.-%, wenigstens eines Polyetheracrylats e),
- 0 bis 10 Gew.-%, bevorzugt 0 bis 5 Gew.-%, wenigstens eines Vernetzers f),
gegebenenfalls in Gegenwart von bis zu 25 Gew.-%, bezogen auf auf das Gesamtgewicht der zur Polymerisation eingesetzten Komponenten, wenigstens einer Komponente g).

Besonders bevorzugt sind ampholytische Copolymere, die erhältlich sind durch radikalische Copolymerisation von
- 0,1 bis 25 Gew.-%, bevorzugt 0,3 bis 20 Gew.-%, bezogen auf das Gesamtgewicht der zur Polymerisation eingesetzten Komponenten, wenigstens eines Monomers, das ausgwählt ist unter Acrylsäure, Methacrylsäure, 2-Acrylamido-2-methylpropansulfonsäure, Styrol-4-sulfonsäure und Mischungen davon,
- 1 bis 20 Gew.-%, bezogen auf das Gesamtgewicht der zur Polymerisation eingesetzten Komponenten, wenigstens einer Verbindung b), die ausgewählt ist unter N,N-Dimethylaminopropyl(meth)acrylat, Vinylimidazol und Mischungen davon,
- 60 bis 98,9 Gew.-%, bezogen auf das Gesamtgewicht der zur Polymerisation eingesetzten Komponenten, wenigstens einer Verbindung c), die ausgewählt ist unter Acrylsäureamid, Methacrylsäureamid, N-Vinylpyrrolidon, N-Vinylcaprolactam, N-Vinylformamid und Mischungen davon.

Desweiteren besonders bevorzugt ist das ampholytische Copolymer erhältlich durch radikalische Polymerisation von
- 5 bis 20 Gew.-%, bezogen auf das Gesamtgewicht der zur Polymerisation eingesetzten Komponenten, wenigstens eines Monomers, das ausgwählt ist unter Acrylsäure, Methacrylsäure, 2-Acrylamido-2-methylpropansulfonsäure, Styrol-4-sulfonsäure und Mischungen davon,
- 10 bis 30 Gew.-%, bezogen auf das Gesamtgewicht der zur Polymerisation eingesetzten Komponenten, wenigstens einer Verbindung b), die ausgewählt ist unter N,N-Dimethylaminopropyl(meth)acrylat, Vinylimidazol und Mischungen davon,
- 50 bis 85 Gew.-%, bezogen auf das Gesamtgewicht der zur Polymerisation eingesetzten Komponenten, wenigstens einer Verbindung c), die ausgewählt ist unter Acrylsäureamid, Methacrylsäureamid, N-Vinylpyrrolidon, N-Vinylcaprolactam, N-Vinylformamid und Mischungen davon,
- 0 bis 25 Gew.-% wenigstens eines weiteren Monomers d),
   0 bis 10 Gew.-%, bevorzugt 0,1 bis 7 Gew.-% wenigstens eines Polyetheracrylats e).

Die zuvor genannten besonders bevorzugt verwendeten Copolymere können zusätzlich 0,05 bis 1 Gew.-% wenigstens eines Vernetzers f) einpolymerisiert enthalten. Dabei handelt es sich insbesondere um Methylenbisacrylamid und/oder N,N'-Diallylweinsäurediamid.

Die zuvor genannten besonders bevorzugt verwendeten Copolymere können zusätzlich 1 bis 10 Gew.-% wenigstens eines Esters einer α,β-ethylenisch ungesättigten Mono- oder Dicarbonsäure mit einem C₁-C₃₀-Alkanol, vorzugsweise Stearyl(meth)acrylat, einpolymerisiert enthalten.

Die zuvor genannten besonders bevorzugt verwendeten Copolymere sind nach einer speziellen Ausführungsform erhältlich durch Copolymerisation in Gegenwart von bis zu 10 Gew.-%, bezogen auf das Gesamtgewicht der zur Polymerisation eingesetzten Komponenten, wenigstens einer Komponente g).

Eine bevorzugte Ausführung sind Mittel, die Copolymere, die aus Wiederholungseinheiten von
- Vinylpyrrolidon,
- Acrylsäure und/oder Methacrylsäure,
- Dimethylaminoethylmethacrylat oder Dimethylaminopropylmethacrylsäureamid oder Vinylimidazol oder tert.-Butylaminoethylmethacrylat und
- wenigstens einem Polyetheracrylat
bestehen enthalten.

Eine weitere bevorzugte Ausführung sind Mittel, die Copolymere, die aus Wiederholungseinheiten von
- Vinylpyrrolidon,
- 2-Acrylamido-2-methylpropansulfonsäure,
- Dimethylaminoethylmethacrylat oder Dimethylaminopropylmethacrylsäureamid oder Vinylimidazol oder tert.-Butylaminoethylmethacrylat und
- wenigstens einem Polyetheracrylat
besteht enthalten.

Eine weitere bevorzugte Ausführung sind Mittel, die Copolymere, die aus Wiederholungseinheiten von
- Vinylpyrrolidon,
- Acrylsäureamid und/oder Methacrylsäureamid,
- Acrylsäure und/oder Methacrylsäure,
- Dimethylaminoethylmethacrylat oder Dimethylaminopropylmethacrylsäureamid oder Vinylimidazol oder tert.-Butylaminoethylmethacrylat und
- wenigstens einem Polyetheracrylat
bestehen enthalten.

Eine weitere bevorzugte Ausführung sind Mittel, die Copolymere, die aus Wiederholungseinheiten von
- Vinylpyrrolidon,
- Acrylsäureamid und/oder Methacrylsäureamid,
- 2-Acrylamido-2-methylpropansulfonsäure,
- Dimethylaminoethylmethacrylat oder Dimethylaminopropylmethacrylsäureamid oder Vinylimidazol oder tert.-Butylaminoethylmethacrylat und
- wenigstens einem Polyetheracrylat
bestehen enthalten.

Eine weitere bevorzugte Ausführung sind Copolymere, die aus wiederholungseinheiten von
- Vinylpyrrolidon,
- Acrylsäure und/oder Methacrylsäure,
- Dimethylaminoethylmethacrylat oder Dimethylaminopropylmethacrylsäureamid oder Vinylimidazol oder tert.-Butylaminoethylmethacrylat und
- wenigstens einem Polyetheracrylat,
- wenigstens einem Monomer der Formel

   CH₂=CR^{c}-C(=O)-O-R^{d}

   worin
   R^{c} für H oder Methyl steht und
   R^{d} für lineares C₁-C₄-Alkyl steht,
bestehen enthalten.

Eine weitere bevorzugte Ausführung sind Mittel, die Copolymere, die aus Wiederholungseinheiten von
- Vinylpyrrolidon,
- 2-Acrylamido-2-methylpropansulfonsäure,
- Dimethylaminoethylmethacrylat oder Dimethylaminopropylmethacrylsäureamid oder Vinylimidazol oder tert.-Butylaminoethylmethacrylat und
- wenigstens einem Polyetheracrylat,
- wenigstens einem Monomer der Formel

   CH₂=CR^{c}-C(=O)-O-R^{d}

   worin
   R^{c} für H oder Methyl steht und
   R^{d} für lineares C₁-C₄-Alkyl steht,
bestehen enthalten.

Eine weitere bevorzugte Ausführung sind Mittel, die Copolymere, die aus Wiederholungseinheiten von
- Vinylpyrrolidon,
- Acrylsäureamid und/oder Methacrylsäureamid,
- Acrylsäure und/oder Methacrylsäure,
- Dimethylaminoethylmethacrylat oder Dimethylaminopropylmethacrylsäureamid oder Vinylimidazol oder tert.-Butylaminoethylmethacrylat und
- wenigstens einem Polyetheracrylat,
- wenigstens einem Monomer der Formel

   CH₂=CR^{c}-C(=O)-O-_{R}d

   worin
   R^{c} für H oder Methyl steht und
   R^{d} für lineares C₁-C₄-Alkyl steht,
bestehen, enthalten

Eine weitere bevorzugte Ausführung sind Copolymere, die aus Wiederholungseinheiten von
- Vinylpyrrolidon,
- Acrylsäureamid und/oder Methacrylsäureamid,
- 2-Acrylamido-2-methylpropansulfonsäure,
- Dimethylaminoethylmethacrylat oder Dimethylaminopropylmethacrylsäureamid oder Vinylimidazol oder tert.-Butylaminoethylmethacrylat und
- wenigstens einem Polyetheracrylat,
- wenigstens einem Monomer der Formel

   CH₂=CR^{c}-C(=O)-O-R^{d}

   worin
   R^{c} für H oder Methyl steht und
   R^{d} für lineares C₁-C₄-Alkyl steht,
bestehen.

Vorzugsweise enthalten die Copolymere der zuvor genannten bevorzugten Ausführungen zusätzlich ein Salz der 2-Acrylamido-2-methylpropansulfonsäure, vorzugsweise das Natriumsalz, einpolymerisiert.

Vorzugsweise enthalten die Copolymere der zuvor genannten bevorzugten Ausführungen zusätzlich ein quaternisiertes Amingruppen-haltiges Monomer, vorzugsweise quaternisiertes Vinylimidazol, einpolymerisiert.

Vorzugsweise enthalten die Copolymere der zuvor genannten bevorzugten Ausführungen zusätzlich bis zu 1 Gew.-%, bezogen auf das Gesamtgewicht der zur Polymerisation eingesetzten Monomere, wenigstens eines Vernetzers einpolymerisiert.

Die Herstellung der ampholytischen Copolymere erfolgt nach üblichen, dem Fachmann bekannten Verfahren, vorzugsweise durch Lösungs-Polymerisation und Fällungs-Polymerisation.

Bevorzugte Lösemittel zur Lösungspolymerisation sind wässrige Lösungsmittel, wie Wasser und Gemische aus Wasser mit wassermischbaren Lösungsmitteln, beispielsweise Alkoholen, wie Methanol, Ethanol, n-Propanol, Isopropanol, n-Butanol, sek.-Butanol, tert.-Butanol, n-Hexanol und Cyclohexanol sowie Glykole, wie Ethylenglykol, Propylenglykol und Butylenglykol sowie die Methyl- oder Ethylether der zweiwertigen Alkohole, Diethylenglykol, Triethylenglykol, Polyethylenglykolen mit zahlenmittleren Molekulargewichten bis etwa 3000, Glycerin und Dioxan. Besonders bevorzugt ist die Polymerisation in Wasser oder einem Wasser/Alkohol-Gemisch, beispielsweise in einem Wasser/Ethanol-Gemisch.

Die Fällungspolymerisation erfolgt beispielsweise in einem Ester, wie Essigsäureethylester oder Essigsäurebutylester als Lösungsmittel. Die resultierenden Polymerteilchen fallen aus der Reaktionslösung aus und können durch übliche Verfahren, wie Filtration mittels Unterdruck, isoliert werden. Bei der Fällungspolymerisation werden in der Regel Polymere mit höheren Molekulargewichten als bei der Lösungspolymerisation erhalten.

Die Polymerisationstemperaturen liegen vorzugsweise in einem Bereich von etwa 30 bis 120 °C, besonders bevorzugt 40 bis 100 °C. Die Polymerisation erfolgt üblicherweise unter atmosphärischem Druck, sie kann jedoch auch unter vermindertem oder erhöhtem Druck ablaufen. Ein geeigneter Druckbereich liegt zwischen 1 und 5 bar.

Zur Herstellung der Polymerisate können die Monomeren gegebenenfalls in Gegenwart der Komponente e) mit Hilfe von Radikale bildenden Initiatoren polymerisiert werden.

Als Initiatoren für die radikalische Polymerisation können die hierfür üblichen Peroxo- und/oder Azo-Verbindungen eingesetzt werden, beispielsweise Alkali- oder Ammoniumperoxidisulfate, Diacetylperoxid, Dibenzoylperoxid, Succinylperoxid, Di-tert.-butylperoxid, tert.-Butylperbenzoat, tert.-Butylperpivalat, tert.-Butylperoxy-2-ethylhexanoat, tert.-Butylpermaleinat, Cumolhydroperoxid, Diisopropylperoxidicarbamat, Bis-(o-toluoyl)-peroxid, Didecanoylperoxid, Dioctanoylperoxid, Dilauroylperoxid, tert.-Butylperisobutyrat, tert.-Butylperacetat, Di-tert.-Amylperoxid, tert.-Butylhydroperoxid, Azo-bis-isobutyronitril, Azo-bis-(2-amidinopropan)dihydrochlorid oder 2-2'-Azo-bis-(2-methyl-butyronitril). Geeignet sind auch Initiatormischungen oder Redox-Initiator-Systeme, wie z. B. Ascorbinsäure/Eisen(II)sulfat/Natriumperoxodisulfat, tert.-Butylhydroperoxid/Natriumdisulfit, tert.-Butylhydroperoxid/Natriumhydroxymethansulfinat, H₂O₂/Cu^{I}.

Zur Einstellung des Molekulargewichts kann die Polymerisation in Gegenwart wenigstens eines Reglers erfolgen. Als Regler können die üblichen, dem Fachmann bekannten Verbindungen, wie z. B. Schwefelverbindungen, z. B. Mercaptoethanol; 2-Ethylhexylthioglycolat, Thioglycolsäure oder Dodecylmercaptan sowie Tribromchlormethan oder andere Verbindungen, die regelnd auf das Molekulargewicht der erhaltenen Polymerisate wirken, eingesetzt werden. Ein bevorzugter Regler ist Cystein.

Zur Erzielung möglichst reiner Polymere mit geringem Restmonomergehalt kann sich an die Polymerisation (Hauptpolymerisation) ein Nachpolymerisationsschritt anschließen. Die Nachpolymerisation kann in Gegenwart desselben oder eines anderen Initiatorsystems wie die Hauptpolymerisation erfolgen. Vorzugsweise erfolgt die Nachpolymerisation mindestens bei der gleichen, vorzugsweise bei einer höheren Temperatur als die Hauptpolymerisation. Die Temperatur bei der Haupt- und der Nachpolymerisation beträgt vorzugsweise höchstens 90 °C. Bevorzugt wird der Reaktionsansatz einem Strippen mit Wasserdampf oder einer Wasserdampf-Destillation unterzogen.

Wird bei der Herstellung der Polymere ein organisches Lösungsmittel eingesetzt, so kann dieses durch übliche, dem Fachmann bekannte Verfahren, z. B. durch Destillation bei vermindertem Druck, entfernt werden.

Die Polymerisation erfolgt, wie eingangs beschrieben, vorzugsweise bei einem pH-Wert im Bereich von 5,5 bis 8,0, besonders bevorzugt von 5,6 bis 7,5 und insbesondere von 5,8 bis 7,3. Dies führt in der Regel auch zur Erzielung möglichst reiner Polymere mit geringem Restmonomergehalt, was eventuell darauf zurückzuführen ist, dass Amine, die von einigen der Monomere als Abspaltprodukt gebildet werden und die unter den Polymerisationsbedingungen gegebenenfalls mit anderen Monomeren zu unerwünschten Nebenprodukten reagieren können, entfernt werden. Die Einstellung des pH-Wertes erfolgt, wie ebenfalls eingangs beschrieben, durch Zugabe einer geeigneten Säure oder durch Zugabe einer geeigneten Base.

Produkte mit besonders hoher Reinheit und entsprechend vorteilhaften Eigenschaften für einen Einsatz in der Kosmetik können erzielt werden, wenn das Reaktionsprodukt nach der Polymerisation, gegebenenfalls vor und/oder nach einer Nachpolymerisation, einer Wasserdampfdestillation bzw. einem Strippen mit Wasserdampf unterzogen wird. Auch diese Behandlung mit Wasserdampf dient z. B. der Entfernung von Aminen und weiterer unerwünschter, mit Wasserdampf entfernbarer Nebenprodukte aus dem Reaktionsgemisch. Vorzugsweise erfolgt die Wasserdampf-Behandlung zumindest zwischen Haupt- und Nachpolymerisation. Der pH-Wert des Polymerisationsprodukts wird vorzugsweise vor der Wasserdampf-Behandlung auf einen Wert von höchstens 6,5 eingestellt. Die Temperatur des eingesetzten Wasserdampfs und der behandelten Polymerlösung beträgt vorzugsweise mindestens 90 °C.

Die Polymerlösungen können durch verschiedene Trocknungsverfahren, wie z. B. Sprühtrocknung, Fluidized Spray Drying, Walzentrocknung oder Gefriertrocknung in Pulverform überführt werden.

Bevorzugt wird die Sprühtrocknung eingesetzt. Die so erhaltenen Polymer-Trockenpulver lassen sich vorteilhafterweise durch Lösen bzw. Redispergieren in Wasser erneut in eine wässrige Lösung bzw. Dispersion überführen. Pulverförmige Copolymere haben den Vorteil einer besseren Lagerfähigkeit, einer einfacheren Transportmöglichkeit und zeigen in der Regel eine geringere Neigung für Keimbefall.

Die anionogenen Gruppen (Säuregruppen) der Polymere können mit einer Base teilweise oder vollständig neutralisiert werden. Als Base für die Neutralisation der Polymere können Alkalimetallbasen wie Natronlauge, Kalilauge, Soda, Natriumhydrogencarbonat, Kaliumcarbonat oder Kaliumhydrogencarbonat und Erdalkalimetallbasen wie Calciumhydroxyd, Calciumoxid, Magnesiumhydroxyd oder Magnesiumcarbonat sowie Amine verwendet werden. Geeignete Amine sind z. B. C₁-C₆-Alkylamine, bevorzugt n-Propylamin und n-Butylamin, Dialkylamine, bevorzugt Diethylpropylamin und Dipropylmethylamin, Trialkylamine, bevorzugt Triethylamin und Triisopropylamin. Bevorzugt sind Aminoalkohole, z.B. Trialkanolamine, wie Triethanolamin, Alkyldialkanolamine, wie Methyl- oder Ethyldiethanolamin und Dialkylalkanolamine, wie Dimethylethanolamin sowie 2-Amino-2-methyl-1-propanol. Besonders für den Einsatz in Haarbehandlungsmitteln haben sich zur Neutralisation der Säuregruppen enthaltenden Polymere NaOH, KOH, 2-Amino-2-methyl-1-propanol, 2-Amino-2-ethylpropan-1,3-diol, Diethylaminopropylamin und Triisopropanolamin bewährt. Die Neutralisation der Säuregruppen kann auch mit Hilfe von Mischungen mehrerer Basen vorgenommen werden, z. B. Mischungen aus Natronlauge und Triisopropanolamin. Die Neutralisation kann je nach Anwendungszweck partiell oder vollständig erfolgen.

Geladene kationische Gruppen lassen sich aus den vorliegenden kationogenen stickstoffhaltigen Gruppen entweder durch Protonierung, z. B. mit ein- oder mehrwertigen Carbonsäuren, wie Milchsäure oder Weinsäure, oder mit Mineralsäuren, wie Phosphorsäure, Schwefelsäure und Salzsäure, oder durch Quaternisierung, z. B. mit Alkylierungsmitteln, wie C₁- bis C₄-Alkylhalogeniden oder -sulfaten, erzeugen. Beispiele solcher Alkylierungsmittel sind Ethylchlorid, Ethylbromid, Methylchlorid, Methylbromid, Dimethylsulfat und Diethylsulfat.

In der Regel erfolgt eine Neutralisation so, dass der pH-Wert einer 0,1 molaren wässrigen Lösung der erfindungsgemäß verwendeten oder erfindungsgemäßen wasserlöslichen ampholytischen Copolymere bei einer Temperatur von 20 °C in einem Bereich von 5,5 bis 8,0, besonders bevorzugt von 5,6 bis 7,5 und insbesondere von 5,8 bis 7,3, liegt.

Ein weiterer Gegenstand der Erfindung sind Polyelektrolyt-Komplexe und Mittel, die solche enthalten, die wenigstens ein ampholytisches Copolymer, wie zuvor definiert, und wenigstens einen weiteren, davon verschiedenen Polyelektrolyten enthalten.

Geeignete weitere Polyelektrolyte sind prinzipiell ausgewählt unter Polymeren mit anionogenen und/oder anionischen Gruppen, Polymeren mit kationogenen und/oder kationischen Gruppen, ampholytischen Copolymeren und Mischungen davon.

Die Polyelektrolyt-Komplexe enthalten vorzugsweise wenigstens ein erfindungsgemäß verwendetes oder erfindungsgemäßes ampholytisches Copolymer und wenigstens einen weiteren, davon verschiedenen Polyelektrolyten in einem Gewichtsmengenverhältniss von etwa 10:1 bis 1:10. Vorzugsweise beträgt bei den erfindungsgemäß verwendeten oder erfindungsgemäßen Polyelektrolyt-Komplexen das molare Verhältnis von anionogenen und anionischen Gruppen zu kationogenen und kationischen Gruppen etwa 0,8:1 bis 1:0,8, bevorzugt 0,9:1 bis 1:0,9.

Als Polyelektrolyte geeignete anionische Polymere sind beispielsweise Homo- und Copolymerisate von Acrylsäure und Methacrylsäure und deren Salze. Dazu zählen auch vernetzte Polymere der Acrylsäure, wie sie unter dem INCI-Namen Carbomer erhältlich sind. Derartige vernetzte Homopolymere der Acrylsäure sind beispielsweise kommerziell unter dem Namen Carbopol® von der Firma Noveon (vormals BF GOODRICH) erhältlich. Bevorzugt sind auch hydrophob modifizierte vernetzte Polyacrylat-Polymere, wie Carbopol® Ultrez 21 von der Fa. Noveon. erfindungsgemäß verwendete oder erfindungsgemäße Polyelektrolyt-Komplexe auf Basis von Homo- und Copolymerisaten von Acrylsäure und Methacrylsäure eignen sich in vorteilhafter Weise zur Formulierung als Gele, beispielsweise für Festigergele, sowie zur Formulierung von Schäumen.

Beispiele für geeignete anionische Polymere sind weiterhin sauer modifizierte Stärken. Sauer modifizierte Stärken und Verfahren zu ihrer Herstellung sind dem Fachmann prinzipiell bekannt und werden beispielsweise in Ullmann's Encyclopedia of Industrial Chemistry, 5. Auflage auf CD-ROM, Starch and other Polysaccharides, 1.2.2.1, Wiley-VCH (1997) beschrieben. Polyelektrolyt-Komplexe auf Basis von anionisch modifizierten Stärken eignen sich in vorteilhafter Weise zur Formulierung von Gelen und für einen Einsatz als Konditioner für Shampoos.

Weitere Beispiele für geeignete anionische Polymere sind Copolymere von Acrylsäure und Acrylamid und deren Salze; Natriumsalze von Polyhydroxycarbonsäuren, wasserlösliche oder wasserdispergierbare Polyester, Polyurethane und Polyharnstoffe. Besonders geeignete Polymere sind Copolymere aus (Meth)acrylsäure und Polyetheracrylaten, wobei die Polyetherkette mit einem C₈-C₃₀-Alkylrest terminiert ist. Dazu zählen z. B. Acrylat/Beheneth-25-methacrylat-Copolymere, die unter der Bezeichnung Aculyn^{®} von der Firma Rohm und Haas erhältlich sind. Besonders geeignete Polymere sind weiterhin Copolymere aus t-Butylacrylat, Ethylacrylat, Methacrylsäure (z. B. Luvimer^{®} 100P), Copolymere aus Ethylacrylat und Methacrylsäure (z. B. Luvimer^{®} MAE und Luviflex^{®} soft), Copolymere aus N-tert.-Butylacrylamid, Ethylacrylat, Acrylsäure (Ultrahold^{®} 8, strong), Copolymere aus Vinylacetat, Crotonsäure und gegebenenfalls weitere Vinylester (z. B. Luviset^{®} Marken), Maleinsäureanhydridcopolymere, gegebenenfalls mit Alkohol umgesetzt, anionische Polysiloxane, z. B. carboxyfunktionelle, t-Butylacrylat, Methacrylsäure (z. B. Luviskol^{®} VBM), Copolymere von Acrylsäure und Methacrylsäure mit hydrophoben Monomeren, wie z. B. C₄-C₃₀-Alkylester der Meth(acrylsäure), C₄-C₃₀-Alkylvinylester, C₄-C₃₀-Alkylvinylether und Hyaluronsäure. Beispiele für anionische Polymere sind weiterhin Vinylacetat/Crotonsäure-Copolymere, wie sie beispielsweise unter den Bezeichnungen Resyn^{®} (National Starch) und Gafset^{®} (GAF) im Handel sind und Vinylpyrrolidon/Vinylacrylat-Copolymere, erhältlich beispielsweise unter dem Warenzeichen Luviflex^{®} (BASF). Weitere geeignete Polymere sind das unter der Bezeichnung Luviflex^{®} VBM-35 (BASF) erhältliche Vinylpyrrolidon/Acrylat-Terpolymer und Natriumsulfonat-haltige Polyamide oder Natriumsulfonat-haltige Polyester.

Weitere geeignete Polymere sind kationische Polymere mit der Bezeichnung Polyquaternium nach INCI, z. B. Copolymere aus Vinylpyrrolidon/N-Vinylimidazoliumsalzen (Luviquat^{®} FC, Luviquat^{®} HM, Luviquat^{®} MS, Luviquat^{®} Care), Copolymere aus N-Vinylpyrrolidon/Dimethylaminoethylmethacrylat, quaternisiert mit Diethylsulfat (Luviquat^{®} PQ 11), Copolymere aus N-Vinylcaprolactam/N-Vinylpyrrolidon/N-Vinylimidazoliumsalzen (Luviquat^{®} Hold); kationische Cellulosederivate (Polyquaternium-4 und -10), Acrylamidocopolymere (Polyquaternium-7) und Chitosan. Geeignete kationische (quaternisierte) Polymere sind auch Merquat^{®} (Polymer auf Basis von Dimethyldiallylammoniumchlorid), Gafquat^{®} (quaternäre Polymere, die durch Reaktion von Polyvinylpyrrolidon mit quaternären Ammoniumverbindungen entstehen), Polymer JR (Hydroxyethylcellulose mit kationischen Gruppen) und kationische Polymere auf pflanzlicher Basis, z. B. Guarpolymere, wie die Jaguar^{®}-Marken der Fa. Rhodia. Geeignet sind auch die in der deutschen Patentanmeldung P 102 43 573.1 beschriebenen Polymere mit (Meth)acrylsäureamideinheiten. Weiterhin geeignet sind auch die in der deutschen Patentanmeldung P 103 31 870.4 beschriebenen Polymere, die Vinylimidazol und/oder ein Derivat davon einpolymerisiert enthalten.

Geeignete Polymere sind auch amphotere oder zwitterionische Polymere, wie die unter den Bezeichnungen Amphomer® (National Starch) erhältlichen Octylacrylamid/Methylmethacrylat/tert.-Butylaminoethylmethacrylat/2-Hydroxypropylmethacrylat-Copolymere sowie zwitterionische Polymere, wie sie beispielsweise in den deutschen Patentanmeldungen DE 39 29 973, DE 21 50 557, DE 28 17 369 und DE 37 08 451 offenbart sind. Acrylamidopropyltrimethylammoniumchlorid/Acrylsäure- bzw. -Methacrylsäure-Copolymerisate und deren Alkali- und Ammoniumsalze sind bevorzugte zwitterionische Polymere. Weiterhin geeignete zwitterionische Polymere sind Methacroylethylbetain/Methacrylat-Copolymere, die unter der Bezeichnung Amersette^{®} (AMERCHOL) im Handel erhältlich sind, und Copolymere aus Hydroxyethylmethacrylat, Methylmethacrylat, N,N-Dimethylaminoethylmethacrylat und Acrylsäure (Jordapon^{®}).

Die erfindungsgemäßen kosmetischen oder pharmazeutischen Mittel, enthalten
A) wenigstens ein ampholytisches Copolymer, wie zuvor definiert, oder einen Polyelektrolyt-Komplex, wie zuvor definiert, und
B) wenigstens einen kosmetisch akzeptablen Träger.

Der kosmetisch akzeptable Träger B) ist vorzugsweise ausgewählt unter
i) Wasser,
ii) wassermischbaren organischen Lösungsmitteln, vorzugsweise C₁-C₄-Alkanolen,
iii)Ölen, Fetten, Wachsen,
iv) von iii) verschiedenen Estern von C₆-C₃₀-Monocarbonsäuren mit ein-, zwei- oder dreiwertigen Alkoholen,
v) gesättigten acyclischen und cyclischen Kohlenwasserstoffen,
vi) Fettsäuren,
vii)Fettalkoholen
und Mischungen davon.

Die erfindungsgemäßen Mittel weisen z. B. eine Öl- bzw. Fettkomponente B) auf, die ausgewählt ist unter: Kohlenwasserstoffen geringer Polarität, wie Mineralölen; linearen gesättigten Kohlenwasserstoffen, vorzugsweise mit mehr als 8 C-Atomen, wie Tetradecan, Hexadecan, Octadecan etc.; cyclischen Kohlenwasserstoffen, wie Decahydronaphthalin; verzweigten Kohlenwasserstoffen; tierischen und pflanzlichen Ölen; Wachsen; Wachsestern; Vaselin; Estern, bevorzugt Estern von Fettsäuren, wie z. B. die Ester von C₁-C₂₄-Monoalkoholen mit C₁-C₂₂-Monocarbonsäuren, wie Isopropylisostearat, n-Propylmyristat, iso-Propylmyristat, n-Propylpalmitat, iso-Propylpalmitat, Hexacosanylpalmitat, Octacosanylpalmitat, Triacontanylpalmitat, Dotriacontanylpalmitat, Tetratriacontanylpalmitat, Hexacosanylstearat, Octacosanylstearat, Triacontanylstearat, Dotriacontanylstearat, Tetratriacontanylstearat; Salicylaten, wie C₁-C₁₀-Salicylaten, z. B. Octylsalicylat; Benzoatestern, wie C₁₀-C₁₅-Alkylbenzoaten, Benzylbenzoat; anderen kosmetischen Estern, wie Fettsäuretriglyceriden, Propylenglykolmonolaurat, Polyethylenglykolmonolaurat, C₁₀-C₁₅-Alkyllactaten, etc. und Mischungen davon.

Geeignete Siliconöle B) sind z. B. lineare Polydimethylsiloxane, Poly(methylphenylsiloxane), cyclische Siloxane und Mischungen davon. Das zahlenmittlere Molekulargewicht der Polydimethylsiloxane und Poly(methylphenylsiloxane) liegt vorzugsweise in einem Bereich von etwa 1000 bis 150000 g/mol. Bevorzugte cyclische Siloxane weisen 4- bis 8-gliedrige Ringe auf. Geeignete cyclische Siloxane sind z. B. unter der Bezeichnung Cyclomethicon kommerziell erhältlich.

Bevorzugte Öl- bzw. Fettkomponenten B) sind ausgewählt unter Paraffin und Paraffinölen; Vaselin; natürlichen Fetten und Ölen, wie Castoröl, Sojaöl, Erdnussöl, Olivenöl, Sonnenblumenöl, Sesamöl, Avocadoöl, Kakaobutter, Mandelöl, Pfirsichkernöl, Ricinusöl, Lebertran, Schweineschmalz, Walrat, Spermacetöl, Spermöl, Weizenkeimöl, Macadamianussöl, Nachtkerzenöl, Jojobaöl; Fettalkoholen, wie Laurylalkohol, Myristylalkohol, Cetylalkohol, Stearylalkohol, Oleylalkohol, Cetylalkohol; Fettsäuren, wie Myristinsäure, Stearinsäure, Palmitinsäure, Ölsäure, Linolsäure, Linolensäure und davon verschiedenen gesättigten, ungesättigten und substituierten Fettsäuren; Wachsen, wie Bienenwachs, Carnaubawachs, Candilillawachs, Walrat sowie Mischungen der zuvor genannten Öl- bzw. Fettkomponenten.

Geeignete kosmetisch und pharmazeutisch verträgliche Öl- bzw. Fettkomponenten B) sind in Karl-Heinz Schrader, Grundlagen und Rezepturen der Kosmetika, 2. Auflage, Verlag Hüthig, Heidelberg, S. 319-355 beschrieben, worauf hier Bezug genommen wird.

Geeignete hydrophile Träger B) sind ausgewählt unter Wasser, 1-, 2- oder mehrwertigen Alkoholen mit vorzugsweise 1 bis 8 Kohlenstoffatomen, wie Ethanol, n-Propanol, iso-Propanol, Propylenglycol, Glycerin, Sorbit, etc.

Bei den erfindungsgemäßen kosmetischen Mitteln kann es sich um hautkosmetische, dermatologische oder haarkosmetische Mittel handeln.

Vorzugsweise liegen die erfindungsgemäßen Mittel in Form eines Gels, Schaums, Sprays, einer Salbe, Creme, Emulsion, Suspension, Lotion, Milch oder Paste vor. Gewünschtenfalls können auch Liposomen oder Mikrosphären eingesetzt werden.

Die erfindungsgemäßen kosmetisch oder pharmazeutisch aktiven Mittel können zusätzlich kosmetisch und/oder dermatologisch aktive Wirkstoffe sowie Hilfsstoffe enthalten.

Vorzugsweise enthalten die erfindungsgemäßen kosmetischen Mittel wenigstens ein wie vorstehend definiertes ampholytisches Copolymer oder einen Polyelektrolyt-Komplex A, wenigstens einen wie vorstehend definierten Träger B und wenigstens einen, von Komponente A verschiedenen Bestandteil, der ausgewählt ist unter kosmetisch aktiven Wirkstoffen, Emulgatoren, Tensiden, Konservierungsmitteln, Parfümölen, Verdickern, Haarpolymeren, Haar- und Hautconditionern, verzweigten Polymeren, vernetzten Polymeren, Pfropfpolymeren, wasserlöslichen oder dispergierbaren silikonhaltigen Polymeren, Lichtschutzmitteln, Bleichmitteln, Gelbildnern, Pflegemitteln, Färbemitteln, Tönungsmitteln, Bräunungsmitteln, Farbstoffen, Pigmenten, Konsistenzgebern, Feuchthaltemitteln, Rückfettern, Collagen, Eiweißhydrolysaten, Lipiden, Antioxidantien, Entschäumern, Antistatika, Emollienzien, Weichmachern.

Geeignete kosmetisch und/oder dermatologisch aktive Wirkstoffe sind z. B. färbende Wirkstoffe, Haut- und Haarpigmentierungsmittel, Tönungsmittel, Bräunungsmittel, Bleichmittel, Keratin-härtende Stoffe, antimikrobielle Wirkstoffe, Lichtfilterwirkstoffe, Repellentwirkstoffe, hyperemisierend wirkende Stoffe, keratolytisch und keratoplastisch wirkende Stoffe, Antischuppenwirkstoffe, Antiphlogistika, keratinisierend wirkende Stoffe, antioxidativ bzw. als Radikalfänger aktive Wirkstoffe, hautbefeuchtende oder -feuchthaltende Stoffe, rückfettende Wirkstoffe, antierythimatös oder antiallergisch aktive Wirkstoffe und Mischungen davon.

Künstlich hautbräunende Wirkstoffe, die geeignet sind, die Haut ohne natürliche oder künstliche Bestrahlung mit UV-Strahlen zu bräunen, sind z. B. Dihydroxyaceton, Alloxan und Walnussschalenextrakt. Geeignete Keratin-härtende Stoffe sind in der Regel Wirkstoffe, wie sie auch in Antitranspirantien eingesetzt werden, wie z. B. Kaliumaluminiumsulfat, Aluminiumhydroxychlorid, Aluminiumlactat, etc. Antimikrobielle Wirkstoffe werden eingesetzt, um Mikroorganismen zu zerstören bzw. ihr Wachstum zu hemmen und dienen somit sowohl als Konservierungsmittel als auch als desodorierend wirkender Stoff, welcher die Entstehung oder die Intensität von Körpergeruch vermindert. Dazu zählen z. B. übliche, dem Fachmann bekannte Konservierungsmittel, wie p-Hydroxybenzoesäureester, Imidazolidinyl-Harnstoff, Formaldehyd, Sorbinsäure, Benzoesäure, Salicylsäure, etc. Vorteilhafte Konservierungsmittel sind z. B. Parabenester, wie Methylparaben, Ethylparaben, Propylparaben, etc. Ein geeignetes Gemisch von Parabenestern in Phenoxyethanol ist kommerziell unter der Bezeichnung Phenonip^{®} (Fa. Clariant) erhältlich. Geeignet sind weiterhin chlorierte und nicht chlorierte Isothiazolone. Solche sind beispielsweise unter der Bezeichnung Euxyl^{®} K 100 (Fa. Schülke & Mayr) erhältlich. Derartige desodorierend wirkende Stoffe sind z.B. Zinkricinoleat, Triclosan, Undecylensäurealkylolamide, Citronensäuretriethylester, Chlorhexidin etc. Geeignete Lichtfilterwirkstoffe sind Stoffe, die UV-Strahlen im UV-B- und/oder UV-A-Bereich absorbieren. Geeignete UV-Filter sind z. B. 2,4,6-Triaryl-1,3,5-triazine, bei denen die Arylgruppen jeweils wenigstens einen Substituenten tragen können, der vorzugsweise ausgewählt ist unter Hydroxy, Alkoxy, speziell Methoxy, Alkoxycarbonyl, speziell Methoxycarbonyl und Ethoxycarbonyl und Mischungen davon. Geeignet sind weiterhin p-Aminobenzoesäureester, Zimtsäureester, Benzophenone, Campherderivate sowie UV-Strahlen abhaltende Pigmente, wie Titandioxid, Talkum und Zinkoxid. Geeignete Repellentwirkstoffe sind Verbindungen, die in der Lage sind, bestimmte Tiere, insbesondere Insekten, vom Menschen abzuhalten oder zu vertreiben. Dazu gehört z. B. 2-Ethyl-1,3-hexandiol, N,N-Diethyl-m-toluamid etc. Geeignete hyperemisierend wirkende Stoffe, welche die Durchblutung der Haut anregen, sind z. B. ätherische Öle, wie Latschenkiefer, Lavendel, Rosmarin, Wacholderbeer, Rosskastanienextrakt, Birkenblätterextrakt, Heublumenextrakt, Ethylacetat, Campher, Menthol, Pfefferminzöl, Rosmarinextrakt, Eukalyptusöl, etc. Geeignete keratolytisch und keratoplastisch wirkende Stoffe sind z. B. Salicylsäure, Kalziumthioglykolat, Thioglykolsäure und ihre Salze, Schwefel, etc. Geeignete Antischuppen-Wirkstoffe sind z. B. Schwefel, Schwefelpolyethylenglykolsorbitanmonooleat, Schwefelricinolpolyethoxylat, Zinkpyrithion, Aluminiumpyrithion, etc. Geeignete Antiphlogistika, die Hautreizungen entgegenwirken, sind z. B. Allantoin, Bisabolol, Dragosantol, Kamillenextrakt, Panthenol, etc.

Die erfindungsgemäßen kosmetischen Mittel können als kosmetischen und/oder pharmazeutischen Wirkstoff (wie auch gegebenenfalls als Hilfsstoff) wenigstens ein kosmetisch oder pharmazeutisch akzeptables von Verbindungen der Komponente A) verschiedenes Polymer enthalten. Dazu zählen ganz allgemein Polymere, die keine an das Polymergerüst gebundenen anionogenen, anionischen, kationogenen oder kationischen Gruppen aufweisen.

Dazu zählen neutrale Polymere, wie Polyvinylpyrrolidone, Copolymere aus N-Vinylpyrrolidon und Vinylacetat und/oder Vinylpropionat, Polysiloxane, Polyvinylcaprolactam und andere Copolymere mit N-Vinylpyrrolidon, Polyethylenimine und deren Salze, Polyvinylamine und deren Salze, Cellulosederivate, Polyasparaginsäuresalze und Derivate. Dazu zählt beispielsweise Luviflex^{®} Swing (teilverseiftes Copolymerisat von Polyvinylacetat und Polyethylenglykol, Fa. BASF).

Geeignete Polymere sind auch nichtionische, wasserlösliche bzw. wasserdispergierbare Polymere oder Oligomere, wie Polyvinylcaprolactam, z. B. Luviskol^{®} Plus (BASF), oder Polyvinylpyrrolidon und deren Copolymere, insbesondere mit Vinylestern, wie Vinylacetat, z. B. Luviskol^{®} VA 37 (BASF); Polyamide, z. B. auf Basis von Itaconsäure und aliphatischen Diaminen, wie sie z. B. in der DE-A-43 33 238 beschrieben sind.

Geeignete Polymere sind auch nichtionische, siloxanhaltige, wasserlösliche oder -dispergierbare Polymere, z. B. Polyethersiloxane, wie Tegopren^{®} (Fa. Goldschmidt) oder Belsil^{®} (Fa. Wacker).

Die Formulierungsgrundlage erfindungsgemäßer pharmazeutischer Mittel enthält bevorzugt pharmazeutisch akzeptable Hilfsstoffe. Pharmazeutisch akzeptabel sind die im Bereich der Pharmazie, der Lebensmitteltechnologie und angrenzenden Gebieten bekanntermaßen verwendbaren Hilfsstoffe, insbesondere die in einschlägigen Arzneibüchern (z. B. DAB Ph. Eur. BP NF) gelisteten sowie andere Hilfsstoffe, deren Eigenschaften einer physiologischen Anwendung nicht entgegenstehen.

Geeignete Hilfsstoffe können sein: Gleitmittel, Netzmittel, emulgierende und suspendierende Mittel, konservierende Mittel, Antioxidantien, Antireizstoffe, Chelatbildner, Emulsionsstabilisatoren, Filmbildner, Gelbildner, Geruchsmaskierungsmittel, Harze, Hydrokolloide, Lösemittel, Lösungsvermittler, Neutralisierungsmittel, Permeationsbeschleuniger, Pigmente, quaternäre Ammoniumverbindungen, Rückfettungs- und Überfettungsmittel, Salben-, Creme- oder Öl-Grundstoffe, Siliconderivate, Stabilisatoren, Sterilantien, Treibmittel, Trocknungsmittel, Trübungsmittel, Verdickungsmittel, Wachse, Weichmacher, Weißöle. Eine diesbezügliche Ausgestaltung beruht auf fachmännischem Wissen, wie sie beispielsweise in Fiedler, H. P. Lexikon der Hilfsstoffe für Pharmazie, Kosmetik und angrenzende Gebiete, 4. Aufl., Aulendorf: ECV-Editio-Kantor-Verlag, 1996, dargestellt sind.

Zur Herstellung der erfindungsgemäßen dermatologischen Mittel können die Wirkstoffe mit einem geeigneten Hilfsstoff (Exzipient) vermischt oder verdünnt werden. Exzipienten können feste, halb feste oder flüssige Materialien sein, die als Vehikel, Träger oder Medium für den Wirkstoff dienen können. Die Zumischung weiterer Hilfsstoffe erfolgt gewünschtenfalls in der dem Fachmann bekannten Weise.

Nach einer ersten bevorzugten Ausführungsform handelt es sich bei den erfindungsgemäßen Mitteln um ein Hautreinigungsmittel.

Bevorzugte Hautreinigungsmittel sind Seifen von flüssiger bis gelförmiger Konsistenz, wie Transparentseifen, Luxusseifen, Deoseifen, Cremeseifen, Babyseifen, Hautschutzseifen, Abrasiveseifen und Syndets, pasteuse Seifen, Schmierseifen und Waschpasten, flüssige Wasch-, Dusch- und Badepräparate, wie Waschlotionen, Duschbäder und -gele, Schaumbäder, Ölbäder und Scrub-Präparate.

Nach einer weiteren bevorzugten Ausführungsform handelt es sich bei den erfindungsgemäßen Mitteln um kosmetische Mittel zur Pflege und zum Schutz der Haut, Nagelpflegemittel oder Zubereitungen für die dekorative Kosmetik.

Besonders bevorzugt handelt es sich um Hautpflegemittel, Intimpflegemittel, Fußpflegemittel, Lichtschutzmittel, Repellents, Rasiermittel, Haarentfernungsmittel, Antiaknemittel, Make-ups, Mascara, Lippenstifte, Lidschatten, Kajalstifte, Eyeliner, Rouges und Augenbrauenstifte.

Bei den erfindungsgemäßen Hautpflegemitteln handelt es sich insbesondere um W/O- oder O/W-Hautcremes, Tag- und Nachtcremes, Augencremes, Gesichtscremes, Antifaltencremes, Feuchthaltecremes, Bleichcremes, Vitamincremes, Hautlotionen, Pflegelotionen und Feuchthaltelotionen.

Hautkosmetische und dermatologische Mittel auf Basis der zuvor beschriebenen Polymere oder Polyelektrolyte A) zeigen vorteilhafte Wirkungen. Die Polymere können unter anderem zur Feuchthaltung und Konditionierung der Haut und zur Verbesserung des Hautgefühls beitragen. Die Polymere können auch als Verdicker in den Formulierungen wirken. Durch Zusatz der erfindungsgemäßen Polymere kann in bestimmten Formulierungen eine erhebliche Verbesserung der Hautverträglichkeit erreicht werden.

Hautkosmetische und dermatologische Mittel enthalten vorzugsweise wenigstens ein ampholytisches Copolymer oder einen Polyelektrolyt-Komplex A) in einem Anteil von etwa 0,001 bis 30 Gew.-%, vorzugsweise 0,01 bis 20 Gew.-%, ganz besonders bevorzugt 0,1 bis 12 Gew.-%, bezogen auf das Gesamtgewicht des Mittels.

Besonders Lichtschutzmittel auf Basis der Komponente A) besitzen die Eigenschaft, die Verweilzeit der UV-absorbierenden Inhaltsstoffe im Vergleich zu gängigen Hilfsmitteln wie Polyvinylpyrrolidon zu erhöhen.

Je nach Anwendungsgebiet können die erfindungsgemäßen Mittel in einer zur Hautpflege geeigneten Form, wie z. B. als Creme, Schaum, Gel, Stift, Mousse, Milch, Spray (Pumpspray oder treibmittelhaltiger Spray) oder Lotion appliziert werden.

Die hautkosmetischen Zubereitungen können neben den ampholytischen Copolymeren oder Polyelektrolyt-Komplexen A) und geeigneten Trägern noch weitere in der Hautkosmetik übliche Wirkstoffe und Hilfsstoffe, wie zuvor beschrieben, enthalten. Dazu zählen vorzugsweise Emulgatoren, Konservierungsmittel, Parfümöle, kosmetische Wirkstoffe wie Phytantriol, Vitamin A, E und C, Retinol, Bisabolol, Panthenol, Lichtschutzmittel, Bleichmittel, Färbemittel, Tönungsmittel, Bräunungsmittel, Collagen, Eiweißhydrolysate, Stabilisatoren, pH-Wert-Regulatoren, Farbstoffe, Salze, Verdicker, Gelbildner, Konsistenzgeber, Silicone, Feuchthaltemittel, Rückfetter und weitere übliche Additive.

Bevorzugte Öl- und Fettkomponenten der hautkosmetischen und dermatologischen Mittel sind die zuvor genannten mineralischen und synthetischen Öle, wie z. B. Paraffine, Siliconöle und aliphatische Kohlenwasserstoffe mit mehr als 8 Kohlenstoffatomen, tierische und pflanzliche Öle, wie z. B. Sonnenblumenöl, Kokosöl, Avocadoöl, Olivenöl, Lanolin, oder Wachse, Fettsäuren, Fettsäureester, wie z. B. Triglyceride von C₆-C₃₀-Fettsäuren, Wachsester, wie z. B. Jojobaöl, Fettalkohole, Vaseline, hydriertes Lanolin und azetyliertes Lanolin sowie Mischungen davon.

Man kann die erfindungsgemäßen Polymere auch mit herkömmlichen Polymeren abmischen, falls spezielle Eigenschaften eingestellt werden sollen.

Zur Einstellung bestimmter Eigenschaften wie z. B. Verbesserung des Anfassgefühls, des Spreitverhaltens, der Wasserresistenz und/oder der Bindung von Wirk- und Hilfsstoffen, wie Pigementen, können die hautkosmetischen und dermatologischen Zubereitungen zusätzlich auch konditionierende Substanzen auf Basis von Siliconverbindungen enthalten. Geeignete Siliconverbindungen sind beispielsweise Polyalkylsiloxane, Polyarylsiloxane, Polyarylalkylsiloxane, Polyethersiloxane oder Siliconharze.

Die Herstellung der kosmetischen oder dermatologischen Zubereitungen erfolgt nach üblichen, dem Fachmann bekannten Verfahren.

Bevorzugt liegen die kosmetischen und dermatologischen Mittel in Form von Emulsionen insbesondere als Wasser-in-Öl-(W/O)- oder Öl-in-Wasser(O/W)-Emulsionen vor. Es ist aber auch möglich, andere Formulierungsarten zu wählen, beispielsweise Hydrodispersionen, Gele, Öle, Oleogele, multiple Emulsionen, beispielsweise in Form von W/O/W- oder O/W/O-Emulsionen, wasserfreie Salben bzw. Salbengrundlagen, usw.

Die Herstellung von Emulsionen erfolgt nach bekannten Methoden. Die Emulsionen enthalten neben dem ampholytischen Copolymer oder Polyelektrolyt-Komplex A) in der Regel übliche Bestandteile, wie Fettalkohole, Fettsäureester und insbesondere Fettsäuretriglyceride, Fettsäuren, Lanolin und Derivate davon, natürliche oder synthetische Öle oder Wachse und Emulgatoren in Anwesenheit von Wasser. Die Auswahl der Emulsionstyp-spezifischen Zusätze und die Herstellung geeigneter Emulsionen ist beispielsweise beschrieben in Schrader, Grundlagen und Rezepturen der Kosmetika, Hüthig Buch Verlag, Heidelberg, 2. Auflage, 1989, dritter Teil, worauf hiermit ausdrücklich Bezug genommen wird.

Eine geeignete Emulsion, z.B. für eine Hautcreme etc., enthält im Allgemeinen eine wässrige Phase, die mittels eines geeigneten Emulgatorsystems in einer Öl- oder Fettphase emulgiert ist.

Der Anteil des Emulgatorsystems beträgt in diesem Emulsionstyp bevorzugt etwa 4 und 35 Gew.-%, bezogen auf das Gesamtgewicht der Emulsion. Vorzugsweise beträgt der Anteil der Fettphase etwa 20 bis 60 Gew.-%. Vorzugsweise beträgt der Anteil der wässrigen Phase etwa 20 und 70 %, jeweils bezogen auf das Gesamtgewicht der Emulsion. Bei den Emulgatoren handelt es sich um solche, die in diesem Emulsionstyp üblicherweise verwendet werden. Sie werden z. B. ausgewählt unter: C₁₂-C₁₈-Sorbitan-Fettsäureestern; Estern von Hydroxystearinsäure und C₁₂-C₃₀-Fettalkoholen; Mono- und Diestern von C₁₂-C₁₈-Fettsäuren und Glycerin oder Polyglycerin; Kondensaten von Ethylenoxid und Propylenglykolen; oxypropylenierten/oxyethylierten C₁₂-C₁₈-Fettalkoholen; polycyclischen Alkoholen, wie Sterolen; aliphatischen Alkoholen mit einem hohen Molekulargewicht, wie Lanolin; Mischungen von oxypropylenierten/polyglycerinierten Alkoholen und Magnesiumisostearat; Succinestern von polyoxyethylenierten oder polyoxypropylenierten Fettalkoholen; und Mischungen von Magnesium-, Calcium-, Lithium-, Zink- oder Aluminiumlanolat und hydriertem Lanolin oder Lanolinalkohol.

Bevorzugte Fettkomponenten, welche in der Fettphase der Emulsionen enthalten sein können, sind: Kohlenwasserstofföle, wie Paraffinöl, Purcellinöl, Perhydrosqualen und Lösungen mikrokristalliner Wachse in diesen Ölen; tierische oder pflanzliche Öle, wie Süßmandelöl, Avocadoöl, Calophylumöl, Lanolin und Derivate davon, Ricinusöl, Sesamöl, Olivenöl, Jojobaöl, Karité-Öl, Hoplostethus-Öl; mineralische Öle, deren Destillationsbeginn unter Atmosphärendruck bei ca. 250 °C und deren Destillationsendpunkt bei 410 °C liegt, wie z. B. Vaselinöl; Ester gesättigter oder ungesättigter Fettsäuren, wie Alkylmyristate, z. B. i-Propyl-, Butyl- oder Cetylmyristat, Hexadecylstearat, Ethyl- oder i-Propylpalmitat, Octan- oder Decansäuretriglyceride und Cetylricinoleat.

Die Fettphase kann auch in anderen Ölen lösliche Siliconöle, wie Dimethylpolysiloxan, Methylphenylpolysiloxan und das Siliconglykol-Copolymer, Fettsäuren und Fettalkohole enthalten.

Um die Retention von Ölen zu begünstigen, können neben den ampholytischen Copolymeren oder Polyelektrolyt-Komplexen A) auch Wachse verwendet werden, wie z. B. Carnaubawachs, Candilillawachs, Bienenwachs, mikrokristallines Wachs, Ozokeritwachs und Ca-, Mg- und Al-Oleate, -Myristate, -Linoleate und -Stearate.

Im Allgemeinen werden die Wasser-in-Öl-Emulsionen so hergestellt, dass die Fettphase und der Emulgator in einen Ansatzbehälter gegeben werden. Man erwärmt diesen bei einer Temperatur von etwa 50 bis 75 °C, gibt dann die in Öl löslichen Wirkstoffe und/oder Hilfsstoffe zu und fügt unter Rühren Wasser hinzu, welches vorher etwa auf die gleiche Temperatur erwärmt wurde und worin man gegebenenfalls die wasserlöslichen Ingredienzien vorher gelöst hat. Man rührt, bis man eine Emulsion der gewünschten Feinheit erhält und lässt dann auf Raumtemperatur abkühlen, wobei gegebenenfalls weniger gerührt wird.

Nach einer weiteren bevorzugten Ausführungsform handelt es sich bei den erfindungsgemäßen Mitteln um ein Duschgel, eine Shampoo-Formulierung oder ein Badepräparat.

Solche Formulierungen enthalten wenigstens ein ampholytisches Copolymer oder einen Polyelektrolyt-Komplex A) sowie üblicherweise anionische Tenside als Basistenside und amphotere und/oder nichtionische Tenside als Cotenside. Weitere geeignete Wirkstoffe und/oder Hilfsstoffe sind im Allgemeinen ausgewählt unter Lipiden, Parfümölen, Farbstoffen, organischen Säuren, Konservierungsstoffen und Antioxidantien sowie Verdickern/Gelbildnern, Hautkonditioniermitteln und Feuchthaltemitteln.

Diese Formulierungen enthalten vorzugsweise 2 bis 50 Gew.-%, bevorzugt 5 bis 40 Gew.-%, besonders bevorzugt 8 bis 30 Gew.-% Tenside, bezogen auf das Gesamtgewicht der Formulierung.

In den Wasch-, Dusch- und Badepräparaten können alle in Körperreinigungsmitteln üblicherweise eingesetzten anionische, neutrale, amphotere oder kationische Tenside verwendet werden.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z. B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid- oder Propylenoxideinheiten, bevorzugt 1 bis 3 Ethylenoxideinheiten im Molekül aufweisen.

Dazu zählen z. B. Natriumlaurylsulfat, Ammoniumlaurylsulfat, Natriumlaurylethersulfat, Ammoniumlaurylethersulfat, Natriumlaurylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind z. B. Alkylbetaine, Alkylamidopropylbetaine, Alkylsulfobetaine, Alkylglycinate, Alkylcarboxyglycihate, Alkylamphoacetate oder -propionate, Alkylamphodiacetate oder -dipropionate.

Beispielsweise können Cocodimethylsulfopropylbetain, Laurylbetain, Cocamidopropylbetain oder Natriumcocamphopropionat eingesetzt werden.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60 Mole auf ein Mol Alkohol. Ferner sind Alkylaminoxide, Mono- oder Dialkylalkanolamide, Fettsäureester von Polyethylenglykolen, ethoxylierte Fettsäureamide, Alkylpolyglycoside oder Sorbitanetherester geeignet.

Außerdem können die Wasch-, Dusch- und Badepräparate übliche kationische Tenside enthalten, wie z. B. quaternäre Ammoniumverbindungen, beispielsweise Cetyltrimethylammoniumchlorid.

Zusätzlich können auch weitere übliche kationische Polymere eingesetzt werden, so z. B. Copolymere aus Acrylamid und Dimethyldiallylammoniumchlorid (Polyquaternium-7), kationische Cellulosederivate (Polyquaternium-4, -10), Guarhydroxypropyltrimethylammoniumchlorid (INCI: Hydroxylpropyl Guar Hydroxypropyltrimonium Chloride), Copolymere aus N-Vinylpyrrolidon und quaternisiertem N-Vinylimidazol (Polyquaterinium-16, -44, -46), Copolymere aus N-Vinylpyrrolidon/Dimethylaminoethylmethacrylat, quaternisiert mit Diethylsulfat (Polyquaternium-11) und andere.

Weiterhin können die Duschgel-/Shampoo-Formulierungen Verdicker, wie z. B. Kochsalz, PEG-55, Propylene Glykol Oleate, PEG-120 Methyl Glucose Dioleate und andere, sowie Konservierungsmittel, weitere Wirk- und Hilfsstoffe und Wasser enthalten.

Nach einer weiteren bevorzugten Ausführungsform handelt es sich bei den erfindungsgemäßen Mitteln um ein Haarbehandlungsmittel.

Erfindungsgemäße Haarbehandlungsmittel enthalten vorzugsweise wenigstens ampholytisches Copolymer oder einen Polyelektrolyt-Komplex A) in einer Menge im Bereich von etwa 0,1 bis 30 Gew.-%, bevorzugt 0,5 bis 20 Gew.-%, bezogen auf das Gesamtgewicht des Mittels.

Vorzugsweise liegen die erfindungsgemäßen Haarbehandlungsmittel in Form eines Schaumfestigers, Haarmousses, Haargels, Shampoos, Haarsprays oder Haarschaums vor. Haarsprays umfassen dabei sowohl Aerosolsprays als auch Pumpsprays ohne Treibgas. Haarschäume umfassen sowohl Aerosolschäume wie auch Pumpschäume ohne Treibgas.

Bevorzugte Haarbehandlungsmittel liegen in Form eines Gels oder eines Schaums vor. Ein solches Haarbehandlungsmittel enthält beispielsweise:
a) 0,1 bis 20 Gew.-%, bevorzugt 1 bis 10 Gew.-%, mindestens eines ampholytischen Copolymers oder Polyelektrolyt-Komplexes A), wie zuvor definiert,
b) 0,1 bis 20 Gew.-%, bevorzugt 1 bis 10 Gew.-%, mindestens eines von Komponente A) verschiedenen wasserlöslichen, beispielsweise neutralen Polymers,
c) 0 bis 40 Gew.-% wenigstens eines Trägers (Lösungsmittels), der ausgewählt ist unter C₂-C₅-Alkoholen, insbesondere Ethanol,
d) 0 bis 5 Gew.-%, bevorzugt 0,01 bis 5 Gew.-%, besonders bevorzugt 0,2 bis 3 Gew.-%, wenigstens eines Verdickers,
e) 0 bis 50 Gew.-% eines Treibmittels,
f) 0 bis 10 Gew.-%, bevorzugt 0,1 bis 3 Gew.-%, mindestens eines von a) verschiedenen Festigerpolymers, vorzugsweise eines wasserlöslichen nichtionischen Polymers,
g) 0 bis 1 Gew.-% wenigstens eines Rückfetters, vorzugsweise ausgewählt unter Glycerin und Glycerinderivaten,
h) 0 bis 30 Gew.-% weiterer Wirk- und/oder Hilfsstoffe, z.B. wenigstens eine Siliconverbindung,
i) Wasser ad 100 Gew.-%.

Die Haarbehandlungsmittel können weiterhin in Form von Haarsprays oder Haarschäumen vorliegen. Haarsprays und Haarschäume umfassen vorzugsweise überwiegend oder ausschließlich wasserlösliche oder wasserdispergierbare Komponenten. Sind die in den erfindungsgemäßen Haarsprays und Haarschäumen eingesetzten Verbindungen wasserdispergierbar, können sie in Form von wässrigen Mikrodispersionen mit Teilchendurchmessern von üblicherweise 1 bis 350 nm, bevorzugt 1 bis 250 nm, zur Anwendung gebracht werden. Die Feststoffgehalte dieser Präparate liegen dabei üblicherweise in einem Bereich von etwa 0,5 bis 20 Gew.-%. Diese Mikrodispersionen benötigen in der Regel keine Emulgatoren oder Tenside zu ihrer Stabilisierung.

Bevorzugte Haarbehandlungsmittel liegen in Form einer wässrigen Dispersion oder in Form einer alkoholischen oder wässrig-alkoholischen Lösung vor. Beispiele geeigneter Alkohole sind Ethanol, Propanol, Isopropanol und Mischungen davon.

Weiter können die erfindungsgemäßen Haarbehandlungsmittel im Allgemeinen übliche kosmetische Hilfsstoffe enthalten, beispielsweise Weichmacher, wie Glycerin und Glykol; Emollienzien; Parfüms; Tenside; UV-Absorber; Farbstoffe; antistatische Mittel; Mittel zur Verbesserung der Kämmbarkeit; Konservierungsmittel; und Entschäumer.

Wenn die erfindungsgemäßen Mittel als Haarspray formuliert sind, enthalten sie eine ausreichende Menge eines Treibmittels, beispielsweise einen niedrigsiedenden Kohlenwasserstoff oder Ether, wie Propan, Butan, Isobutan oder Dimethylether. Als Treibmittel sind auch komprimierte Gase brauchbar, wie Stickstoff, Luft oder Kohlendioxid. Die Menge an Treibmittel kann dabei gering gehalten werden, um den VOC-Gehalt nicht unnötig zu erhöhen. Sie beträgt dann im Allgemeinen nicht mehr als 55 Gew.-%, bezogen auf das Gesamtgewicht des Mittels. Gewünschtenfalls sind aber auch höhere VOC-Gehälte von 85 Gew.-% und darüber möglich.

Die erfindungsgemässen ampholytischen Copolymere können auch in Kombination mit erfindungsgemäßen Polyelektrolyt-Komplexen oder in Kombination mit anderen nichtionischen Haarpolymeren in den Mitteln zur Anwendung kommen. Geeignete Polymere sind die zuvor beschriebenen.

Die anderen Haarpolymere sind vorzugsweise in Mengen bis zu 10 Gew.-%, bezogen auf das Gesamtgewicht des Mittels enthalten.

Ein bevorzugtes Haarbehandlungsmittel in Form eines Haarsprays enthält:
a) 0,3 bis 20 Gew.-%, bevorzugt 0,5 bis 10 Gew.-%, insbesondere 1 bis 5 Gew.-%, mindestens eines Polymers A), wie zuvor definiert,
b) 50 bis 99,5 Gew.-%, bevorzugt 55 bis 99 Gew.-%, eines Trägers (Lösungsmittels), ausgewählt unter Wasser und wassermischbaren Lösungsmitteln, bevorzugt C₂-C₅-Alkoholen, insbesondere Ethanol, und Mischungen davon,
c) 0 bis 50 Gew.-%, bevorzugt 0,1 bis 40 Gew.-%, eines Treibmittels, vorzugsweise ausgewählt unter Dimethylether und Alkanen, wie z. B. Propan/Butan-Gemischen,
d) 0 bis 10 Gew.-%, bevorzugt 0,1 bis 10 Gew.-%, insbesondere 0,2 bis 7 Gew.-%, mindestens eines von a) verschiedenen Haarpolymers, vorzugsweise eines in Wasser löslichen oder dispergierbaren Polymers,
e) 0 bis 0,5 Gew.-%, bevorzugt 0,001 bis 2 Gew.-%, mindestens einer wasserlöslichen oder wasserdispergierbaren Siliconverbindung,
sowie gegebenenfalls weitere Wirkstoffe und/oder Hilfsstoffe, wie zuvor definiert.

Das erfindungsgemäße Mittel kann als Komponente e) mindestens ein nichtionisches, siloxanhaltiges, wasserlösliches oder -dispergierbares Polymer, insbesondere ausgewählt unter den zuvor beschriebenen Polyethersiloxanen, enthalten. Der Anteil dieser Komponente beträgt dann im Allgemeinen etwa 0,001 bis 2 Gew.-%, bezogen auf das Gesamtgewicht des Mittels.

Die ampholytischen Copolymere oder Polyelektrolyt-Komplexe A) eignen sich in vorteilhafter Weise als Hilfsmittel in der Pharmazie, zur Modifizierung rheologischer Eigenschaften (als Verdikker), als oberflächenaktive Substanz (polymerer Emulgator), bevorzugt als oder in Beschichtungsmittel(n) für die Textil-, Papier-, Druck- und Lederindustrie.

Die Erfindung wird anhand der folgenden nicht einschränkenden Beispiele näher erläutert.

### Beispiele:

### 1. Herstellung von Copolymerisaten (Lösungspolymerisation)

### Beispiel 14: Copolymer aus VP/ MAM/ DADMAC/ DMAPMAM/ MAS

### Zulauf 1: Monomerengemisch aus:

| | |
|---|---|
| 300 g | Vinylpyrrolidon |
| 1200 g | einer 15%igen wässrigen Lösung von Methacrylamid (= 180 g Methacrylamid und 1020 g Wasser) |
| 95 g | Diallyldimethylammoniumchlorid |
| 42 g | Dimethylaminopropylmethacrylamid |
| 21 g | Methacrylsäure |

### Zulauf 2: Initiatorlösung aus:

| | |
|---|---|
| 6 g | Wako V 50 [2,2'-Azobis(2-amidinopropan)-dihydrochlorid] und |
| 123 g | Wasser |

### Zulauf 3: Initiatorlösung aus:

| | |
|---|---|
| 4 g | Wako V 50 [2,2'-Azobis(2-amidinopropan)-dihydrochlorid] und |
| 82 g | Wasser |

In einer Rührapparatur mit Rückflusskühler, Innenthermometer und 3 separaten Zulaufvorrichtungen wurden 166 g von Zulauf 1, 12,9 g von Zulauf 2 und 137 g Wasser vorgelegt und die Mischung unter Rühren auf ca. 65 °C aufgeheizt. Nach dem Anpolymerisieren, erkennbar an einer beginnenden Viskositätserhöhung, wurde bei 65 °C der Rest von Zulauf 1 innerhalb von 3 h und der Rest von Zulauf 2 in 4 h zugegeben, wobei die Innentemperatur auf ca. 68 °C erhöht wurde. Nach dem Ende der Zugabe wurde das Reaktionsgemisch noch ca. 2 h bei 70°C gerührt. Anschließend wurde der Zulauf 3 innerhalb von 30 Minuten bei einer Temperatur von 70°C hinzugefügt und die Polymerlösung anschließend noch ca. 2 h bei einer Temperatur von ca. 80°C nachpolymerisiert. Die Polymerlösung wurde 2 h mit Wasserdampf behandelt. Man erhielt eine ca. 30%ige Polymerlösung.

Zum Stabilisieren wurde die Lösung mit 100 ppm Euxyl K100 der Fa. Schülke & Mayr (5-Chlor-2-methyl-3-(2)-isothiazolon/2-Methyl-3-(2H)-isothiazolon/Benzylalkohol) versetzt.

Pulverförmige Produkte wurden durch Sprühtrocknen oder Gefriertrocknen erhalten.

Analog wurden die Polymere 1 bis 13 hergestellt.

### 2. Herstellung von Copolymerisaten (Fällungspolymerisation)

### Beispiel 15: Copolymer aus VP/ VFA/ DADMAC/ DMAPMAM/ MAS/ DAWA

### Zulauf 1: Monomerengemisch aus:

| | |
|---|---|
| 240 g | Vinylpyrrolidon |
| 240 g | Vinylformamid |
| 105 g | 60%iges Diallyldimethylammoniumchlorid (63 g und 42 g Wasser) |
| 36 g | Dimethylaminopropylmethacrylamid |
| 18 g | Methacrylsäure |
| 6 g | 50%iges Diallylweinsäurediamid (3 g und 3 g Wasser) |

### Zulauf 2: Initiatorlösung aus:

| | |
|---|---|
| 1,8 g | Wako V 50 [2,2'-Azobis(2-amidinopropan)-dihydrochlorid] und |
| 25 g | Essigester |

In einer Rührapparatur mit Rückflusskühler, Innenthermometer und zwei separaten Zulaufvorrichtungen wurden 65 g von Zulauf 1 und 2,7 g von Zulauf 2 in 1370 g Essigester vorgelegt und die Mischung unter Rühren auf ca. 75 °C aufgeheizt. Nach dem Anpolymerisieren, erkennbar an einer leichten Trübung, wurde der Rest von Zulauf 1 innerhalb von drei Stunden und der Rest von Zulauf 2 innerhalb von vier Stunden zugegeben, wobei die Innentemperatur auf ca. 78 °C erhöht wurde. Die Reaktionslösung wurde weiter unter Rückfluss zwei Stunden gerührt. Das Produkt fällt in Form eines feinen Pulvers aus. Nach dem Abkühlen wird das Polymer abgesaugt, dreimal mit Aceton gespült und im Trockenschrank bei 40 °C unter Vakuum über Nacht getrocknet.

Analog wurden die Polymere Nr. 16 - 30 hergestellt.

**Tabelle 1:**

| | VP [Gew.%] | Vcap [Gew.%] | MAM [Gew.%] | VFA [Gew.%] | DADMAC [Gew.%] | DMAP- MAM:MAS [Gew.%] | VI:AS [Gew.%] | DAWA [Gew.%] | MBAA [Gew.%] |
|---|---|---|---|---|---|---|---|---|---|
| 1 | 70 | 27 | -- | -- | -- | -- | 2,5: 0,5 | -- | -- |
| 2 | 70 | -- | 26 | -- | -- | -- | 3:1 | -- | -- |
| 3 | 70 | | -- | 26 | -- | -- | 3:1 | -- | -- |
| 4 | 60 | 37 | -- | -- | -- | -- | 2,5: 0,5 | -- | -- |
| 5 | 60 | -- | 37 | -- | -- | -- | 2,5: 0,5 | -- | -- |
| 6 | 60 | | -- | 37 | -- | -- | 2,5: 0,5 | -- | -- |
| 7 | 60 | -- | 35,5 | -- | -- | 3:1,5 | -- | -- | -- |
| 8 | 60 | | -- | 35,5 | -- | 3:1,5 | -- | -- | -- |
| 9 | 60 | 29,5 | -- | -- | -- | 7:3,5 | -- | -- | -- |
| 10 | 60 | -- | 29,5 | -- | -- | 7:3,5 | -- | -- | -- |
| 11 | 80 | -- | -- | -- | 9,5 | 7 : 3 , 5 | -- | -- | -- |
| 12 | 60 | -- | 20 | -- | 9,5 | -- | 8:2,5 | -- | -- |
| 13 | 50 | -- | 30 | -- | 9,5 | 7:3,5 | -- | -- | -- |
| 14 | 40 | -- | -- | 40 | 9,5 | -- | 8:2,5 | -- | -- |
| 15 | 40 | -- | -- | 40 | 10,5 | 6:3 | -- | 0,5 | -- |
| 16 | 30 | -- | -- | 30 | 19,5 | 13:7 | -- | 0,5 | -- |
| 17 | 30 | -- | -- | 30 | 9,5 | 20:10 | -- | 0,5 | -- |
| 18 | 26 | -- | -- | 40 | 9,5 | -- | 18:6 | -- | 0,5 |
| 19 | -- | -- | -- | 64,5 | 5 | -- | 23:7 | -- | 0,5 |
| 20 | -- | -- | -- | 64,5 | 5 | 20:10 | -- | -- | 0,5 |

**Tabelle 2:**

| | VP [Gew.%] | Vcap [Gew.%] | SMA [Gew.%] | VFA [Gew.%] | DMAP-MAM:MAS [Gew.%] | MBAA [Gew.%] |
|---|---|---|---|---|---|---|
| 21 | 63,8 | -- | -- | -- | 24:12 | 0,2 |
| 22 | 40 | 23,8 | -- | -- | 24:12 | 0,2 |
| 23 | 40 | -- | -- | 23,8 | 24:12 | 0,2 |
| 24 | -- | 23,8 | -- | 40 | 24:12 | 0,2 |
| 25 | -- | -- | -- | 63,8 | 24:12 | 0,2 |
| 26 | 52 | -- | 2,8 | -- | 30:15 | 0,2 |
| 27 | 30 | 22 | 2,8 | -- | 30:15 | 0,2 |
| 28 | 30 | -- | 2,8 | 22 | 30:15 | 0,2 |
| 29 | -- | 30 | 2,8 | 22 | 30:15 | 0,2 |
| 30 | -- | -- | 2,8 | 52 | 30:15 | 0,2 |

| | | | | | | |
|---|---|---|---|---|---|---|
| VP = Vinylpyrrolidon VCap = Vinylcaprolactam MAM = Methacrylsäureamid VFA = Vinylformamid DADMAC = Diallyldimethylammoniumchlorid DMAPMAM = Dimethylaminopropylmethacrylsäureamid MAS = Methacrylsäure VI = Vinylimidazol AS = Acrylsäure DAWA = N,N'-Diallylweinsäurediamid MBAA = Methylenbisacrylamid SMA = Stearylmethacrylat | | | | | | |

### 3. Anwendungstechnische Beispiele

Verwendung in der Haarkosmetik:

### 1) Haargele mit einem anionischen Verdicker: Beispiele Nr. 21-28

| Phase 1: | [%] | CTFA |
|---|---|---|
| Polymer 1-8 (30%ige wässrige Lösung) | 10,0 | |
| Glycerin | 0,3 | |
| Wasser dest. Weitere Zusatzstoffe: Konservierungs- | 39,2 | |
| mittel, lösliches ethoxiliertes Silikon, Parfüm | | q.s. |

| Phase 2: | | |
|---|---|---|
| Carbopol 940 (1%ige wässrige Suspension) | 30,0 | Carbomer |
| Triethanolamin | 0,5 | |
| Wasser dest. | 20,0 | |

Zur Herstellung von Haargelen werden die Komponenten eingewogen und homogenisiert. Dabei bildet die Phase 2 ein klares, festes Gel, in das Phase 1 langsam eingerührt wird.

### 2) Haargele mit einem weiteren Festigerpolymer und anionischem Verdicker: Beispiele Nr. 29 - 36

| Phase 1: | [%] | CTFA |
|---|---|---|
| Polymer 1-8 (30%ige wässrige Lösung) | 7,0 | |
| Luviskol VA 64 | 1,0 | Vinylpyrrolidon-vinylacetat-Copolymer |
| Belsil DMC 60 31 | 0,1 | ethoxiliertes Polydimethylsiloxan |
| Uvinul MS 40 | 0,2 | Benzophenon-4 |
| Glycerin | 0,1 | |
| D-Panthenol USP | 0,1 | Panthenol |
| Ethanol | 20,0 | |
| Wasser dest. | 21,0 | |
| Weitere Zusatzstoffe: Konservierungs-mittel, lösliches ethoxyliertes Silikon, Parfüm | q.s. | |

| Phase 2: | | |
|---|---|---|
| Carbopol 940 (1%ige wässrige Suspension) | 30,0 | Carbomer |
| Triethanolamin | 0,5 | |
| Wasser dest. | 20,0 | |

Herstellung: Die Komponenten der beiden Phasen werden nach dem Einwiegen homogenisiert. Phase 2 bildet ein klares, festes Gel. Phase 1 wird langsam in Phase 2 eingerührt.

### 3) Flüssige Haargele: Beispiele Nr. 37 - 50

| | [%] | CTFA |
|---|---|---|
| Polymer 1-14 (30%ige wässrige Lösung) | 5,3 | |
| Natrosol 250 L (2%ige wässrige Lösung) | 25,0 | Hydroxyethyl-cellulose (Fa. Hercules) |
| C-Dry MD 1915 (10%ige wässrige Lösung) | 25,0 | abgebaute Stärke (Fa. Cerestar) |
| Wasser dest. | 44,7 | |
| Weitere Zusatzstoffe: Konservierungsmittel, lösliches ethoxyliertes Silikon, Parfüm | q.s. | |

Herstellung: Einwiegen und bei Raumtemperatur langsam Homogenisieren.

### 4) Kationische selbstverdickende Haargele (ohne zusätzlichen Verdicker): Beispiele Nr. 51 - 56

| Phase 1: | [%] | CTFA |
|---|---|---|
| Polymer 15-20 (in Pulverform) | 3,0 | |
| Glycerin | 0,1 | |
| Wasser dest. | 96,9 | |
| Weitere Zusatzstoffe: Konservierungsmittel, lösliches ethoxyliertes Silikon, Parfüm | q.s. | |

| Phase 2: | [%] | CTFA |
|---|---|---|
| 45%ige Phosphorsäure | | |

Herstellung: Die Komponenten der Phase 1 werden eingewogen, bei 40 °C homogenisiert und anschließend unter Rühren Phase 2 zugegeben, bis ein pH-Wert von 5,5 bis 6 erreicht war.

### 5) Anionische selbstverdickende Haargele (ohne zusätzlichen Verdicker): Beispiele Nr. 57 -60

Phase 1: [%] CTFA

| | |
|---|---|
| Polymer 17-20 (in Pulverform) | 3,0 |
| Glycerin | 0,1 |
| Wasser dest. Weitere Zusatzstoffe: Konservierungs- | 96,9 |
| mittel, lösliches ethoxyliertes Silikon, Parfüm | q.s. |

| Phase 2: | [%] | CTFA |
|---|---|---|
| Aminomethylpropanol (45%ige wässrige Lösung) | | |

Herstellung: Die Komponenten der Phase 1 werden eingewogen, bei 40 °C homogenisiert und anschließend unter Rühren Phase 2 zugegeben, bis ein pH-Wert von 8,5 erreicht war.

### 4. Herstellung von Copolymerisaten auf Basis von 2-Acrylamido-2-methylpropansulfonsäure

Allgemeine Herstellungsvorschrift: Lösungspolymerisation in Ethanol/Wasser (1:1)

### Beispiel 60:

500 g einer 30%igen Polymerlösung (AMPS/ Na-AMPS/ DMAPMAM/ VP/ FettalkoholethoxilatMA)

### Zulauf 1: Monomerengemisch aus :

| | |
|---|---|
| 120 g | Vinylpyrrolidon |
| 7,5 g | C₁₆/C₁₈-Fettalkoholethoxilatmethacrylat (25 EO) |
| 7,5 g | 2-Acrylamido-2-methylpropansulfonsäure (AMPS) |
| 7,5 g | AMPS-Natriumsalz |
| 7,5 g | Dimethylaminopropylmethacrylamid |

### Zulauf 2: Initiatorlösung aus:

| | |
|---|---|
| 0,3 g | Wako^{®} 50 [2,2'-Azobis(2-amindinopropan)dihydrochlorid] |
| 105 g | Wasser |

### Zulauf 3: Initiatorlösung aus:

| | |
|---|---|
| 0,75 g | tert.-Butylperpivalat 75%ig |
| 61,5 g | Ethanol |

### Zulauf 4:

| | |
|---|---|
| 1 g | NaOH |
| 28 g | Wasser |

In einer Rührapparatur mit Rückflusskühler, Innenthermometer und vier separaten Zulaufvorrichtungen wurden 11 g von Zulauf 1, 5 g von Zulauf 2, 70 g Wasser und 44 g Ethanol vorgelegt und die Mischung unter Rühren auf ca. 70 °C aufgeheizt. Nach dem Anpolymerisieren, erkennbar an einer leichten Viskositätserhöhung, wurde bei 70 °C der Rest von Zulauf 1 innerhalb von drei Stunden und der Rest von Zulauf 2 innerhalb von vier Stunden zugegeben, wobei die Innentemperatur auf ca. 73 °C erhöht wurde. Die Reaktionslösung wurde noch ca. zwei Stunden bei 70 °C nachgerührt und anschließend der Zulauf 3 innerhalb von 30 Minuten bei 70 °C zudosiert. Nach der Zugabe wurde noch ca. zwei Stunden bei einer Temperatur von 80 °C nachpolymerisiert. Die Polymerlösung wurde mit NaOH-Lösung (Zulauf 4, Zugabedauer 10 Minuten) auf pH 8 eingestellt. Man erhielt eine ca. 30%ige wässrig/ethanolische Lösung.

Analog wurden die Polymere Nr. 31 - 59 und 61 - 80 hergestellt.

**Tabelle 3**

| Bsp.-Nr. | VP [Gew.-%] | MAM [Gew.-%] | AMPS [Gew.-%] | DMAPMAM [Gew.-%] | VI [Gew.-%] | NtBAEMA [Gew.-%] | FAEMA [Gew.-%] | Plex 6877-0 [Gew.-%] | MBAA | neutr. auf pH 6-8 mit |
|---|---|---|---|---|---|---|---|---|---|---|
| 31 | 98 | - | 0,55 | 0,45 | - | - | 1,0 | - | - | AMP |
| 32 | 97 | - | 0,65 | - | 0,35 | - | 2,0 | - | - | AMP |
| 33 | 97 | - | 0,55 | 0,45 | - | - | 2,0 | - | - | AMP |
| 34 | 96,5 | - | 0,55 | - | - | 0,45 | 2,5 | - | - | AMP |
| 35 | 95 | - | 1,1 | 0,9 | - | - | 3,0 | - | - | AMP |
| 36 | 94 | - | 0,55 | 0,45 | - | - | 5,0 | - | - | AMP |
| 37 | 90 | - | 3,4 | - | 1,6 | - | 5,0 | - | - | AMP |
| 38 | 90 | - | 2,7 | 2,3 | - | - | 5,0 | - | - | AMP |
| 39 | 90 | - | 2,7 | - | - | 2,3 | 5,0 | - | - | AMP |
| 40 | 87 | - | 3,4 | - | 1,6 | - | 8,0 | - | - | AMP |
| 41 | 87 | - | 2,7 | 2,3 | - | - | 8,0 | - | - | AMP |
| 42 | 85 | - | 5,4 | 4,6 | - | - | 5,0 | - | - | NaOH***) |
| 43 | 75 | - | 11 | 9 | - | - | 5,0 | - | - | NaOH***) |
| 44 | 70 | - | 11 | 9 | - | - | 10,0 | - | - | NaOH***) |
| 45 | 55 | - | 22 | 18 | - | - | 5,0 | - | - | NaOH***) |
| 46 | 77 | 20 | 0,55 | 0,45 | - | - | 2,0 | - | - | AMP |
| 47 | 65 | 30 | 1,1 | 0,9 | - | - | 3,0 | - | - | AMP |
| 48 | 60 | 30 | 3,4 | - | 1,6 | - | 5,0 | - | - | AMP |
| 49 | 55 | 30 | 5,4 | 4,6 | - | - | 5,0 | - | - | AMP |
| 50 | 94 | - | 0,55 | 0,45 | - | - | - | 5,0 | - | AMP |
| 51 | 91 | - | 0,55 | 0,45 | - | - | - | 8,0 | - | AMP |
| 52 | 79 | - | 0,55 | 0,45 | - | - | - | 20 | - | AMP |
| 53 | 75 | - | 3,4 | - | 1,6 | - | - | 20 | - | AMP |
| 54 | 70 | - | 5,4 | 4,6 | - | - | - | 20 | - | AMP |
| 55 | 55 | 36 | 0,55 | 0,45 | - | - | - | 8,0 | - | AMP |
| 56 | 50 | 25 | 3,4 | 1,6 | - | - | - | 20 | - | AMP |
| 57 | 96 | - | 0,3/ 0,9*) | 0,3 | - | - | 2,5 | - | - | NaOH |
| 58 | 93,5 | - | 0,3/ 0,9*) | 0,3 | - | - | 5,0 | - | - | NaOH |
| 59 | 85 | - | 2,5/ 5,0*) | 2,5 | - | - | 5,0 | - | - | NaOH |
| 60 | 80 | - | 5,0/ 5,0*) | 5,0 | - | - | 5,0 | - | - | NaOH |
| 61 | 80 | - | 6,5/ 5,0*) | - | 3,5 | - | 5,0 | - | - | NaOH |
| 62 | 70 | - | 6,5/ 15,0*) | - | 3,5 | - | 5,0 | - | - | NaOH |
| 63 | 88,5 | - | 0,3/ 0,9*) | 0,3 | - | - | - | 10 | - | NaOH |
| 64 | 78,5 | - | 0,3/ 0,9*) | 0,3 | - | - | - | 20 | - | NaOH |
| 65 | 70 | - | 2,5/ 5,0*) | 2,5 | - | - | - | 20 | -' | NaOH |
| 66 | 65 | - | 5,0/ 5,0*) | 5,0 | - | - | - | 20 | - | NaOH |
| 67 | 95 | - | 0,4 | - | 0,3/ 3,3**) | - | 1,0 | - | - | AMP |
| 68 | 97,5 | - | 3,0 | - | 2,0/ 5,0**) | - | 2,5 | - | - | AMP |
| 69 | 70 | - | 6,5 | - | 3,5/ 15**) | - | 5,0 | - | - | AMP |
| 70 | 81 | - | 0,4 | - | 0,3/ 8,3**) | - | - | 10 | - | AMP |
| 71 | 60 | - | 1,0 | - | 0,6/ 18,4**) | - | - | 20 | - | AMP |
| 72 | 96,5 | - | 0,5 | 0,4 | 0,4 | - | 2,5 | - | 0,1 | AMP |
| 73 | 90 | - | 2,65 | 2,3 | 2,3 | - | 5,0 | - | 0,05 | AMP |
| 74 | 85 | - | 6,5 | - | - | - | 5,0 | - | 0,07 | AMP |
| 75 | 85 | - | 3,5/ 4,0*) | - | - | - | 5,0 | - | 0,07 | AMP |
| 76 | 89 | - | 0,5 | 0,4 | 0,4 | - | - | 10 | 0,1 | AMP |
| 77 | 75 | - | 2,65 | 2,3 | 2,3 | - | - | 20 | 0,05 | AMP |
| 78 | 70 | - | 3,0/ 3,93*) | 3,0 | 3,0 | - | - | 20 | 0,07 | AMP |
| 79 | 55 | 34 | 0,5 | 0,4 | 0,4 | - | - | 10 | 0,1 | AMP |
| 80 | 50 | 25 | 2,65 | 2,3 | 2,3 | - | - | 20 | 0,05 | AMP |

| | | | | | | | | | | |
|---|---|---|---|---|---|---|---|---|---|---|
| VP = Vinylpyrrolidon MAM = Methacrylsäureamid AMPS = 2-Acrylamido-2-methylpropansulfonsäure *) = AMPS-Natriumsalz DMAPMAM = Dimethylaminopropylmethacrylamid VI = Vinylimidazol **) = VI quaternisiert mit Dimethylsulfat NtBAEMA = tert.Butylaminoethylmethacrylat FAEMA = C₁₆/C₁₈-Fettalkoholethoxilatmethacrylat (25 EO) Plex 6877-0 = Gemisch aus 25 Gew.-% C₁₆/C₁₈-Fettalkoholethoxilatmethacrylat (25 EO) und 75 Gew.-% Methylmethacrylat (Fa. Röhm) MBAA = Methylenbisacrylamid. AMP = 2-Amino-3-methylpropanol ***) zuerst mit NaOH auf einen pH-Wert > 8 eingestellt, dann durch Zugabe von Milchsäure auf pH 6-8 eingestellt | | | | | | | | | | |

### Anwendungstechnische Beispiele:

### Anwendung in der Haarkosmetik:

### 1. Conditioner Shampoo (Beispiele Nr. 81 - 130)

| | [%] |
|---|---|
| A) Texapon NSO 28%ig | |
| (Sodium Laureth Sulfphate/Henkel) | 50,0 |
| Comperlan KD (Coamide DEA/Henkel) | 1,0 |
| Polymer 31-80 (20%ige wässrige Lösung) | 3,0 |
| Wasser | 17,0 |
| q. s. Parfümöl | 27,5 |
| | |
| B) Wasser | 27,5 |
| Natriumchlorid | 1,5 |
| q. s. Konservierungsmittel | |

Herstellung: Einwiegen und unter Rühren die Phasen A und B getrennt lösen und mischen, Phase B langsam in die Phase A einrühren.

### 2. Schaumfestiger: (Beispiele 131 - 180)

| | [%] |
|---|---|
| Polymer 31-80 (20 %ige wässrige Lösung) | 5,0 |
| Cremophor A 25 (Ceteareth 25/BASF) | 0,2 |
| Comperland KD (Coamide DEA/Henkel) | 0,1 |
| Wasser | 84,7 |
| Dimethylether 3,5 bar (20°C) | 10,0 |
| Weiterer Zusatz: Parfüm, Konservierungsmittel | |

Herstellung: Einwiegen und unter Rühren lösen, Abfüllen und Treibgas zusetzen.

### 3. Haargele mit einem anionischen Verdicker: (Beispiele Nr. 181 - 230)

| | [%} CTFA |
|---|---|
| Phase 1: | |
| Polymer 31-80 (20%ige Lösung) | 15,0 |
| Glycerin | 0,3 |
| Wasser dest. 2-Amino-2-methylpropanol bis pH 8 | 34,2 |
| Weiterer Zusatz: Konservierungsmittel, lösliches ethoxiliertes Silikon, Parfüm ... | |
| | |

| Phase 2: | |
|---|---|
| Aculyn 28 (1%ige wässrige Suspension) | 50,0 |
| 2-Amino-2-methylpropanol | 0,5 |

Herstellung: Einwiegen und Homogenisieren. In Phase 2 bildet sich ein klares festes Gel. Die Phase 1 langsam in die Phase 2 einrühren.

### 4. Haargele mit einem weiteren Festigerpolymer und Verdicker: (Beispiele Nr. 231 - 280)

| | [%] | CTFA |
|---|---|---|
| Phase 1: | | |
| Polymer 31-80 (20%ige wässrige Lösung) | 7,0 | |
| Luviset Clear^{®} | 1,0 | VP/Methacrylamide/Vinylimidazole Copolymer |
| Belsil DMC 6031 | 0,1 | ethoxiliertes Polysiloxan (Goldschmidt) |
| Uvinul MS 40 | 0,2 | Benzophenone-4 |
| Glycerin | 0,1 | |
| D-Panthenol USP | 0,3 | Panthenol |
| Ethanol | 10,0 | |
| Wasser dest. | 31,0 | |
| Weiterer Zusatz: Konservierungsmittel, lösliches ethoxiliertes Silikon, Parfüm ... | | |
| | | |
| Phase 2: | | |
| Aculyn 28 (1%ige wässrige Suspension) | 30,0 | |
| 2-Amino-2-methylpropanol | 0,3 | |
| Wasser dest. | 20,0 | |

Herstellung: Einwiegen und Homogenisieren. Die Phase 1 langsam in die Phase 2 einrühren. In Phase 2 bildet sich ein klares festes Gel.

### 5. Anionische selbstverdickende Haargele (Beispiele Nr. 281-330) (ohne zusätzlichen Verdicker):

| | [%] | CTFA |
|---|---|---|
| Polymer 31-80 (20%ige wässrige Lösung) | 10,0 | |
| Luviset Clear^{®} | 2,0 | VP/Methacrylamide/Vinylimidazole Copolymer |
| Uvinul MS 40 | 0,2 | Benzophenone-4 |
| D-Panthenol USP | 0,3 | Panthenol |
| Wasser dest. | 57,5 | |
| Weiterer Zusatz: Konservierungsmittel, lösliches ethoxiliertes Silikon, Parfüm ... | | |

Herstellung: Einwiegen und Homogenisieren. Die Polymerlösung wird dann mit 2-Amino-2-methylpropanol (25%ig) auf pH 7,5 eingestellt. Es bildet sich dabei ein viskoses klares Gel.

### Verwendung in der Hautkosmetik:

### 6. Standard O/W-Creme: (Beispiele Nr. 331-380)

| Ölphase | % | CTFA-Name |
|---|---|---|
| Cremophor A6 | 3,0 | Ceteareth-6 (and) Stearyl Alkohol |
| Cremophor A25 | 3,0 | Ceteareth-25 |
| Glycerinmonostearat s.e. | 2,5 | Glyceryl Stearate |
| Paraffinöl | 7,5 | Paraffin Oil |
| Cetylakohol | 3,5 | Cetyl Alkohol |
| Luvitol EHO | 3,2 | Cetearyl Octanoate |
| Vitamin-E-acetate | 1,0 | Tocopheryl Acetate |
| Nip-Nip | 0,1 | Methyl- und Propyl-4-hydroxybenzoate (7:3) |

| Wasserphase: | % | |
|---|---|---|
| Polymer 31-80 (20%ige wässrige Lösung) Wasser 1,2-Propylenglykol Germall II | 3,0 | |
| | 74,6 | Water |
| | 1,5 | Propylene Glycol |
| | 0,1 | Imidazolidinyl-Urea |

Herstellung: Einwiegen und unter Rühren die Ölphase und Wasserphase getrennt bei einer Temperatur von ca. 80 °C homogeniserien. Die Wasserphase langsam in die Ölphase einrühren. Unter Rühren langsam auf Raumtemperatur abkühlen.

## Patentansprüche

1. Kosmetisches oder pharmazeutisches Mittel, enthaltend
A) wenigstens ein ampholytisches Copolymer, erhältlich durch radikalische Copolymerisation von
a) wenigstens einer Verbindung mit einer radikalisch polymerisierbaren, α,β-ethylenisch ungesättigten Doppelbindung und mindestens einer anionogenen und/oder anionischen Gruppe pro Molekül,
b) wenigstens einer Verbindung mit einer radikalisch polymerisierbaren, α,β-ethylenisch ungesättigten Doppelbindung und mindestens einer kationogenen und/oder kationischen Gruppe pro Molekül,
c) wenigstens einer α,β-ethylenisch ungesättigten amidgruppenhaltigen Verbindung der allgemeinen Formel I 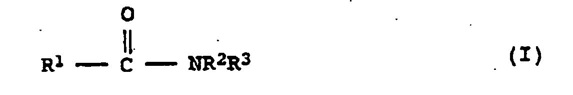
worin
einer der Rest R¹ bis R³ für eine Gruppe der Formel CH₂=CR⁴- mit R⁴ = B oder C₁-C₄-ALkyl steht und die übrigen Reste R¹ bis R³ unabhängig voneinander für H, Alkyl, Cycloalkyl, Heterocycloalkyl, Aryl oder Betaryl stehen,
wobei R¹ und R² gemeinsam mit der Amidgruppe, an die sie gebunden sind, auch für ein Lactam mit 5 bis 8 Ringatomen stehen können,
wobei R² und R³ gemeinsam mit dem Stickstoffatom, an das sie gebunden sind, auch für einen fünf- bis siebengliedrigen Heterocyclus stehen können,
mit der Maßgabe, dass die Summe der Kohlenstoffatome der Reste R¹, R² und R³ höchstens 8 beträgt,
oder einen Polyelektrolytkomplex, enthaltend wenigstens ein solches ampholytisches Copolymer und wenigstens einen weiteren, davon verschiedenen Polyelektrolyten und
B) wenigstens einen kosmetisch akzeptablen Träger.

2. Mittel nach Anspruch 1, wobei das Molmengenverhältnis von Verbindungen a) zu Verbindungen b) in einem Bereich von 0,5:1 bis weniger als 2:1, bevorzugt von 0,7:1 bis 1,8:1, liegt.

3. Mittel nach einem der vorhergehenden Ansprüche, wobei wenigstens ein Teil der Verbindungen a) und b) in Form einer Monomerzusammensetzung eingesetzt wird, wobei das molare verhältnis von anionogenen Gruppen der Komponente a) zu kationogenen Gruppen der Komponente b) etwa 1:1 beträgt.

4. Mittel nach einem der vorhergehenden Ansprüche, das zusätzlich wenigstens ein weiteren Monomers d) einpolymerisiert enthält, das ausgewählt unter Estern α,β-ethylenisch ungesättigter Mono- und Dicarbonsäuren mit C₁-C₃₀-Alkanolen und C₁-C₃₀-Alkandiolen, Amiden α,β-ethylenisch ungesättigter Mono- und Dicarbonsäuren mit C₂-C₃₀-Aminoalkoholen, die eine primäre oder sekundäre Aminogruppe aufweisen, N-Alkyl- und N,N-Dialkylamiden α,β-ethylenisch ungesättigter Monocarbonsäuren, die zusätzlich zu dem Carbonyl-Kohlenstoffatom der Amidgruppe mehr als 8 weitere Kohlenstoffatome aufweisen, Estern von vinylalkohol und Allylalkohol mit C₁-C₃₀-Monocarbonsäuren, Vinylethern, Vinylaromaten, vinylhalogeniden, Vinylidenhalogeniden, C₁-C₈-Monoolefinen, nicht aromatischen Kohlenwasserstoffen mit mindestens zwei konjugierten Doppelbindungen, Siloxanmacromeren und Mischungen davon.

5. Mittel nach einem der vorhergehenden Ansprüche, das zusätzlich als Komponente e) wenigstens ein Polyetheracrylat einpolymerisiert enthält.

6. Mittel nach einem der vorhergehenden Ansprüche, das erhältlich ist durch radikalische Copolymerisation in Gegenwart einer Komponente g), die ausgewählt ist unter
g1) polyetherhaltigen Verbindungen,
g2) Polymeren, die mindestens 50 Gew.-% Wiederholungseinheiten aufweisen, die sich von Vinylalkohol ableiten,
g3) Cellulose, Stärke und Derivaten davon,
und Mischungen davon.

7. Mittel nach einem der vorhergehenden Ansprüche, wobei die Komponente a) ausgewählt ist unter monoethylenisch ungesättigten Carbonsäuren, Sulfonsäuren, Phosphonsäuren und Mischungen davon.

8. Mittel nach Anspruch 7, wobei die Komponente a) ausgewählt ist unter Acrylsäure, Methacrylsäure, Ethacrylsäure, α-Chloracrylsäure, Crotonsäure, Maleinsäure, Maleinsäureanhydrid, Fumarsäure, Itaconsäure, Citraconsäure, Mesaconsäure, Glutaconsäure, Aconitsäure, Vinylsulfonsäure, Allylsulfonsäure, Sulfoethylacrylat, Sulfoethylmethacrylat, Sulfopropylacrylat, Sulfopropylmethacrylat, 2-Bydroxy-3-acryloxypropylsul fonsäure, 2-Hydroxy-3-methacryloxypropylsulfonsäure, Styrolsulfonsäure, 2-Acrylamido-2-methylpropansulfonsäure, Vinylphosphonsäure und Allylphosphonsäure und Mischungen davon.

9. Mittel nach Anspruch 8, wobei die Komponente a) ausgewählt ist unter Acrylsäure, Methacrylsäure und Mischungen, die Acrylsäure und/oder Methacrylsäure enthalten.

10. Mittel nach Anspruch 8, wobei die Komponente a) ausgewählt ist unter 2-Acrylamido-2-methylpropansnlfoasäure und Mischungen, die diese enthalten.

11. Mittel nach einem der vorhergehenden Ansprüche, wobei die Komponente b) ausgewählt ist unter Estern α,β-ethylenisch ungesättigter Mono- und Dicarbonsäuren mit Aminoalkoholen, welche am Aminstickstoff mono- oder dialkyliert sein können, Amiden α,β-ethylenisch ungesättigter Mono- und Dicarbonsäuren mit Diaminen, welche mindestens eine primäre oder sekundäre Aminogruppe aufweisen, N,N-Diallylamln, N,N-Diallyl-N-alkylaminen und deren Derivaten, vinyl- und allylsubstituierten Stickstoffheterocyclen, vinyl- und allylsubstituierten heteroaromatischen Verbindungen und Mischungen davon.

12. Mittel nach Anspruch 11, wobei die Komponente b) ausgewählt ist unter N,N-Dimethylaminoethyl(meth)acrylat, N,N-Dimethylaminopropyl(meth)acrylat, vinylimidazol, N-[3-(dimethylamino)propyl](meth)acrylamid, N-(tert.-Butyl)aminoethyl(meth)acrylat, N,N-Diallylamin, N,N-Diallyl-N-methylamin und Mischungen davon.

13. Mittel nach einem der vorhergehenden Ansprüche, wobei die Komponente c) ausgewählt ist unter primären Amiden α,β-ethylenisch ungesättigter Monocarbonsäuren, N-Vinylamiden gesättigter Monocarbonsäuren, N-Vinyllactamen, N-Alkyl- und N,N-Dialkylamiden α,β-ethylenisch ungesättigter Monocarbonsäuren und Mischungen davon.

14. Mittel nach Anspruch 13, wobei die Komponente c) ausgewählt ist unter Acrylsäureamid, Methacrylsäureamid, N-Vinylpyrrolidon, N-vinylcaprolactam, N-Vinylformamid, N-vinylacetamid und Mischungen davon.

15. Mittel nach einem der vorhergehenden Ansprüche, das zusätzlich wenigstens eine radikalisch polymerisierbare vernetzende Verbindung f mit wenigstens zwei α,β-ethylenisch ungesättigten Doppelbindungen pro Molekül einpolymerisiert enthält.

16. Mittel nach einem der Ansprüche 1 bis 15, das aus Wiederholungseinheiten von
- Vinylpyrrolidon,
- Acrylsäure und/oder Methacrylsäure,
- Dimethylaminoethylmethacrylat oder Dimethylaminopropylmethacrylsäureamid oder Vinylimidazol oder tert.-Butylaminoethylmethacrylat und
- wenigstens einem Polyetheracrylat
besteht.

17. Mittel nach einem der Ansprüche 1 bis 15, das aus Wiederholungseinheiten von
- Vinylpyrrolidon,
- 2-Acrylamido-2-methylpropansulfonsäure,
- Dimethylaminoethylmethacrylat oder Dimethylaminopropylmethacrylsäureamid oder Vinylimidazol oder tert.-Butylaminoethylmethacrylat und
- wenigstens einem Polyetheracrylat
besteht.

18. Mittel nach einem der Ansprüche 1 bis 15, das aus Wiederholungseinheiten von
- Vinylpyrrolidon,
- Acrylsäureamid und/oder Methacrylsäureamid,
- Acrylsäure und/oder Methacrylsäure,
- Dimethylaminoethylmethacrylat oder Dimethylaminopropylmethacrylsäureamid oder Vinylimidazol oder tert.-Butylaminoethylmethacrylat und
- wenigstens einem Polyetheracrylat
besteht.

19. Mittel nach einem der Ansprüche 1 bis 15, das aus wiederholungseinheiten von
- Vinylpyrrolidon,
- Acrylsäureamid und/oder Methacrylsäureamid,
- 2-Acrylamido-2-methylpropansulfonsäure,
- Dimethylaminoethylmethacrylat oder Dimethylaminopropylmethacrylsäureamid oder Vinylimidazol oder tert.-Butylaminoethylmethacrylat und
- wenigstens einem Polyetheracrylat
besteht.

20. Mittel nach einem der Ansprüche 1 bis 15, das aus Wiederholungseinheiten von
- Vinylpyrrolidon,
- Acrylsäure und/oder Methacrylsäure,
- Dimethylaminoethylmethacrylat oder Dimethylaminopropylmethacrylsäureamid oder vinylimidazol oder tert.-Butylaminoethylmethacrylat und
- wenigstens einem Polyetheracrylat,
- wenigstens einem Monomer der Formel
CH₂=CR^{c}-C(=O)-O-R^{d}
worin
R^{c} für H oder Methyl steht und
R^{d} für lineares C₁-C₄-Alkyl steht,
besteht.

21. Mittel nach einem der Ansprüche 1 bis 15, das aus Wiederholungseinheiten von
- vinylpyrrolidon,
- 2-Acrylamido-2-methylpropansulfonsäure,
- Dimethylaminoethylmethacrylat oder Dimethylaminopropylmethacrylsäureamid oder Vinylimidazol oder tert.-Butylaminoethylmethacrylat und
- wenigstens einem Polyetheracrylat,
- wenigstens einem Monomer der Formel
CH₂=CR^{c}-C(=O)-O-R^{d}
worin
R^{c} für H oder Methyl steht und
R^{d} für lineares C₁-C₄-Alkyl steht,
besteht.

22. Mittel nach einem der Ansprüche 1 bis 15, das aus Wiederholungseinheiten von
- vinylpyrrolidon,
- Acrylsäureamid und/oder Methacrylsäureamid,
- Acrylsäure und/oder Methacrylsäure,
- Dimethylaminoethylmethacrylat oder Dimethylaminopropylmethacrylsäureamid oder Vinylimidazol oder tert.-Butylaminoethylmethacrylat und
- wenigstens einem Polyetheracrylat,
- wenigstens einem Monomer der Formel
CH₂=CR^{c}-C(=O)-O-R^{d}
worin
R^{c} für H oder Methyl steht und
R^{d} für lineares C₁-C₄-Alkyl steht,
besteht.

23. Mittel nach einem der Ansprüche 1 bis 15, das aus Wiederholungseinheiten von
- Vinylpyrrolidon,
- Acrylsäureamid und/oder Methacrylsäureamid,
- 2-Acrylamido-2-methylpropansulfonsäure,
- Dimethylaminoethylmethacrylat oder Dimethylaminopropylmethacrylsäureamid oder Vinylimidazol oder tert.-Butylaminoethylmethacrylat und
- wenigstens einem Polyetheracrylat,
- wenigstens einem Monomer der Formel
CH₂=CR^{c}-C(=O)-O-R^{d}
worin
R^{c} für H oder Methyl steht und
R^{d} für lineares C₁-C₄-Alkyl steht,
besteht.

24. Mittel nach einem der Ansprüche 16 bis 23, das zusätzlich ein Salz der 2-Acrylamido-2-methylpropansulfonsäure, vorzugsweise das Natriumsalz, einpolymerisiert enthält.

25. Mittel nach einem der Ansprüche 16 bis 24, das zusätzlich ein quaternisiertes Amingruppen-haltiges Monomer, vorzugsweise quaternisiertes Vinylimidazol, einpolymerisiert enthält.

26. Mittel nach einem der Ansprüche 16 bis 25, das zusätzlich bis zu 1 Gew.-%, bezogen auf das Gesamtgewicht der zur Polymerisation eingesetzten Monomere, wenigstens eines vernetzers einpolymerisiert enthält.

27. Mittel nach einem der vorhergehenden Ansprüche, wobei die Komponente B) ausgewählt ist unter
i) Wasser,
ii) wassermischbaren organischen Lösungsmitteln, vorzugsweise C₁-C₄-Alkanolen,
iii) ölen, Fetten, Wachsen,
iv) von iii) verschiedenen Estern von C₆-C₃₀-Monocarbonsäuren mit ein-, zwei- oder dreiwertigen Alkoholen,
v) gesättigten acyclischen und cyclischen Kohlenwasserstoffen,
vi) Fettsäuren,
vii) Fettalkoholen
und Mischungen davon.

28. Mittel nach einem der vorhergehenden Ansprüche, enthaltend außerdem wenigstens einen von der Komponente A) verschiedenen Bestandteil, der ausgewählt ist unter kosmetisch aktiven Wirkstoffen, Emulgatoren, Tensiden, Konservierungsmitteln, Parfümölen, Verdickern, Haarpolymeren, Haar- und Hautconditionern, Pfropfpolymeren, wasserlöslichen oder dispergierbaren silikonhaltigen Polymeren, Lichtschutzmitteln, Bleichmitteln, Gelbildnern, Pflegemitteln, Färbemitteln, Tönungsmitteln, Bräunungsmitteln, Farbstoffen, Pigmenten, Konsistenzgebern, Feuchthaltemitteln, Rückfettern, Collagen, Eiweißhydrolysaten, Lipiden, Antioxidantien, Entschäumern, Antistatika, Emollienzien und Weichmachern.

29. Mittel nach einem der vorhergehenden Ansprüche in Form eines Gels, Schaums, Sprays, einer Salbe, Creme, Emulsion, Suspension, Lotion, Milch oder Paste.

30. Ampholytisches Copolymer, erhältlich durch radikalische Copolymerisation von
a) wenigstens einer Verbindung mit einer radikalisch polymerisierbaren, α,β-ethylenisch ungesättigten Doppelbindung und mindestens einer anionogenen und/oder anionischen Gruppe pro Molekül,
b) wenigstens einer Verbindung mit einer radikalisch polymerisierbaren, α,β-ethylenisch ungesättigten Doppelbindung und mindestens einer kationogenen und/oder kationischen Gruppe pro Molekül,
c) wenigstens einer α,β-ethylenisch ungesättigten amidgruppenhaltigen Verbindung der allgemeinen Formel I 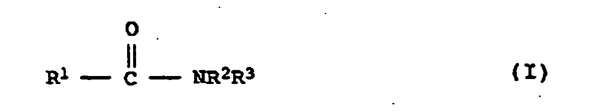
worin
einer der Rest R¹ bis R³ für eine Gruppe der Formel CH₂=CR⁴- mit R⁴ = H oder C₁-C₄-Alkyl steht und die übrigen Reste R¹ bis R³ unabhängig voneinander für H, Alkyl, Cycloalkyl, Heterocycloalkyl, Aryl oder Hetaryl stehen,
wobei R¹ und R² gemeinsam mit der Amidgruppe, an die sie gebunden sind, auch für ein Lactam mit 5 bis 8 Ringatomen stehen können,
wobei R² und R³ gemeinsam mit dem Stickstoffatom, an das sie gebunden sind, auch für einen fünf- bis siebengliedrigen Heterocyclus stehen können,
mit der Maßgabe, dass die Summe der Kohlenstoffatome der Reste R¹, R² und R³ höchstens 8 beträgt, wobei die Komponente c) ausgewählt ist unter N-vinylamiden gesättigter Monocarbonsäuren, N-vinyllactamen, N-Alkyl- und N,N-Dialkylamiden α,β-ethylenisch ungesättigter Monocarbonsäuren und Mischungen davon.

31. Polyelektrolyt-Komplex, enthaltend wenigstens ein ampholytisches Copolymer, wie in Anspruch 30 definiert und wenigstens einen weiteren, davon verschiedenen Polyelektrolyten.

32. Verwendung eines Polymers, wie in Anspruch 30 definiert oder eines Polyelektrolyt-Komplexes, wie in Anspruch 31 definiert, in Hautreinigungsmitteln, Mitteln zur Pflege und zum Schutz der Haut, Nagelpflegemitteln, Zubereitungen für die dekorative Kosmetik und Haarbehandlungsmitteln.

33. Verwendung nach Anspruch 32 in Haarbehandlungsmittein als Festiger und/oder als Conditioner.

34. Verwendung nach Anspruch 33, wobei das Mittel in Form eines Haargels, Shampoos, Schaumfestigers, Haarwassers, Haarsprays oder Haarschaums vorliegt.

35. Verwendung eines Polymers, wie in Anspruch 30 definiert oder eines Polyelektrolyt-Komplexes, wie in Anspruch 31 definiert, als Hilfsmittel in der Pharmazie, bevorzugt als oder in Beschichtungsmittel(n) für feste Arzneiformen, zur Modifizierung rheologischer Eigenschaften, als oberflächenaktive Verbindung sowie als oder in Beschichtungsmittel(n) für die Textil-, Papier-, Druck- und Lederindustrie.

## Claims

1. A cosmetic or pharmaceutical composition comprising
A) at least one ampholytic copolymer, obtainable by free-radical copolymerization of
a) at least one compound with a free-radically polymerizable, α,β-ethylenically unsaturated double bond and at least one anionogenic and/or anionic group per molecule,
b) at least one compound with a free-radically polymerizable, α,β-ethylenically unsaturated double bond and at least one cationogenic and/or cationic group per molecule,
c) at least one α,β-ethylenically unsaturated amide-group-containing compound of the formula I 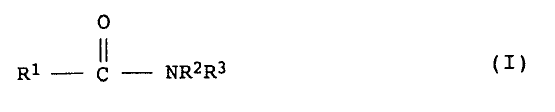 in which
one of the radicals R¹ to R³ is a group of the formula CH₂=CR⁴- where R⁴ = H or C₁-C₄-alkyl, and the other radicals R¹ to R³, independently of one another, are H, alkyl, cycloalkyl, heterocycloalkyl, aryl or hetaryl,
where R¹ and R² together with the amide group to which they are bonded may also be a lactam with 5 to 8 ring atoms,
where R² and R³ together with the nitrogen atom to which they are bonded may also be a five- to seven-membered heterocycle,
with the proviso that the sum of the carbon atoms of the radicals R¹, R² and R³ is at most 8,
or a polyelectrolyte complex comprising at least one ampholytic copolymer of this kind and at least one further polyelectrolyte different therefrom, and
B) at least one cosmetically acceptable carrier.

2. The composition according to claim 1, where the quantitative molar ratio of compounds a) to compounds b) is in a range from 0.5:1 to less than 2:1, preferably from 0.7:1 to 1.8:1.

3. The composition according to either of the preceding claims, where at least some of the compounds a) and b) are used in the form of a monomer composition, where the molar ratio of anionogenic groups of component a) to cationogenic groups of component b) is about 1:1.

4. The composition according to one of the preceding claims which additionally comprises, in copolymerized form, at least one further monomer d) which is chosen from esters of α,β-ethylenically unsaturated mono- and dicarboxylic acids with C₁-C₃₀-alkanols and C₁-C₃₀-alkanediols, amides of α,β-ethylenically unsaturated mono- and dicarboxylic acids with C₂-C₃₀-aminoalcohols which have a primary or secondary amino group, N-alkyl- and N,N-dialkylamides of α,β-ethylenically unsaturated monocarboxylic acids which, in addition to the carbonyl carbon atom of the amide group, have more than 8 further carbon atoms, esters of vinyl alcohol and allyl alcohol with C₁-C₃₀-monocarboxylic acids, vinyl ethers, vinylaromatics, vinyl halides, vinylidene halides, C₁-C₈-monoolefins, nonaromatic hydrocarbons with at least two conjugated double bonds, siloxane macromers and mixtures thereof.

5. The composition according to one of the preceding claims, which additionally comprises, as component e), at least one polyether acrylate in copolymerized form.

6. The composition according to one of the preceding claims, which is obtainable by free-radical copolymerization in the presence of a component g) which is chosen from
g1) polyether-containing compounds,
g2) polymers which have at least 50% by weight of repeat units which are derived from vinyl alcohol,
g3) cellulose, starch and derivatives thereof,
and mixtures thereof.

7. The composition according to one of the preceding claims, where component a) is chosen from monoethylenically unsaturated carboxylic acids, sulfonic acids, phosphonic acids and mixtures thereof.

8. The composition according to claim 7, where component a) is chosen from acrylic acid, methacrylic acid, ethacrylic acid, α-chloroacrylic acid, crotonic acid, maleic acid, maleic anhydride, fumaric acid, itaconic acid, citraconic acid, mesaconic acid, glutaconic acid, aconitic acid, vinylsulfonic acid, allylsulfonic acid, sulfoethyl acrylate, sulfoethyl methacrylate, sulfopropyl acrylate, sulfopropyl methacrylate, 2-hydroxy-3-acryloxypropylsulfonic acid, 2-hydroxy-3-methacryloxypropylsulfonic acid, styrenesulfonic acid, 2-acrylamido-2-methylpropanesulfonic acid, vinylphosphonic acid and allylphosphonic acid and mixtures thereof.

9. The composition according to claim 8, where component a) is chosen from acrylic acid, methacrylic acid and mixtures which comprise acrylic acid and/or methacrylic acid.

10. The composition according to claim 8, where component a) is chosen from 2-acrylamido-2-methylpropanesulfonic acid and mixtures which comprise this.

11. The composition according to one of the preceding claims, where component b) is chosen from esters of α,β-ethylenically unsaturated mono- and dicarboxylic acids with amino alcohols which may be mono- or dialkylated on the amine nitrogen, amides of α,β-ethylenically unsaturated mono- and dicarboxylic acids with diamines which have at least one primary or secondary amino group, N,N-diallylamine, N,N-diallyl-N-alkylamines and derivatives thereof, vinyl- and allyl-substituted nitrogen heterocycles, vinyl- and allyl-substituted heteroaromatic compounds and mixtures thereof.

12. The composition according to claim 11, where component b) is chosen from N,N-dimethylaminoethyl (meth)acrylate, N,N-dimethylaminopropyl (meth)-acrylate, vinylimidazole, N-[3-(dimethylamino)propyl](meth)acrylamide, N-[tert-butyl)-aminoethyl (meth)acrylate, N,N-diallylamine, N,N-diallyl-N-methylamine and mixtures thereof.

13. The composition according to one of the preceding claims, where component c) is chosen from primary amides of α,β-ethylenically unsaturated monocarboxylic acids, N-vinylamides of saturated monocarboxylic acids, N-vinyllactams, N-alkyl- and N,N-dialkylamides of α,β-ethylenically unsaturated monocarboxylic acids and mixtures thereof.

14. The composition according to claim 13, where component c) is chosen from acrylamide, methacrylamide, N-vinylpyrrolidone, N-vinylcaprolactam, N-vinylformamide, N-vinylacetamide and mixtures thereof.

15. The composition according to one of the preceding claims, which additionally comprises, in copolymerized form, at least one free-radically polymerizable crosslinking compound f with at least two α,β-ethylenically unsaturated double bonds per molecule.

16. The composition according to one of claims 1 to 15, which consists of repeat units of
- vinylpyrrolidone,
- acrylic acid and/or methacrylic acid,
- dimethylaminoethyl methacrylate or dimethylaminopropylmethacrylamide or vinylimidazole or tert-butylaminoethyl methacrylate and
- at least one polyether acrylate.

17. The composition according to one of claims 1 to 15, which consists of repeat units of
- vinylpyrrolidone,
- 2-acrylamido-2-methylpropanesulfonic acid,
- dimethylaminoethyl methacrylate or dimethylaminopropylmethacrylamide or vinylimidazole or tert-butylaminoethyl methacrylate and
- at least one polyether acrylate.

18. The composition according to one of claims 1 to 15, which consists of repeat units of
- vinylpyrrolidone,
- acrylamide and/or methacrylamide,
- acrylic acid and/or methacrylic acid,
- dimethylaminoethyl methacrylate or dimethylaminopropylmethacrylamide or vinylimidazole or tert-butylaminoethyl methacrylate and
- at least one polyether acrylate.

19. The composition according to one of claims 1 to 15, which consists of repeat units of
- vinylpyrrolidone,
- acrylamide and/or methacrylamide,
- 2-acrylamido-2-methylpropanesulfonic acid,
- dimethylaminoethyl methacrylate or dimethylaminopropylmethacrylamide or vinylimidazole or tert-butylaminoethyl methacrylate and
- at least one polyether acrylate.

20. The composition according to one of claims 1 to 15, which consists of repeat units of
- vinylpyrrolidone,
- acrylic acid and/or methacrylic acid,
- dimethylaminoethyl methacrylate or dimethylaminopropylmethacylamide or vinylimidazole or tert-butylaminoethyl methacrylate and
- at least one polyether acrylate,
- at least one monomer of the formula
CH₂-CR^{c}-C (=O) -O-R^{d}
in which
R^{c} is H or methyl and
R^{d} is linear C₁-C₄-alkyl.

21. The composition according to one of claims 1 to 15, which consists of repeat units of
- vinylpyrrolidone,
- 2-acrylamido-2-methylpropanesulfonic acid,
- dimethylaminoethyl methacrylate or dimethylaminopropylmethacrylamide or vinylimidazole or tert-butylaminoethyl methacrylate and
- at least one polyether acrylate,
- at least one monomer of the formula
CH₂=CR^{c}-C (=O) -O-R^{d}
in which
R^{c} is H or methyl and
R^{d} is linear C₁-C₄-alkyl.

22. The composition according to one of claims 1 to 15, which consists of repeat units of
- vinylpyrrolidone,
- acrylamide and/or methacrylamide,
- acrylic acid and/or methacrylic acid,
- dimethylaminoethyl methacrylate or dimethylaminopropylmethacrylamide or vinylimidazole or tert-butylaminoethyl methacrylate and
- at least one polyether acrylate,
- at least one monomer of the formula
CH₂=CR^{c}-C(=O)-O-R^{d}
in which
R^{c} is H or methyl and
R^{d} is linear C₁-C₄-alkyl.

23. The composition according to one of claims 1 to 15, which consists of repeat units of
- vinylpyrrolidone,
- acrylamide and/or methacrylamide,
- 2-acrylamido-2-methylpropanesulfonic acid,
- dimethylaminoethyl methacrylate or dimethylaminopropylmethacrylamide or vinylimidazole or tert-butylaminoethyl methacrylate and
- at least one polyether acrylate,
- at least one monomer of the formula
CH₂=CR^{c}-C(=O)-O-R^{d}
in which
R^{c} is H or methyl and
R^{d} is linear C₁-C₄-alkyl.

24. The composition according to one of claims 16 to 23 which additionally comprises a salt of 2-acrylamido-2- methylpropanesulfonic acid, preferably the sodium salt, in copolymerized form.

25. The composition according to one of claims 16 to 24, which additionally comprises a quaternized monomer containing amine groups, preferably quaternized vinylimidazole, in copolymerized form.

26. The composition according to one of claims 16 to 25, which additionally comprises up to 1% by weight, based on the total weight of the monomers used for the polymerization, of at least one crosslinker in copolymerized form.

27. The composition according to one of the preceding claims, where component B) is chosen from
i) water,
ii) water-miscible organic solvents, preferably C₁-C₄-alkanoles,
iii) oils, fats, waxes,
iv) esters of C₆-C₃₀-monocarboxylic acids with mono-, di- or trihydric alcohols different from iii),
v) saturated acyclic and cyclic hydrocarbons,
vi) fatty acids,
vii) fatty alcohols
and mixtures thereof.

28. The composition according to one of the preceding claims, further comprising at least one constituent different from component A) which is chosen from cosmetically active ingredients, emulsifiers, surfactants, preservatives, perfume oils, thickeners, hair polymers, hair and skin conditioners, graft polymers, water-soluble or dispersible silicone-containing polymers, light protection agents, bleaches, gel formers, care agents, colorants, tinting agents, tanning agents, dyes, pigments, bodying agents, moisturizers, refatting agents, collagen, protein hydrolyzates, lipids, antioxidants, defoamers, antistats, emollients and softeners.

29. The composition according to one of the preceding claims in the form of a gel, foam, spray, an ointment, cream, emulsion, suspension, lotion, milk or paste.

30. An ampholytic copolymer obtainable by free-radical copolymerization of
a) at least one compound with a free-radically polymerizable, α,β-ethylenically unsaturated double bond and at least one anionogenic and/or anionic group per molecule,
b) at least one compound with a free-radically polymerizable, α,β-ethylenically unsaturated double bond and at least one cationogenic and/or cationic group per molecule,
c) at least one α,β-ethylenically unsaturated amide-group-containing compound of the formula I 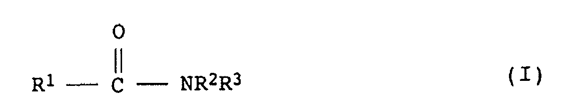
in which
one of the radicals R¹ to R³ is a group of the formula CH₂=CR⁴- where R⁴ = H or C₁-C₄-alkyl, and the other radicals R¹ to R³, independently of one another, are H, alkyl, cycloalkyl, heterocycloalkyl, aryl or hetaryl,
where R¹ and R² together with the amide group to which they are bonded may also be a lactam with 5 to 8 ring atoms,
where R² and R³ together with the nitrogen atom to which they are bonded may also be a five- to seven-membered heterocycle,
with the proviso that the sum of the carbon atoms of the radicals R¹, R² and R³ is at most 8, where component c) is chosen from N-vinylamides of saturated monocarboxylic acids, N-vinyllactams, N-alkyl- and N,N-dialkylamides of α,β-ethylenically unsaturated monocarboxylic acids and mixtures thereof.

31. A polyelectrolyte complex comprising at least one ampholytic copolymer, as defined in claim 30, and at least one further polyelectrolyte different therefrom.

32. The use of a polymer, as defined in claim 30, or of a polyelectrolyte complex, as defined in claim 31, in skin-cleansing compositions, compositions for the care and protection of the skin, nail care compositions, preparations for decorative cosmetics and hair-treatment compositions.

33. The use according to claim 32 in hair-treatment compositions as setting agent and/or as conditioner.

34. The use according to claim 33, where the composition is in the form of a hair gel, shampoo, setting foam, hair tonic, hair spray or hair foam.

35. The use of a polymer, as defined in claim 30, or of a polyelectrolyte complex, as defined in claim 31, as auxiliary in pharmacy, preferably as or in (a) coating(s) for solid medicaments, for the modification of rheological properties, as surface-active compound, and as or in (a) coating(s) for the textile, paper, printing and leather industry.

## Revendications

1. Produit cosmétique ou pharmaceutique contenant
A) au moins un copolymère ampholytique que l'on peut obtenir par copolymérisation radicalaire
a) d'au moins un composé comportant une double liaison α,β-éthyléniquement insaturée, polymérisable par voie radicalaire, et au moins un groupe anionogène et/ou anionique par molécule,
b) d'au moins un composé comportant une double liaison α,β-éthyléniquement insaturée, polymérisable par voie radicalaire, et au moins un groupe cationogène et/ou cationique par molécule,
c) d'au moins un composé α,β-éthyléniquement insaturé contenant des groupes amide et répondant à la formule générale I : 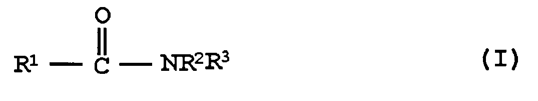
dans laquelle
un des radicaux R¹ à R³ représente un groupe de la formule CH₂=CR⁴- où R⁴ = H ou un groupe alkyle en C₁-C₄, et les autres radicaux R¹ à R³ représentent indépendamment l'un de l'autre H ou un groupe alkyle, cycloalkyle, hétérocycloalkyle, aryle ou hétaryle,
R¹ et R² pouvant aussi représenter, conjointement au groupe amide sur lequel ils sont fixés, un lactame présentant 5 à 8 atomes de noyau,
R² et R³ pouvant aussi représenter, conjointement à l'atome d'azote sur lequel ils sont fixés, un hétérocycle pentagonal à heptagonal,
à la condition que la somme des atomes de carbone des radicaux R¹, R² et R³ soit au maximum de 8,
ou un complexe polyélectrolytique contenant au moins un tel copolymère ampholytique et au moins un autre polyélectrolyte différent de celui-ci, et
B) au moins un support acceptable en cosmétique.

2. Produit suivant la revendication 1, dans lequel les proportions molaires entre les composés a) et les composés b) sont de l'ordre de 0,5/1 à moins de 2/1, de préférence de 0,7/1 à 1,8/1.

3. Produit suivant l'une des revendications précédentes, dans lequel au moins une partie des composés a) et b) est mise en oeuvre sous la forme d'une composition de monomères, le rapport molaire entre les groupes anionogènes du composant a) et les groupes cationogènes du composant b) étant d'environ 1/1.

4. Produit suivant l'une des revendications précédentes qui contient en supplément, à l'état copolymérisé, au moins un autre monomère d) qui est choisi parmi des esters d'acides monocarboxyliques et dicarboxyliques α,β-éthyléniquement insaturés avec des alcanols en C₁-C₃₀ et des alcanediols en C₁-C₃₀, des amides d'acides monocarboxyliques et dicarboxyliques α,β-éthyléniquement insaturés avec des aminoalcools en C₂-C₃₀ qui présentent un groupe amino primaire ou secondaire, des N-alkylamides et des N,N-dialkylamides d'acides monocarboxyliques α,β-éthyléniquement insaturés qui présentent en supplément de l'atome de carbone du carbonyle du groupe amide plus de 8 autres atomes de carbone, des esters d'alcool vinylique et d'alcool allylique avec des acides monocarboxyliques en C₁-C₃₀, des éthers vinyliques, des substances vinylaromatiques, des halogénures de vinyle, des halogénures de vinylidène, des monooléfines en C₁-C₈, des hydrocarbures non aromatiques comportant au moins deux doubles liaisons conjuguées, des macromères de siloxane et leurs mélanges.

5. Produit suivant l'une des revendications précédentes qui contient en supplément, comme composant e), au moins un polyétheracrylate à l'état copolymérisé.

6. Produit suivant l'une des revendications précédentes qui peut être obtenu par copolymérisation radicalaire en présence d'un composant g) qui est choisi parmi
g1) des composés contenant du polyéther,
g2) des polymères qui présentent au moins 50 % en poids d'unités répétitives qui dérivent de l'alcool vinylique,
g3) de la cellulose, de l'amidon et leurs dérivés,
et des mélanges de ceux-ci.

7. Produit suivant l'une des revendications précédentes dans lequel le composant a) est choisi parmi des acides carboxyliques monoéthyléniquement insaturés, des acides sulfoniques, des acides phosphoniques et leurs mélanges.

8. Produit suivant la revendication 7, dans lequel le composant a) est choisi parmi de l'acide acrylique, de l'acide méthacrylique, de l'acide éthacrylique, de l'acide α-chloroacrylique, de l'acide crotonique, de l'acide maléique, de l'anhydride maléique, de l'acide fumarique, de l'acide itaconique, de l'acide citraconique, de l'acide mésaconique, de l'acide glutaconique, de l'acide aconitique, de l'acide vinylsulfonique, de l'acide allylsulfonique, de l'acrylate de sulfoéthyle, du méthacrylate de sulfoéthyle, de l'acrylate de sulfopropyle, du méthacrylate de sulfopropyle, de l'acide 2-hydroxy-3-acryloxypropylsulfonique, de l'acide 2-hydroxy-3-méthacryloxypropylsulfonique, de l'acide styrènesulfonique, de l'acide 2-acrylamido-2-méthyl-propanesulfonique, de l'acide vinylphosphonique et de l'acide allylphosphonique et leurs mélanges.

9. Produit suivant la revendication 8, dans lequel le composant a) est choisi parmi de l'acide acrylique, de l'acide méthacrylique et des mélanges qui contiennent de l'acide acrylique et/ou de l'acide méthacrylique.

10. Produit suivant la revendication 8, dans lequel le composant a) est choisi parmi de l'acide 2-acrylamido-2-méthylpropanesulfonique et des mélanges qui contiennent celui-ci.

11. Produit suivant l'une des revendications précédentes, dans lequel le composant b) est choisi parmi des esters d'acides monocarboxyliques et dicarboxyliques α,β-éthyléniquement insaturés avec des aminoalcools qui peuvent être monoalkylés ou dialkylés sur l'azote d'amine, des amides d'acides monocarboxyliques et dicarboxyliques α,β-éthyléniquement insaturés avec des diamines qui présentent au moins un groupe amino primaire ou secondaire, de la N,N-diallylamine, des N,N-diallyl-N-alkylamines et leurs dérivés, des hétérocycles azotés substitués par du vinyle et de l'allyle, des composés hétéroaromatiques substitués par du vinyle et de l'allyle et leurs mélanges.

12. Produit suivant la revendication 11, dans lequel le composant b) est choisi parmi du (méth)acrylate de N,N-diméthylaminoéthyle, du (méth)acrylate de N,N-diméthylaminopropyle, du vinylimidazole, du N-[3-(diméthylamino)propyl](méth)acrylamide, du (méth)acrylate de N-(tert-butyl)aminoéthyle, de la N,N-diallylamine, de la N,N-diallyl-N-méthylamine et leurs mélanges.

13. Produit suivant l'une des revendications précédentes, dans lequel le composant c) est choisi parmi des amides primaires d'acides monocarboxyliques α,β-éthyléniquement insaturés, des N-vinylamides d'acides monocarboxyliques saturés, des N-vinyllactames, des N-alkylamides et N,N-dialkylamides d'acides monocarboxyliques α,β-éthyléniquement insaturés et leurs mélanges.

14. Produit suivant la revendication 13, dans lequel le composant c) est choisi parmi de l'amide d'acide acrylique, de l'amide d'acide méthacrylique, de la N-vinylpyrrolidone, du N-vinylcaprolactame, du N-vinylformamide, du N-vinylacétamide et leurs mélanges.

15. Produit suivant l'une des revendications précédentes qui contient en supplément, à l'état copolymérisé, au moins un composé f réticulant qui est polymérisable par voie radicalaire et comporte au moins deux doubles liaisons α,β-éthyléniquement insaturées par molécule.

16. Produit suivant l'une des revendications 1 à 15 qui est constitué d'unités répétitives
- de vinylpyrrolidone,
- d'acide acrylique et/ou d'acide méthacrylique,
- de méthacrylate de diméthylaminoéthyle ou d'amide d'acide diméthylaminopropylméthacrylique ou de vinylimidazole ou de méthacrylate de tert-butylaminoéthyle, et
- d'au moins un polyétheracrylate.

17. Produit suivant l'une des revendications 1 à 15 qui est constitué d'unités répétitives
- de vinylpyrrolidone,
- d'acide 2-acrylamido-2-méthylpropanesulfonique,
- de méthacrylate de diméthylaminoéthyle ou d'amide d'acide diméthylaminopropylméthacrylique ou de vinylimidazole ou de méthacrylate de tert-butylaminoéthyle, et
- d'au moins un polyétheracrylate.

18. Produit suivant l'une des revendications 1 à 15 qui est constitué d'unités répétitives
- de vinylpyrrolidone,
- d'amide d'acide acrylique et/ou d'amide d'acide méthacrylique,
- d'acide acrylique et/ou d'acide méthacrylique,
- de méthacrylate de diméthylaminoéthyle ou d'amide d'acide diméthylaminopropylméthacrylique ou de vinylimidazole ou de méthacrylate de tert-butylaminoéthyle, et
- d'au moins un polyétheracrylate.

19. Produit suivant l'une des revendications 1 à 15 qui est constitué d'unités répétitives
- de vinylpyrrolidone,
- d'amide d'acide acrylique et/ou d'amide d'acide méthacrylique,
- d'acide 2-acrylamido-2-méthylpropanesulfonique,
- de méthacrylate de diméthylaminoéthyle ou d'amide d'acide diméthylaminopropylméthacrylique ou de vinylimidazole ou de méthacrylate de tert-butylaminoéthyle, et
- d'au moins un polyétheracrylate.

20. Produit suivant l'une des revendications 1 à 15 qui est constitué d'unités répétitives
- de vinylpyrrolidone,
- d'acide acrylique et/ou d'acide méthacrylique,
- de méthacrylate de diméthylaminoéthyle ou d'amide d'acide diméthylaminopropylméthacrylique ou de vinylimidazole ou de méthacrylate de tert-butylaminoéthyle, et
- d'au moins un polyétheracrylate,
- d'au moins un monomère de la formule :
CH₂=CR^{c}-C (=O) -O-R^{d}
dans laquelle
R^{c} représente H ou du méthyle, et
R^{d} représente un groupe alkyle en C₁-C₄ linéaire.

21. Produit suivant l'une des revendications 1 à 15 qui est constitué d'unités répétitives
- de vinylpyrrolidone,
- d'acide 2-acrylamido-2-méthylpropanesulfonique,
- de méthacrylate de diméthylaminoéthyle ou d'amide d'acide diméthylaminopropylméthacrylique ou de vinylimidazole ou de méthacrylate de tert-butylaminoéthyle, et
- d'au moins un polyétheracrylate,
- d'au moins un monomère de la formule :
CH₂=CR^{c}-C(=O) -O-R^{d}
dans laquelle
R^{c} représente H ou du méthyle, et
R^{d} représente un groupe alkyle en C₁-C₄ linéaire.

22. Produit suivant l'une des revendications 1 à 15 qui est constitué d'unités répétitives
- de vinylpyrrolidone,
- d'amide d'acide acrylique et/ou d'amide d'acide méthacrylique,
- d'acide acrylique et/ou d'acide méthacrylique,
- de méthacrylate de diméthylaminoéthyle ou d'amide d'acide diméthylaminopropylméthacrylique ou de vinylimidazole ou de méthacrylate de tert-butylaminoéthyle, et
- d'au moins un polyétheracrylate,
- d'au moins un monomère de la formule :
CH₂=CR^{c}-C (=O) -O-R^{d}
dans laquelle
R^{c} représente H ou du méthyle, et
R^{d} représente un groupe alkyle en C₁-C₄ linéaire.

23. Produit suivant l'une des revendications 1 à 15 qui est constitué d'unités répétitives
- de vinylpyrrolidone,
- d'amide d'acide acrylique et/ou d'amide d'acide méthacrylique,
- d'acide 2-acrylamido-2-méthylpropanesulfonique,
- de méthacrylate de diméthylaminoéthyle ou d'amide d'acide diméthylaminopropylméthacrylique ou de vinylimidazole ou de méthacrylate de tert-butylaminoéthyle, et
- d'au moins un polyétheracrylate,
- d'au moins un monomère de la formule :
CH₂=CR^{c}-C(=O)-O-R^{d}
dans laquelle
R^{c} représente H ou du méthyle, et
R^{d} représente un groupe alkyle en C₁-C₄ linéaire.

24. Produit suivant l'une des revendications 16 à 23 qui contient en supplément, à l'état copolymérisé, un sel de l'acide 2-acrylamido-2-méthylpropanesulfonique, de préférence le sel de sodium.

25. Produit suivant l'une des revendications 16 à 24 qui contient en supplément, à l'état copolymérisé, un monomère contenant des groupes amine quaternisé, de préférence du vinylimidazole quaternisé.

26. Produit suivant l'une des revendications 16 à 25 qui contient en supplément, à l'état copolymérisé, jusqu'à 1 % en poids d'au moins un agent de réticulation, par rapport au poids total des monomères mis en oeuvre pour la polymérisation.

27. Produit suivant l'une des revendications précédentes, dans lequel le composant B) est choisi parmi
i) de l'eau,
ii) des solvants organiques miscibles à l'eau, de préférence des alcanols en C₁-C₄,
iii) des huiles, des graisses, des cires,
iv) des esters différents de iii) d'acides monocarboxyliques en C₆-C₃₀ avec des alcools monofonctionnels, difonctionnels ou trifonctionnels,
v) des hydrocarbures cycliques et acycliques saturés,
vi) des acides gras,
vii) des alcools gras,
et leurs mélanges.

28. Produit suivant l'une des revendications précédentes contenant en outre au moins un élément différent du composant A) qui est choisi parmi des substances cosmétiquement actives, des agents émulsionnants, des agents tensioactifs, des antiseptiques, des huiles de parfum, des épaississants, des polymères pour cheveux, des conditionneurs de cheveux et de peau, des polymères greffés, des polymères contenant de la silice solubles ou dispersibles dans l'eau, des agents de protection contre la lumière, des agents de blanchiment, des générateurs de gel, des produits de soins, des colorants, des agents de nuancement, des agents de brunissement, des matières tinctoriales, des pigments, des agents donnant de la consistance, des agents humidifiants, des agents regraissants, du collagène, des produits d'hydrolyse de protéine, des lipides, des antioxydants, des agents antimousse, des antistatiques, des émollients et des agents ramollisants.

29. Produit suivant l'une des revendications précédentes sous la forme d'un gel, d'une mousse, d'une pulvérisation, d'une pommade, d'une crème, d'une émulsion, d'une suspension, d'une lotion, d'un lait ou d'une pâte.

30. Copolymère ampholytique que l'on peut obtenir par copolymérisation radicalaire
a) d'au moins un composé comportant une double liaison α,β-éthyléniquement insaturée, polymérisable par voie radicalaire, et au moins un groupe anionogène et/ou anionique par molécule,
b) d'au moins un composé comportant une double liaison α,β-éthyléniquement insaturée, polymérisable par voie radicalaire, et au moins un groupe cationogène et/ou cationique par molécule,
c) d'au moins un composé contenant des groupes amide α,β-éthyléniquement insaturés de la formule générale I : 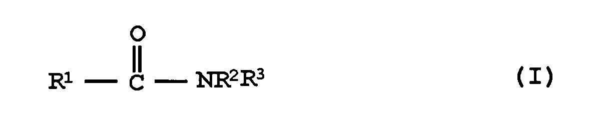
dans laquelle
un des radicaux R¹ à R³ représente un groupe de la formule CH₂=CR⁴-, où R⁴ = H ou un groupe alkyle en C₁-C₄, et les autres radicaux R¹ à R³ représentent, indépendamment l'un de l'autre, H
ou un groupe alkyle, cycloalkyle, hétérocycloalkyle, aryle ou hétaryle,
R¹ et R² pouvant aussi représenter, conjointement au groupe amide sur lequel ils sont fixés, un lactame présentant 5 à 8 atomes de noyau,
R² et R³ pouvant aussi représenter, conjointement à l'atome d'azote sur lequel ils sont fixés, un hétérocycle pentagonal à heptagonal,
à la condition que la somme des atomes de carbone des radicaux R¹, R² et R³ soit au maximum de 8, le composant c) étant choisi parmi des
N-vinylamides d'acides monocarboxyliques saturés, des N-vinyllactames, des N-alkylamides et N,N-dialkylamides d'acides monocarboxyliques α,β-éthyléniquement insaturés et leurs mélanges.

31. Complexe polyélectrolytique contenant au moins un copolymère ampholytique, tel que défini dans la revendication 30, et au moins un autre polyélectrolyte différent de celui-ci.

32. Utilisation d'un polymère, tel que défini dans la revendication 30, ou d'un complexe polyélectrolytique, tel que défini dans la revendication 31, dans des produits de nettoyage de la peau, des produits pour les soins et pour la protection de la peau, des produits pour les soins des ongles, des compositions pour la cosmétique décorative et des produits de traitement de cheveux.

33. Utilisation suivant la revendication 32 dans des produits de traitement de cheveux, comme des renforçateurs et/ou des conditionneurs.

34. Utilisation suivant la revendication 33, dans laquelle le produit se présente sous la forme d'un gel pour les cheveux, d'un shampooing, d'un renforçateur mousse, d'une eau pour les cheveux, d'une pulvérisation pour les cheveux ou d'une mousse pour les cheveux.

35. Utilisation d'un polymère, tel que défini dans la revendication 30, ou d'un complexe polyélectrolytique, tel que défini dans la revendication 31, comme adjuvant dans la pharmacie, de préférence sous la forme d'agent(s) de revêtement pour les médicaments solides ou dans ceux-ci, pour la modification de propriétés rhéologiques, comme composé tensioactif ainsi que comme agent(s) de revêtement pour l'industrie textile, du papier, de l'impression et du cuir ou dans ceux-ci.
